(19) Europäisches Patentamt
European Patent Office
Office européen des brevets

(11) EP 2 220 255 B1

(12) **FASCICULE DE BREVET EUROPEEN**

(45) Date de publication et mention de la délivrance du brevet:
**07.01.2015 Bulletin 2015/02**

(21) Numéro de dépôt: **08871977.8**

(22) Date de dépôt: **25.11.2008**

(51) Int Cl.:
*C12Q 1/68* (2006.01)

(86) Numéro de dépôt international:
**PCT/FR2008/001640**

(87) Numéro de publication internationale:
**WO 2009/095567 (06.08.2009 Gazette 2009/32)**

(54) **PROCEDE D'ETUDE DE LA DIVERSITE COMBINATOIRE V(D)J**

VERFAHREN ZUR UNTERSUCHUNG DER V(D)J-KOMBINATIONSDIVERSITÄT

METHOD FOR STUDYING THE V(D)J COMBINATORIAL DIVERSITY

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **26.11.2007 EP 07291401**

(43) Date de publication de la demande:
**25.08.2010 Bulletin 2010/34**

(73) Titulaires:
- **ImmunID**
**38100 Grenoble (FR)**
- **Commissariat à l'Énergie Atomique et aux Énergies Alternatives**
**75015 Paris (FR)**

(72) Inventeurs:
- **PASQUAL, Nicolas**
**F-38100 Grenoble (FR)**
- **WEISBUCH, Sébastien**
**F-38090 Villefontaine (FR)**

(74) Mandataire: **Marcadé, Véronique et al**
**Cabinet Orès**
**36, rue de St Pétersbourg**
**75008 Paris (FR)**

(56) Documents cités:
**WO-A-2004/033728 WO-A-2005/056828**
- **DONGEN VAN J J M ET AL: "DESIGN AND STANDARDIZATION OF PCR PRIMERS AND PROTOCOLS FOR DETECTION OF CLONAL IMMUNOGLOBULIN AND T-CELL RECEPTOR GENE RECOMBINATIONS IN SUSPECT LYMPHOPROLIFERATIONS: REPORT OF THE BIOMED-2 CONCERTED ACTION BMH4-CT98-3936" LEUKEMIA, MACMILLAN PRESS LTD, US, vol. 17, 2003, pages 2257-2317, XP008093070 ISSN: 0887-6924**
- **PASQUAL N ET AL: "Quantitative and qualitative changes in V-J alpha rearrangements during mouse thymocytes differentiation: implication for a limited T cell receptor alpha chain repertoire" JOURNAL OF EXPERIMENTAL MEDICINE, TOKYO, JP, vol. 196, no. 9, 4 novembre 2002 (2002-11-04), pages 1163-1173, XP002322207 ISSN: 0022-1007**
- **DATABASE Geneseq [Online] 25 mars 2004 (2004-03-25), "Human Vbeta gene." XP002535776 extrait de EBI accession no. GSN:ADH69807 Database accession no. ADH69807**
- **DATABASE EMBL [Online] 15 juin 1991 (1991-06-15), "Human T-cell receptor germline gamma-chain J2 gene." XP002535777 extrait de EBI accession no. EMBL:M12961 Database accession no. M12961**
- **DATABASE EMBL [Online] 14 août 1991 (1991-08-14), "Human J6 to enhancer DNA of the immunoglobulin heavy-chain gene locus" XP002535778 extrait de EBI accession no. EMBL: X54712 Database accession no. X54712**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

• DATABASE Geneseq [Online] 25 août 2005 (2005-08-25), "Human TRG gene genomic sequence SEQ ID NO:97." XP002535779 extrait de EBI accession no. GSN:AEA61187 Database accession no. AEA61187

SEQUENCE LIFTING

<110> IMMUNID COMMISSARIAT A L'ENERGIE ATOMIQUE PASQUAL, Nicolas WEISBUCH, sébastien

<120> PROCEDE D'ETUDE DE LA DIVERSITE COMBINATOIRE V(D)J.

<130> VMAahF2110/1

<160> 119

<170> PatentIn version 3.3

<210> 1
<211> 25
<212> DNA
<213> Artificial

<220>
<223> amorce

<400> 1
cttggtgcat ggctatgtaa tcctg 25

<210> 2
<211> 22
<212> DNA
<213> Artificial

<220>
<223> amorce

<400> 2
ctcgccctct gctcagcttt cc 22

<210> 3
<211> 26

<212> DNA
<213> Artificial

<220>
<223> amorce

<400> 3
cacacagatg ggacaggaag tgatct 26

<210> 4
<211> 23
<212> DNA
<213> Artificial

<220>
<223> amorce

<400> 4
gcttctcacc tgaatgcccc aac 23

<210> 5
<211> 25
<212> DNA
<213> Artificial

<220>
<223> amorce

<400> 5
ctgatcaaaa cgagaggaca gcaag 25

<210> 6
<211> 25
<212> DNA
<213> Artificial

<220>
<223> amorce

<400> 6
ctgatcaaaa cgagaggaca gcacg 25

<210> 7
<211> 23
<212> DNA
<213> Artificial

<220>
<223> amorce

<400> 7
gatcacccag gcaccaacat ctc 23

<210> 8
<211> 25
<212> DNA
<213> Artificial

<220>
<223> amorce

<400> 8
cagatcacac aggagctgga gtctc 25

<210> 9
<211> 27
<212> DNA
<213> Artificial

<220>
<223> amorce

<400> 9
cacagatcac gcagatactg gagtctc 27

<210> 10
<211> 23
<212> DNA
<213> Artificial

<220>
<223> amorce

<400> 10
cgcacaacag ttccctgact tgc 23

<210> 11
<211> 27
<212> DNA
<213> Arti fi ci al

<220>
<223> amorce

<400> 11
ttcacagttg cctaaggatc gattttc 27

<210> 12
<211> 27
<212> DNA
<213> Artificial

<220>
<223> amorce

<400> 12
ttctctggta cagacagacc tttgtgc 27

<210> 13
<211> 27
<212> DNA
<213> Artificial

<220>
<223> amorce

<400> 13
ttttctggta cagagatacc ttcgtgc 27

<210> 14
<211> 25
<212> DNA
<213> Artificial

<220>
<223> amorce

<400> 14
gttgctgaag tgtcaaactc tcccg 25

<210> 15
<211> 24
<212> DNA
<213> Artificial

<220>
<223> amorce

<400> 15
tccccagcca cagcgtaata gaga 24

<210> 16
<211> 24
<212> DNA
<213> Artificial

<220>
<223> amorce

<400> 16
ccccaaagct gctgttccac tact 24

<210> 17
<211> 22
<212> DNA
<213> Artificial

<220>
<223> amorce

<400> 17
ctcctggtga agaagtcgcc ca 22

<210> 18
<211> 22
<212> DNA
<213> Artificial

<220>
<223> amorce

<400> 18
tagtgcgagg agattcggca gc 22

<210> 19
<211> 23
<212> DNA
<213> Artificial

<220>
<223> amorce

<400> 19
ctgggagcaa gtgagtcctg ggt 23

<210> 20
<211> 24
<212> DNA
<213> Artificial

<220>
<223> amorce

<400> 20
tcatcaacca tgcaagcctg acct 24

<210> 21
<211> 24
<212> DNA
<213> Artificial

<220>
<223> amorce

<400> 21
agtgtctctc gacaggcaca ggct 24

<210> 22
<211> 23
<212> DNA
<213> Artificial

<220>
<223> amorce

<400> 22
cctctttgtt gggtttgtgc ctg 23

<210> 23
<211> 22
<212> DNA
<213> Artificial

<220>
<223> amorce

<400> 23
gtccccttcc tttacaggcc cc 22

<210> 24
<211> 21
<212> DNA

<213> Artificial

<220>
<223> amorce

<400> 24
ccatcagccg cccaaaccta a 21

<210> 25
<211> 26
<212> DNA
<213> Artificial

<220>
<223> amorce

<400> 25
tgctcttcta ctccgttggt attggc 26

<210> 26
<211> 25
<212> DNA
<213> Artificial

<220>
<223> amorce

<400> 26
ttctctataa ggacatgccc caacg 25

<210> 27
<211> 23
<212> DNA
<213> Artificial

<220>
<223> amorce

<400> 27
ttggagaggg gtgggtactg gag 23

<210> 28
<211> 26
<212> DNA
<213> Artificial

<220>
<223> amorce

<400> 28
ctcccaccca cttcactata aatgcc 26

<210> 29
<211> 21
<212> DNA
<213> Artificial

<220>

<223> amorce

<400> 29
gagcaggtgg gcacagtgag c 21

<210> 30
<211> 24
<212> DNA
<213> Artificial

<220>
<223> amorce

<400> 30
tcccccaagt attgcatttg gatt 24

<210> 31
<211> 25
<212> DNA
<213> Artificial

<220>
<223> amorce

<400> 31
aactcaacag ggtccttgcc actta 25

<210> 32
<211> 28
<212> DNA
<213> Artificial

<220>
<223> amorce

<400> 32
ccacccacat ttgatgtttt tatttctt 28

<210> 33
<211> 24
<212> DNA
<213> Artificial

<220>
<223> amorce

<400> 33
tagtgtctcc tctcccgtgc agtc 24

<210> 34
<211> 28
<212> DNA
<213> Artificial

<220>
<223> amorce

<400> 34

gttccagtcc caaaggttaa tttctcat 28

<210> 35
<211> 24
<212> DNA
<213> Artificial

<220>
<223> amorce

<400> 35
agaacaagct ggaggcaact aggc 24

<210> 36
<211> 28
<212> DNA
<213> Artificial

<220>
<223> amorce

<400> 36
aacaccagtc tgatctctca tttttgct 28

<210> 37
<211> 29
<212> DNA
<213> Artificial

<220>
<223> amorce

<400> 37
caagactaaa ggagttaatt catctcccc 29

<210> 38
<211> 24
<212> DNA
<213> Artificial

<220>
<223> amorce

<400> 38
aatccctctg atgggcacca tatc 24

<210> 39
<211> 20
<212> DNA
<213> Artificial

<220>
<223> amorce

<400> 39
acatgggtgg gatggggtca 20

<210> 40

<211> 20
<212> DNA
<213> Artificial

<220>
<223> amorce

<400> 40
tgggagtaaa gggctggggc 20

<210> 41
<211> 25
<212> DNA
<213> Artificial

<220>
<223> amorce

<400> 41
aacctcaatt ccaggcagca gtatc 25

<210> 42
<211> 26
<212> DNA
<213> Artificial

<220>
<223> amorce

<400> 42
ggtcgttttt cttcattcct tagtcg 26

<210> 43
<211> 22
<212> DNA
<213> Artificial

<220>
<223> amorce

<400> 43
tccccttccc attttccact cg 22

<210> 44
<211> 23
<212> DNA
<213> Artificial

<220>
<223> amorce

<400> 44
ccctgtttat ccctgccgac aga 23

<210> 45
<211> 24
<212> DNA
<213> Artificial

<220>
<223> amorce

<400> 45
ctggatgcag acacaaagca aagc 24

<210> 46
<211> 27
<212> DNA
<213> Artificial

<220>
<223> amorce

<400> 46
aatgaagatg gaaggtttac agcacag 27

<210> 47
<211> 28
<212> DNA
<213> Artificial

<220>
<223> amorce

<400> 47
acaaagaaga tggaaggttt acagcaca 28

<210> 48
<211> 22
<212> DNA
<213> Artificial

<220>
<223> amorce

<400> 48
cgccaacctt gtcatctccg ct 22

<210> 49
<211> 27
<212> DNA
<213> Artificial

<220>
<223> amorce

<400> 49
ctagagagag catcaaaggc ttcactg 27

<210> 50
<211> 22
<212> DNA
<213> Artificial

<220>
<223> amorce

<400> 50
cgggcagcag acactgcttc tt 22

<210> 51
<211> 24
<212> DNA
<213> Artificial

<220>
<223> amorce

<400> 51
tcgtcggaac tcttttgatg agca 24

<210> 52
<211> 24
<212> DNA
<213> Artificial

<220>
<223> amorce

<400> 52
tgcctcgctg gataaatcat cagg 24

<210> 53
<211> 21
<212> DNA
<213> Artificial

<220>
<223> amorce

<400> 53
cccagaccac agactcaggc g 21

<210> 54
<211> 28
<212> DNA
<213> Artificial

<220>
<223> amorce

<400> 54
cgtccagatg tgagtgaaaa gaaagaag 28

<210> 55
<211> 27
<212> DNA
<213> Artificial

<220>
<223> amorce

<400> 55
tggacatccc gttttttga tacagtt 27

<210> 56
<211> 27
<212> DNA
<213> Artificial

<220>
<223> amorce

<400> 56
tggtgacagt agttacgggt ggagaag 27

<210> 57
<211> 24
<212> DNA
<213> Artificial

<220>
<223> amorce

<400> 57
agcaaaattc accatccctg agcg 24

<210> 58
<211> 24
<212> DNA
<213> Artificial

<220>
<223> amorce

<400> 58
tgaagggtgg agaacagaag ggtc 24

<210> 59
<211> 27
<212> DNA
<213> Artificial

<220>
<223> amorce

<400> 59
ggctgggaag tttggtgata tagtgtc 27

<210> 60
<211> 27
<212> DNA
<213> Artificial

<220>
<223> amorce

<400> 60
acatttttct acacaggggt gagcagt 27

<210> 61
<211> 28
<212> DNA

<213> Artificial

<220>
<223> amorce

<400> 61
gccctcctga aaatgtgtaa agaaatgt 28

<210> 62
<211> 26
<212> DNA
<213> Artificial

<220>
<223> amorce

<400> 62
acatatgagc cctttatgga agtccg 26

<210> 63
<211> 30
<212> DNA
<213> Artificial

<220>
<223> amorce

<400> 63
tattatactt acgcaagcac aaggaacaac 30

<210> 64
<211> 28
<212> DNA
<213> Artificial

<220>
<223> amorce

<400> 64
tgtactatga ctcctacacc tccagcgt 28

<210> 65
<211> 23
<212> DNA
<213> Artificial

<220>
<223> amorce

<400> 65
aagggggaac gaagtcagtc acg 23

<210> 66
<211> 26
<212> DNA
<213> Artificial

<220>

<223> amorce

<400> 66
gtgttggaat caggaatcag tcgaga 26

<210> 67
<211> 23
<212> DNA
<213> Artificial

<220>
<223> amorce

<400> 67
tgcctcctta gatggaggat gcc 23

<210> 68
<211> 26
<212> DNA
<213> Artificial

<220>
<223> amorce

<400> 68
gcaaggaggc acgcatacta gttagc 26

<210> 69
<211> 28
<212> DNA
<213> Artificial

<220>
<223> amorce

<400> 69
acaaagaaga tggaaggttt acagcaca 28

<210> 70
<211> 22
<212> DNA
<213> Artificial

<220>
<223> amorce

<400> 70
cgccaacctt gtcatctccg ct 22

<210> 71
<211> 27
<212> DNA
<213> Artificial

<220>
<223> amorce

<400> 71

ctagagagag catcaaaggc ttcactg 27

<210> 72
<211> 22
<212> DNA
<213> Artificiel

<220>
<223> amorce

<400> 72
cgggcagcag acactgcttc tt 22

<210> 73
<211> 24
<212> DNA
<213> Artificial

<220>
<223> amorce

<400> 73
tgcctcgctg gataaatcat cagg 24

<210> 74
<211> 21
<212> DNA
<213> Artificial

<220>
<223> amorce

<400> 74
caggagggag ccaattccac g 21

<210> 75
<211> 28
<212> DNA
<213> Artificial

<220>
<223> amorce

<400> 75
cgtccagatg tgagtgaaaa gaaagaag 28

<210> 76
<211> 27
<212> DNA
<213> Artificial

<220>
<223> amorce

<400> 76
tggacatccc gtttttttga tacagtt 27

<210> 77

<211> 24
<212> DNA
<213> Artificial

<220>
<223> amorce

<400> 77
agcaaaattc accatccctg agcg 24

<210> 78
<211> 24
<212> DNA
<213> Artificial

<220>
<223> amorce

<400> 78
tgaagggtgg agaacagaag ggtc 24

<210> 79
<211> 27
<212> DNA
<213> Artificial

<220>
<223> amorce

<400> 79
ggctgggaag tttggtgata tagtgtc 27

<210> 80
<211> 26
<212> DNA
<213> Artificial

<220>
<223> amorce

<400> 80
agtgaagaca aggtggtaca aagccc 26

<210> 81
<211> 26
<212> DNA
<213> Artificial

<220>
<223> amorce

<400> 81
gggaggaaca ggattattgg ggtaac 26

<210> 82
<211> 28
<212> DNA
<213> Artificial

<220>
<223> amorce

<400> 82
gccctcctga aaatgtgtaa agaaatgt 28

<210> 83
<211> 25
<212> DNA
<213> Artificial

<220>
<223> amorce

<400> 83
cagtatccat gccagtgagg aaagc 25

<210> 84
<211> 24
<212> DNA
<213> Artificial

<220>
<223> amorce

<400> 84
gacaaagtaa cccagagttc cccg 24

<210> 85
<211> 25
<212> DNA
<213> Artificial

<220>
<223> amorce

<400> 85
gatcttgcag tcctacagac accgc 25

<210> 86
<211> 22
<212> DNA
<213> Artificial

<220>
<223> amorce

<400> 86
gacaagggct tgagtggatg gg 22

<210> 87
<211> 22
<212> DNA
<213> Artificial

<220>
<223> amorce

<400> 87
catggaccct gtggacacag cc 22

<210> 88
<211> 24
<212> DNA
<213> Artificial

<220>
<223> amorce

<400> 88
tgtttgcagg tgtccagtgt gagg 24

<210> 89
<211> 25
<212> DNA
<213> Artificial

<220>
<223> amorce

<400> 89
gaaccagttc tccctgaagc tgagc 25

<210> 90
<211> 21
<212> DNA
<213> Artificial

<220>
<223> amorce

<400> 90
tgcagtggag cagcctgaag g 21

<210> 91
<211> 23
<212> DNA
<213> Artificial

<220>
<223> amorce

<400> 91
agcagcattc acagactgag ggg 23

<210> 92
<211> 23
<212> DNA
<213> Artificial

<220>
<223> amorce

<400> 92
gattctgaac agccccgagt cac 23

<210> 93
<211> 22
<212> DNA
<213> Artificial

<220>
<223> amorce

<400> 93
ggacaggagg attttgtggg gg 22

<210> 94
<211> 20
<212> DNA
<213> Artificial

<220>
<223> amorce

<400> 94
aggtcagccc tggacatccc 20

<210> 95
<211> 19
<212> DNA
<213> Artificiel

<220>
<223> amorce

<400> 95
atccccagga cgcagcacc 19

<210> 96
<211> 20
<212> DNA
<213> Artificial

<220>
<223> amorce

<400> 96
agctcctcct gacagccccg 20

<210> 97
<211> 21
<212> DNA
<213> Artificiel

<220>
<223> amorce

<400> 97
acaccagaca gaggggcagg c 21

<210> 98
<211> 20
<212> DNA

<213> Artificial

<220>
<223> amorce

<400> 98
agaccgcagc cacatcagcc 20

<210> 99
<211> 23
<212> DNA
<213> Artificial

<220>
<223> amorce

<400> 99
gtggctgttc actctgcgga gtc 23

<210> 100
.<211> 28

<212> DNA
<213> Artificial

<220>
<223> amorce

<400> 100
tcaaaaactt atggacaatc agactgcc 28

<210> 101
<211> 26
<212> DNA
<213> Artificial

<220>
<223> amorce

<400> 101
caggacccaa agtcttacag atccca 26

<210> 102
<211> 25
<212> DNA
<213> Artificial

<220>
<223> amorce

<400> 102
ttgtaaacga aacagttcca aggcg 25

<210> 103
<211> 24
<212> DNA
<213> Artificial

<220>
<223> amorce

<400> 103
ttggaatgtg agcaacatct ggga 24

<210> 104
<211> 25
<212> DNA
<213> Artificial

<220>
<223> amorce

<400> 104
cccagcagat tctcagtcca acagt 25

<210> 105
<211> 22
<212> DNA
<213> Artificial

<220>
<223> amorce

<400> 105
tggaggctgc agtcacccaa ag 22

<210> 106
<211> 28
<212> DNA
<213> Artificial

<220>
<223> amorce

<400> 106
gttatagata attcacagtt gccctcgg 28

<210> 107
<211> 24
<212> DNA
<213> Artificial

<220>
<223> amorce

<400> 107
ttccgtgttc ataactccac agcg 24

<210> 108
<211> 23
<212> DNA
<213> Artificial

<220>
<223> amorce

<400> 108
ctgaagatcc agagcacgca acc 23

<210> 109
<211> 25
<212> DNA
<213> Artificial

<220>
<223> amorce

<400> 109
ttttgagaag ttccaatcca gtcgg 25

<210> 110
<211> 27
<212> DNA
<213> Artificial

<220>
<223> amorce

<400> 110
cgatctatct gaaggctatg atgcgtc 27

<210> 111
<211> 25
<212> DNA
<213> Artificial

<220>
<223> amorce

<400> 111
ctgtagcttg gtatcgtcaa tcgcc 25

<210> 112
<211> 27
<212> DNA
<213> Artificial

<220>
<223> amorce

<400> 112
aacacaccca aataattttc cttgctg 27

<210> 113
<211> 27
<212> DNA
<213> Artificial

<220>
<223> amorce

<400> 113
tggaaatcct atcctctgaa gaagacg 27

<210> 114
<211> 24
<212> DNA
<213> Artificial

<220>
<223> amorce

<400> 114
tctttgacct ggagattgcc aacc 24

<210> 115
<211> 24
<212> DNA
<213> Artificial

<220>
<223> amorce

<400> 115
atacagggtc tcacggaaga agcg 24

<210> 116
<211> 23
<212> DNA
<213> Artificial

<220>
<223> amorce

<400> 116
atggcaactg caaatgaagg ctc 23

<210> 117
<211> 24
<212> DNA
<213> Artificial

<220>
<223> amorce

<400> 117
acgaccaatt catcctaagc acgg 24

<210> 118
<211> 26
<212> DNA
<213> Artificial

<220>
<223> amorce

<400> 118
gcatggctat ttgaaacagt ggctct 26

<210> 119
<211> 22
<212> DNA

<213> Artificial

<220>
<223> amorce

<400> 119
ccttgtcctg gcttgcgaga ga 22

## Description

La présente invention concerne le domaine de l'immunologie. Plus précisément, la présente invention est relative à une méthode d'analyse *in vitro* de la diversité du répertoire des lymphocytes T et/ou B d'un individu et à ses applications, notamment dans le suivi d'un traitement ou dans le diagnostic et/ou le pronostic de certaines pathologies.

Un lymphocyte, pour être fonctionnel, doit avoir un système de reconnaissance spécifique de l'antigène. Ce paramètre est essentiel : il définit la fonction même de la cellule T ou B. Au cours des étapes précoces de la différenciation de lymphocytes T, les loci codant les chaînes clonotypiques du récepteur TCR subissent des réarrangements pour permettre l'expression d'un récepteur fonctionnel. De même, dans les lymphocytes B, les loci codant les chaînes des immunoglobulines (IG) subissent des réarrangements pour permettre l'expression d'une IG fonctionnelle.

Le mécanisme de réarrangement V(D)J est spécifique aux lymphocytes T et B. Les gènes V, D et J codant pour le TCR sont répartis sur de longues portions germinales au sein des différents loci du TCR. Pour donner une protéine, ces gènes doivent être associés en un exon par un processus de réarrangement des gènes, appelé recombinaison V(D)J. Le principe de la recombinaison est basé sur la reconnaissance de séquences RSS spécifiques des gènes V(D)J et l'excision de la région chromosomique intercalée entre les deux gènes réarrangés. Chaque gène V et J possède, à une de ses extrémités, une séquence signal de recombinaison (RSS). Quant aux gènes D, ils en possèdent aux deux extrémités. Les RSS sont des séquences reconnues par les enzymes spécifiques de la recombinase, RAG I et RAG II, exprimées spécifiquement dans les lymphocytes. Ces protéines sont les actrices principales du réarrangement. Une fois associées aux protéines HMG (*High mobility group*), les enzymes RAG reconnaissent le nonamère du RSS grâce à leur homéodomaine et induisent une coupure entre le segment de gène V, D, J et l'heptamère, de façon à générer une extrémité codante et une extrémité signal. L'achèvement d'un réarrangement est obtenu après ligation des deux extrémités codantes V et J. Cette étape est précédée par l'action de l'enzyme TdT et d'une nucléase au niveau de la jonction V-J. Une fois réarrangé, le gène néoformé est transcrit puis épissé en ARNm avant d'être traduit en protéine membranaire.

Un TCR donné reconnaît de façon spécifique un nombre limité de peptides antigéniques différents. De ce fait, un vaste répertoire de récepteurs est requis pour assurer la défense de l'individu contre les multiples infections qu'il est susceptible de rencontrer dans son environnement. Pour cela, le système immunitaire a développé un mécanisme d'assemblage d'un grand nombre de segments de gènes V, D, J positionnés de façon discontinue dans le génome. Ce mécanisme « d'assemblage » appelé recombinaison V(D)J est indépendant d'une cellule à l'autre et permet d'obtenir un seul « fragment » de gène codant pour le TCR. Ce système permet, avec un nombre modeste de gènes, de générer un grand nombre de récepteurs différents. Chaque cellule utilise une combinaison de segments géniques selon des règles précises et obtient une chaîne TCR potentiellement unique.

Quatre mécanismes majeurs contribuent à générer la diversité du répertoire : 1) une diversité combinatoire qui correspond à la première étape de réarrangement entre un segment V (un segment D) et un segment J ; 2) une diversité jonctionnelle, générée au niveau de la jonction entre les segments de gènes réarrangés ; 3) des hypermutations somatiques dans les gènes réarrangés V-J et V-D-J ; 4) une diversité d'appariement des hétérodimères protéiques TCR$\alpha$ x TCR$\beta$ ou TCR$\gamma$ x TCR$\delta$.

La première étape de génération de diversité est basée sur le principe du réarrangement des gènes V(D)J (figure 1). Les combinaisons résultantes de l'appariement d'un nombre fixe de gènes V, D et J forment la diversité combinatoire. Le calcul de cette diversité consiste à estimer le nombre de combinaisons mV x nD x pJ possibles. Le mécanisme régulant la recombinaison V(D)J n'est pas aléatoire : il est régulé de façon spatio-temporelle au cours de l'ontogénie (Aude-Garcia et al., 2001; Jouvin-Marche et al., 1998; Pasqual et al., 2002; Rytkonen et al., 1996). Une simple multiplication ne suffit donc pas à estimer le nombre total de combinaisons de gènes attendus. Cette première étape de génération de diversité définit l'ordre de grandeur du répertoire. En effet, même si cette étape ne génère qu'une modeste variabilité de combinaison (de l'ordre de quelques milliers de combinaisons possibles par rapport au répertoire maximal théorique estimé à $10^{15}$ (Davis and Bjorkman, 1988), la diversité combinatoire maximale est directement liée au nombre de gènes V, D et J initialement disponibles : les deux autres étapes de génération de la diversité amplifient de manière exponentielle la diversité du répertoire primaire.

La diversité de jonction permet de générer une très grande variabilité au niveau de la région CDR3 du récepteur en contact avec le peptide antigénique. Deux mécanismes contribuent à augmenter la diversité jonctionnelle : 1) le premier mécanisme est dû à l'ajout de nucléotides P (pour palindromiques), provenant de la résolution de l'épingle à cheveux des segments réarrangés (Fugmann et al., 2000). La diversité générée n'est pas aussi grande que celle provenant du deuxième mécanisme faisant intervenir l'enzyme terminal desoxynucléotidyl transférase ; 2) la TdT produit une importante diversité, en ajoutant de façon aléatoire des nuclétotides N à l'extrémité 3' de chaque segment codant, sans besoin de matrice génomique (Bogue et al., 1992). Des études sur la souris TdT -/- ont permis d'estimer que, chez ces animaux, le répertoire TCR$\alpha\beta$ ne représentait que 5 à 10% du répertoire normal et donc que la TdT contribuait à hauteur de 90% à la génération de la diversité totale du TCR$\alpha\beta$. De plus, ces résultats ont montré que la longueur des CDR3 dans les transcrits TCR$\beta$ était nettement diminuée contrairement au CDR3 des transcrits TCR$\alpha$. Cette observation

vérifie, comme attendu, une contribution plus importante de la TdT sur les recombinaisons V-D-J que sur les réarrangements V-J (Cabaniols et al., 2001).

Le mécanisme des réarrangements secondaires contribue à « conserver » de la diversité : la diversité jonctionnelle représente le plus grand facteur d'amplification de la diversité du répertoire, mais si il n'y avait pas le mécanisme de réarrangement secondaire pour sauver 2/3 des thymocytes ayant interrompu leur cadre de lecture, ce bénéfice en terme de diversité représenterait un coût important pour l'organisme, avant même l'étape de sélection positive. Ces réarrangements non productifs ne peuvent donner une protéine TCR fonctionnelle. La cellule a alors la possibilité de tenter un deuxième réarrangement avec les gènes V(D)J encore disponibles sur le locus. La propriété d'ouverture concentrique du locus TRAD favorise ce processus en laissant le plus de chances possibles à la cellule, puisque les premiers réarrangements effectués par la cellule ont lieu entre un couple de gène V-J proches l'un de l'autre (Huang and Kanagawa, 2001; Pasqual et al., 2002; Wang et al., 1998). Si ces premiers réarrangements ne sont pas productifs, la cellule a la possibilité de tenter des réarrangements sur son second chromosome, ou encore d'utiliser les gènes V et J disponibles de part et d'autre du premier réarrangement. Ainsi, les réarrangements secondaires permettent la survie d'un grand nombre de cellules qui, à l'issue d'un premier réarrangement non productif, auraient dû être éliminées.

Les hypermutations somatiques (HMS) ont lieu pendant la différenciation des lymphocytes B dans les ganglions lymphatiques, lors de la rencontre avec un antigène (Berek et al., 1985). Les HMS sont situées dans des "*hot spot motifs*" des gènes réarrangés V-J et V(D)J des Ig (Chaudhuri et al., 2003; Oprea and Kepler, 1999) mais aussi dans certains cas, dans des gènes réarrangés V-J et V(D)J des TCR (Kotani et al., 2005). Le TCR peut être la cible d'HMS au niveau des gènes variables, si le lymphocyte surexprime l'enzyme AID (*Activation-induced cytidine deaminase*) qui est normalement spécifique des LB. En temps normal le TCR ne subit pas d'HMS car le LT ne synthétise tout simplement pas l'AID Néanmoins, si le lymphocyte T se met à l'exprimer, le TCR est aussi sensible à cette enzyme que le BCR car il possède tous la séquence sur laquelle elle agit. Au total, il est décrit dans la littérature que ce mécanisme induit une diversité supplémentaire d'un facteur 1000 dans le but d'augmenter les chances de reconnaître un antigène.

L'estimation de la diversité issue de l'appariement entre une chaîne TCR$\alpha$ et une chaîne TCR$\beta$ est obtenue en multipliant le nombre de combinaisons différentes d'une chaîne TCR$\alpha$, par le nombre de combinaisons possibles pour la chaîne TCR$\beta$. La diversité générée par ce mécanisme est directement dépendante du nombre de combinaisons primaires obtenu lors du réarrangement. En effet, si on examine le nombre de combinaisons primaires TCR$\gamma\delta$ chez la souris, sans prendre en compte la diversité jonctionnelle, le résultat est seulement de 40 TCR$\delta$ (=10V*2D*2J) x 28 TCR$\gamma$ (=7V*4J) = 1120 combinaisons différentes, alors que le même calcul conduit à 5,6 $10^6$ combinaisons pour TCR$\alpha\beta$ (calculé comme suit : 102V$\alpha$*60J$\alpha$*33V$\beta$*2D$\beta$*14J$\beta$).

Le séquençage complet du génome humain et de la souris a récemment permis d'obtenir les cartes définitives de chacun des loci du TCR et rend dès lors possible de nouvelles approches génétiques pour découvrir les mécanismes de régulation de la recombinaison. Chaque cellule possède 4 loci capables de réarranger des gènes du TCR. Chez l'homme et la souris, les chaînes TCR$\alpha$ et TCR$\delta$ sont réarrangées dans deux loci associés sur le même chromosome 14. Les loci TCR$\gamma$ et TCR$\beta$ humains sont sur les chromosomes 7 (ou 13 chez la souris) et 7 (ou 6 chez la souris), respectivement (voir tableau 1).

Tableau 1 : Tableau comparatif des principales caractéristiques connues des 4 loci chez l'homme et la souris BALB/c. (D'après les données IMGT Lefranc MP).

| Chaîne | **TCR**α | | **TCR**δ | | **TCR**β | | **TGR**γ | |
|---|---|---|---|---|---|---|---|---|
| Espèce | Humain | Souris | Humain | Souris | Humain | Souris | Humain | Souris |
| Chromosome | 14q11.2 | 14c1 | 14q11.2 | 14c1 | 7q34 | 6A-C | 7p14 | 13A2-3 |
| Taille du locus | 1000Kb | 1500Kb | 1000Kb | 1500Kb | 620Kb | 700Kb | 160Kb | 205Kb |
| Nombre de gènes **V** | 54 | 102 | 6+nVa | 10+nVa | 63-67 | 33 | 12-15 | 7 |
| Nombre de familles **V** | 41 | 23 | 6+nVa | 10+nVa | 30 | 30 | 6 | 7 |
| Nombre de gènes **D** | - | - | 3 | 2 | 2 | 2 | - | - |
| Nombre de gènes **J** | 61 | 60 | 4 | 2 | 6+7 | 7+7 | 5 | 4 |
| Nombre de gènes **C** | 1 | 1 | 1 | 1 | 2 | 2 | 2 | 4 |
| Nombre de pseudo V<br>*transcription*<br>*réarrangement* | 8<br>- | 15<br>- | 0<br>0 | 0<br>0 | 12-13<br>- | 13<br>- | | -<br>- |
| Nombre de pseudo J<br>*transcription*<br>*réarrangement (P-R)* | 8<br>8 | 6<br>16 | 0<br>0 | 0<br>0 | 2<br>2 | -<br>2 | 0<br>0 | -<br>- |
| Combinatoire V(D)J maximale Sans les P-R | 54V*(61J-8 PR)= 2915 | 102V *(60J-16PR)= **4488** | 6V*3D*4 J= 72 | 10V*2D*2J= **40** | (67V*D1*6J1*2C )+(67*D1*( 8J2-2PR)*C1+67*D2* (8J2-2PR)*C2= 804+402+402 = **1608** | (33V*D1*7J1-PR*2C)+(33*D 1*(7 J2-1PR)*C1+33*D2*(7 J2-1PR)*C2= 396+198+198=792. | 15*5= **75** | 7*4= **28** |

Remarque #1 : les tirés (-) indiquent l'absence d'études exhaustives permettant de dénombrer avec certitude le nombre de pseudogènes de réarrangement.

Remarque #2 : estimation des combinaisons TCRβ. D'après la structure du locus TCRβ chez l'homme, les gènes réarrangés sur le segment D1 peuvent se réarranger sur les segments J1.1-J1.6 puis sont épissés avec BC1 ou BC2. Ils peuvent également se réarranger sur le second set de segments J2.1-J2.8 mais sont alors uniquement épissés sur BC2. Si c'est le segment D2 qui est utilisé, seul le set J2.1 à J2.8 peut être utilisé et l'épissage se fera sur la chaîne BC2. Il en découle une combinatoire fractionnée en fonction du segment D1 ou D2 choisi lors du réarrangement D-J : (67V*1D1*6J1*2BC)+(67*1D1*(8J2-2PR)*1BC1 +67*1D2*(8J2-2PR)*1BC2= 1608 combinaisons possibles.

La nomenclature des gènes V(D)J a changé à plusieurs reprises pendant ces dernières années. Tout d'abord attribué selon leur ordre de découverte, le nom des gènes est maintenant défini en fonction de leur position dans le locus. La dernière nomenclature en date est celle d'IMGT (http://imgt.cines.fr), qui ordonne logiquement les familles V les unes par rapport aux autres et classe de manière intuitive les membres d'une famille sur le locus. Cette nomenclature IMGT sous entend que tous les gènes V soient connus. Le gène TRAV1 est le plus loin des segments AJ. C'est le gène le plus en amont (du coté 5') de la région V. Plus on se rapproche de la région J et plus le chiffre des familles V augmente : ainsi la famille TRAV41 humaine est la plus proche de la région AJ. Les membres d'une même famille ont un nom composé par le numéro de la famille, puis le numéro du membre. Par exemple TRAV8.1 est le membre le plus en amont (5') de la famille TRAV8 alors que le membre TRAV8.6 est le plus en aval (3') sur la région V. Dans ce qui suit, la nomenclature IMGT est utilisée.

La diversité du répertoire des immunoglobulines produites par les lymphocytes B résulte des mêmes mécanismes que ceux décrits ci-dessus pour les lymphocytes T.

La mesure de la diversité immunologique permet entre autres d'étudier les mécanismes de mise en place du répertoire immunitaire, l'homéostasie, les lymphocytes T ou B impliqués dans une réponse immunitaire, dans une leucémie ou encore d'évaluer l'immunodéficience induite par un traitement ou à l'inverse l'activation du système immunitaire spécifique. Cette liste n'est pas exhaustive.

L'étude du répertoire immunitaire d'une population lymphocytaire a conduit au développement de plusieurs approches multiparamétriques, permettant à la fois de mesurer le niveau de diversité et d'identifier la présence de certains clones T ou B spécifiques. Certaines approches mises au point par les immunologistes pour évaluer ces différents niveaux de diversité sont listées ci-dessous selon le principe et le « niveau » de diversité mesurée.

- **Mesure de la diversité V**

- Par cytométrie (Van den Beemd, van Dongen et al. 2000)
- Par Q-PCR au niveau génomique et transcriptomique (Fuschiotti et al., 2007; Lang et al., 1997; Pasqual et al., 2002).

- **Mesure de la diversité jonctionnelle CDR3 :**

- Par Immunoscope® (Cochet et al., 1992; Pannetier et al., 1995).
- Q-PCR couplé à l'immunoscope (TcLandscape®)
- Par séquençage
- Par la méthode Amplicot au niveau génomique (Baum and McCune, 2006).
- Par puce à ADN (Bonarius et al., 2006).

- **Etude des hypermutations somatiques (HMS) :**

- PCR / séquençage (Hamblin et al., 1999).

- **Mesure indirecte *via* la décroissance des cercles d'excisions TREC's**

- Par PCR (Douek et al., 1998).
- Par Q-PCR (Pham et al., 2003).

Si certaines de ces approches ont déjà fait leurs preuves en recherche fondamentale, notamment l'Immunoscope® (Pannetier, C., J. Even, et al., 1995) ou la cytométrie en flux (Van den Beemd, van Dongen et al., 2000), il reste encore un certain nombre de validations scientifiques et techniques à apporter pour évaluer la pertinence de son utilisation comme bio-marqueur médical. Face à la complexité du système immunitaire, le scientifique aurait besoin de coupler des approches technologiques complémentaires pour décrypter l'ensemble des informations contenues dans le répertoire immunitaire et relevantes d'une pathologie donnée.

D'autres méthodes, basées sur l'utilisation de PCR amplifiant spécifiquement des segments d'acides nucléiques caractéristiques de certains réarrangements, ont été décrites.

Par exemple, les brevets US 5,296,351 et US 5,418,134 présentent une méthode de détection des leucémies lymphoïdes ou des lymphomes B ou T, basée sur l'amplifications de séquences codant pour des immunoglobuline et/ou des récepteurs T, utilisant des amorces "consensus" pour amplifier simultanément plusieurs réarrangements V-J.

La demande WO2005/056828 décrit une méthode basée sur l'utilisation de PCR multiplexes, qui sont des réactions d'amplification en chaîne par polymérase (ACP, ou *Polymerase Chain Reaction, PCR)* dans lesquelles plusieurs fragments différents, correspondant à des réarrangements différents, sont amplifiés avec un même couple d'amorces, à partir d'ADN génomique.

Toutefois, aucune des méthodes décrites ci-dessus ne permet d'obtenir dans un temps raisonnable la résolution des réarrangements V(D)J du répertoire immunitaire, ni même sur l'ensemble des réarrangements intervenus dans un locus donné. En effet, le nombre de PCR multiplexes à effectuer pour cela serait trop élevé pour qu'une utilisation en routine de ces méthodes soit envisageable.

La présente invention est basée sur plusieurs perfectionnements de la méthode décrite dans la demande WO2005/056828, pour permettre l'analyse d'un plus grand nombre de réarrangements V(D)J de façon fiable, facile et rapide. De ce fait, les méthodes de la présente invention constituent des outils de choix pour analyser la diversité du répertoire des lymphocytes B et/ou T dans un échantillon dans de nombreuses applications, telles que le suivi immunitaire (*immunomonitoring*) en phases précliniques ou cliniques, pour étudier l'effet d'un traitement sur la (re)constitution du système immunitaire, le diagnostic personnalisé, le pronostic notamment en onco-hématologie, *etc.*

La présente invention porte donc en premier lieu sur une méthode d'analyse *in vitro* de la diversité du répertoire des lymphocytes T et/ou B d'un individu humain ou animal, à partir d'ADN génomique provenant d'un échantillon biologique dudit individu, comprenant les étapes suivantes :

A) amplification de fragments dudit ADN génomique par des PCR multiplexes, dont l'une au moins est une PCR multi-n-plexe avec n≥2, effectuée avec une combinaison d'au moins 3 amorces, constituant au moins 2 couples d'amorces présentant les caractéristiques suivantes :

(i) trois desdites au moins 3 amorces constituent 2 couples d'amorces distincts ;
(ii) chaque couple d'amorces est constitué d'une amorce s'hybridant spécifiquement en amont et/ou dans un gène V ou D donné et d'une amorce s'hybridant spécifiquement en aval et/ou dans un gène J donné, de façon à permettre l'amplification, avec chaque couple d'amorces, d'au moins deux fragments caractéristiques de deux réarrangements V-J ou D-J distincts;
(iii) les amorces (*i.e.,* toutes les amorces utilisées dans une même réaction d'amplification) sont thermodynamiquement compatibles ;
(iv) les amorces sont choisies de telle façon que les fragments amplifiés avec le premier couple d'amorces peuvent être distingués des fragments amplifiés avec le second couple d'amorces ;

B) détection des produits d'amplification obtenus à l'étape A ;
C) interprétation des résultats.

Pour la mise en oeuvre de cette méthode, l'ADN génomique est de préférence purifié. Toutefois, l'homme du métier peut, en fonction de l'évolution des technologies, choisir de travailler sur des échantillons bruts. Tout échantillon biologique susceptible de contenir des lymphocytes peut être utilisé ; à titre d'exemples non limitatifs d'échantillons utilisables, on peut citer des échantillons de sang, thymus, ganglion, rate, PBMC, foie, peau, urine...

La mise en oeuvre de la méthode ci-dessus nécessite une enzyme Taq polymérase de haute performance. Il est notamment préférable d'utiliser une enzyme ayant la capacité d'amplifier des fragments de haute taille à une vitesse élevée et pouvant "passer" sur des zones riches en GC. De façon encore préférée, l'homme du métier choisira, pour effectuer des réactions de PCR multi-n-plexes dans le cadre de la présente invention, une polymérase présentant les caractéristiques suivantes :

(i) elle est capable d'amplifier des fragments de plusieurs dizaines de kb ;
(ii) sa vitesse d'élongation est d'au moins 1 kb/minute ;

(iii) sa robustesse est telle qu'elle n'introduit pas plus d'une erreur par kb, en moyenne. A titre d'exemples non limitatifs d'enzymes utilisables pour mettre en oeuvre cette méthode, on peut citer les polymérases *HerculaseII* de Stratagène et ou *Iproof* de Biorad.

Dans le présent texte, on appelle "PCR multi-n-plexe" une réaction d'amplification en chaîne par polymérase présentant n degrés de multiplexage, c'est-à-dire mettant en oeuvre, dans une même réaction, n couples d'amorces différents, permettant chacun l'amplification d'au moins deux fragments d'ADN, caractéristiques d'au moins deux réarrangements chromosomiques différents. Par exemple, dans le locus TRA, une réaction de PCR multi-2-plexe peut être réalisée en utilisant une amorce "sens" s'hybridant spécifiquement en amont et/ou dans un gène $V_x$ donné et deux amorces "antisens" s'hybridant spécifiquement en aval et/ou dans deux gènes $J_y$ et $J_z$ distincts, permettant au moins l'amplification, en une seule réaction, de fragments caractéristiques des réarrangements $V_xJ_y$, $V_xJ_{y+1}$, $V_xJ_z$ et $V_xJ_{z+1}$. Bien entendu, une réaction de PCR multi-n-plexe n'est possible que si les amorces des n couples utilisés sont thermodynamiquement compatibles. La compatibilité thermodynamique des amorces est une notion bien connue de l'homme du métier, qui dispose d'algorithmes pour la vérifier. Elle implique notamment que les différentes amorces aient des températures de fusion (Tm) identiques ou proches. En outre, les différentes amorces ne doivent pas s'hybrider entre elles, ni former des épingles à cheveux. Dans la suite de ce texte, les contraintes liées à la compatibilité des amorces pour leur utilisation simultanée dans une PCR multi-n-plexe ne seront pas systématiquement rappelées, étant donné qu'elles font partie des connaissances générales de tout biologiste moléculaire.

Dans le présent texte, une "amorce s'hybridant spécifiquement en aval et/ou dans un gène J donné" pourra être appelée, par abus de langage, "amorce spécifique du gène J". De même, une "amorce s'hybridant spécifiquement en amont et/ou dans un gène V donné" pourra être appelée "amorce spécifique du gène V". S'agissant des gènes V, il est important de noter qu'ils sont, pour la plupart des loci, regroupés en familles, suivant leur degré d'homologie. Dans ce cas, une "amorce spécifique d'un gène V donné" peut en réalité désigner une amorce reconnaissant spécifiquement tous les membres ou certains membres d'une famille de gènes V. Des exemples d'amorces spécifiques d'une famille de gènes V sont présentés ci-après. Par ailleurs, lorsqu'un couple d'amorces permet de détecter les réarrangements de tous les membres ou de certains membres d'une même famille, ces réarrangements pourront être appelés par abus de langage "le réarrangement", "l'amplicon", ou "le produit de PCR".

La méthode ci-dessus peut en particulier être appliquée à l'analyse de la diversité combinatoire des réarrangements V-J d'au moins un locus génétique choisi parmi les loci TRA, TRB, TRG, TRD, IGH, IGK, IGL ...

Dans une mise en oeuvre particulière de la méthode selon l'invention, au moins une amorce de chaque couple d'amorces est marquée. Lorsqu'une amorce est commune à plusieurs couples d'amorces utilisés dans une même PCR multi-n-plexe, l'autre amorce de chaque couple sera préférentiellement marquée. Des marquages différents, permettant de distinguer les produits d'amplification par chacun des couples, peuvent être avantageusement utilisés. L'homme du métier dispose d'une grande variété de marqueurs utilisables pour marquer les amorces, parmi lesquels on peut citer les marqueurs colorimétriques, fluorescents, enzymatiques, radioactifs, la biotine, la streptavidine, *etc.*

Selon une mise en oeuvre particulière de la méthode décrite ci-dessus, l'étape B) comporte une étape de mesure en temps réel de l'amplification des fragments d'ADN ; l'interprétation des courbes d'amplification obtenues est alors effectuée de la façon suivante :

(i) si une ou quelques courbes, en nombre inférieur à la moitié des courbes et en particulier en nombre égal à 1, 2 ou 3, présentent un décalage par rapport aux autres courbes, tel que les autres courbes présentent un point d'inflexion au moins 2 cycles après le point d'inflexion de la première courbe, de préférence au moins 3 ou 4 cycles, ou ne montrent aucune amplification, le résultat indique la présence d'une lymphoprolifération clonale ou oligoclonale ;
(ii) si, au contraire, toutes les courbes présentent un point d'inflexion au même cycle, ou dans un décalage maximum de 2 ou 3 cycles d'amplification, le résultat permet d'écarter l'hypothèse d'une lymphoprolifération d'un clone résultant d'un des réarrangements correspondant aux fragments amplifiés.

Dans cette mise en oeuvre, et suivant la technologie utilisée pour réaliser la PCR quantitative (TaqMan®, mesure de l'incorporation d'un intercalant fluorescent tel que le SYBR-green®, ...), l'amplification mesurée est soit la somme des amplifications réalisées avec tous les couples d'amorces utilisés dans une même réaction (cas de l'utilisation du SYBR-green®), soit, au contraire, l'amplification par chacun des couples d'amorces, séparément (cas de la technologie TaqMan®, en utilisant des amorces marquées différemment pour chacun des couples). Quoi qu'il en soit, les inventeurs ont observé que lorsqu'une population lymphocytaire donnée est sur-représentée dans un échantillon, l'amplification du fragment correspondant au réarrangement intervenu dans ces lymphocytes est efficace, alors que l'amplification des fragments correspondant aux autres réarrangements conduit à une courbe décalée, voire à un défaut d'amplification (courbe plate).

Lorsque la mesure de l'amplification est effectuée avec un intercalant fluorescent, la méthode décrite ci-dessus peut également comporter, une étape (optionnelle) de confirmation d'une lymphoprolifération, par la mesure continue de la fluorescence dans chaque tube au cours d'une montée en température entre 40°C et 95°C, l'observation d'un pic majoritaire étant indicative de la présence d'un amplicon majoritaire et donc d'une lymphoprolifération, alors que l'observation de plusieurs pics de tailles similaires indique au contraire une diversité lymphocytaire.

Cette méthode permet également d'effectuer une étape de mesure de la diversité moléculaire des réarrangements observés au niveau de l'ADN génomique, par mesure de la diversité moléculaire des amplicons. Cette étape optionnelle ajoute un degré d'information supplémentaire, car la "diversité moléculaire" résulte de la combinaison de la diversité de jonction (CDR3), la diversité combinatoire (V-J) et la diversité issue des hypermutations somatiques. Elle peut être mesurée de la façon suivante :

(i) après déshybridation des amplicons à 95°C, la température des produits d'amplification est rapidement ramenée en dessous de 40°C, de préférence à 30°C ou en dessous ; cette baisse de température peut être effectuée dans le thermocycleur, ou en mettant le tube dans la glace pendant quelques minutes ; la chute de température doit être effectuée en un temps réduit, de préférence inférieur à 2 minutes, de façon encore préférée inférieur à 30 secondes.
(ii) la fluorescence est mesurée régulièrement et de préférence en continu au cours de la réhybridation ;
(iii) une réhybridation rapide (de l'ordre de la seconde) est indicative de la présence d'un amplicon majoritaire, et donc d'une lymphoprolifération clonale, alors qu'une réhybridation plus lente (de l'ordre de plusieurs dizaines de secondes, voire minutes) est indicative d'une bonne diversité moléculaire (au moins plusieurs dizaines de molécules, voire plusieurs milliers).

Dans la méthode décrite ci-dessus, l'étape B) de détection des produits d'amplification peut comprendre une étape de séparation desdits produits suivant leur taille. L'homme du métier dispose de plusieurs techniques permettant la séparation des amplicons suivant leur taille. A titre d'exemples non limitatifs, on peut citer l'électrophorèse sur gel d'agarose ou de polyacrylamide, ou l'électrophorèse capillaire, qui présente l'avantage d'être plus facilement automatisable. Les amplicons séparés suivant leur taille peuvent être détectés par tout moyen connu de l'homme du métier, notamment grâce à des marqueurs liés aux amorces, ou en utilisant des intercalants fluorescents tels que le bromure d'éthydium, le SYBR-green®, *etc.* La résolution des amplicons et leur détection permettent d'identifier les différents réarrangements chromosomiques ayant donné lieu à amplification. L'utilisation d'une technique d'amplification semi-quantitative ou quantitative permet en outre de déterminer la fréquence, dans l'échantillon testé, des lymphocytes ayant subi le réarrangement V(D)J correspondant à chacun des amplicons observés. Cette mise en oeuvre de la méthode permet donc d'évaluer finement la diversité combinatoire d'une partie du répertoire immunitaire, cette partie étant d'autant plus grande que le nombre de réarrangements susceptibles de donner lieu à amplification est important, ce dernier paramètre dépendant du nombre de PCR multiplexes réalisées, de leur degré de multiplexage, et du choix des amorces.

L'homme du métier pourra décider de mettre en oeuvre la méthode de l'invention en effectuant une première évaluation de la diversité du répertoire lymphocytaire par simple mesure en temps réel de l'amplification des fragments d'ADN, comme mentionné plus haut, et d'effectuer ensuite une séparation des amplicons suivant leur taille pour obtenir davantage d'informations sur la distribution des différents réarrangements dans la population lymphocytaire. Dans le cas où une lymphoprolifération est détectée par PCR multi-n-plexe quantitative, la deuxième étape de détection, par séparation des amplicons suivant leur taille, permettrait en particulier d'identifier le réarrangement V(D)J présent dans le clone ou les quelques clones prolifératif(s). Il est important de noter que cette démarche ne nécessite pas d'effectuer des PCR supplémentaires, puisqu'il suffit alors d'utiliser les produits des PCR déjà effectuées, pour en séparer les amplicons suivant leur taille. Alternativement, l'homme du métier peut choisir de ne pas effectuer la première étape de recherche d'une lymphoprolifération par PCR en temps réel, et d'effectuer directement la séparation des produits d'amplification. Il obtiendra alors directement le nom et la fréquence (intensité) des différents réarrangements V(D)J ayant eu lieu dans la population lymphocytaire testée.

Dans cette mise en oeuvre de l'invention, les couples d'amorces utilisés en combinaison dans chaque réaction de PCR multi-n-plexe avec $n \geq 2$ sont de préférence choisis de façon à ce que la majorité des amplicons obtenus puissent être résolus suivant leur taille. Par "résolus", on entend ici que chacun des amplicons puisse être observé de façon univoque, suite à leur séparation par la taille par une méthode d'électrophorèse ou toute autre méthode. Lorsque des tailles d'amplicons sont trop proches, il n'est pas toujours possible de les discerner distinctement avec des conditions de séparation compatibles avec les conditions permettant de "résoudre" l'ensemble des autres tailles d'amplicons. Les différents amplicons obtenus avec un couple d'amorces donné correspondent à des réarrangements différents et sont donc de tailles significativement différentes. Il faut donc veiller à ce qu'au moins certains des produits de l'amplification par un couple d'amorces présentent des tailles différentes des produits de l'amplification par l'autre ou les autres couple(s) d'amorces. Bien entendu, les différences de tailles pour obtenir une bonne résolution des bandes dépendent de la technologie utilisée. A titre indicatif, des différences de taille de 10% permettent en général d'obtenir une bonne résolution. Idéalement, les amorces sont choisies pour permettre la résolution de tous les amplicons. Toutefois, si certaines bandes, correspondant à des amplicons obtenus avec différents couples d'amorces dans une même PCR multi-n-plexe, sont trop proches ou se chevauchent, d'autres moyens peuvent être mis en oeuvre pour identifier et quantifier les réarrangements correspondant. Par exemple, des marqueurs distincts peuvent être liés aux amorces correspondantes (au moins à une amorce de chaque couple concerné).

Dans une mise en oeuvre particulière de l'invention, au moins une PCR multi-n-plexe (avec n≥2) est effectuée en utilisant une combinaison d'au moins 3 amorces constituant au moins 2 couples d'amorces comportant une amorce "sens" commune spécifique d'un gène V donné, chaque couple d'amorces comportant en outre une amorce "antisens" spécifique d'un gène J donné. Plus particulièrement, cette méthode peut être avantageusement mise en oeuvre en effectuant plusieurs PCR multi-2-plexes avec des triplets d'amorces constitués chacun d'une amorce sens s'hybridant spécifiquement en amont et/ou dans un gène V donné et de deux amorces antisens s'hybridant spécifiquement en aval et/ou dans deux gènes J distincts.

Une manière alternative de combiner les amorces pour effectuer les PCR multi-n-plexes selon l'invention est de combiner au moins 3 amorces constituant au moins 2 couples d'amorces comportant une amorce antisens commune spécifique d'un gène J donné, chaque couple d'amorces comportant en outre une amorce sens spécifique d'un gène V donné.

Le locus TRB présente une configuration particulière, puisque les gènes J sont disposés en deux groupes (ou *clusters*) éloignés l'un de l'autre, le premier groupe (dans le sens 5' -> 3') comportant les gènes BJ1.1 à BJ1.6, et le second groupe comportant les gènes BJ2.1 à BJ2.7. Tirant parti de cette configuration particulière, les inventeurs ont déterminé des paramètres de choix des amorces pour obtenir une excellente résolution des amplicons correspondant aux réarrangements d'un gène V donné avec l'ensemble ou une partie des gènes J. Selon cette mise en oeuvre particulière de l'invention, au moins une réaction de PCR multi-n-plexe avec n≥2 est effectuée pour analyser certains réarrangements du locus TRB, en utilisant une combinaison d'au moins 3 amorces constituant au moins 2 couples d'amorces présentant les caractéristiques suivantes :

(i) trois desdites au moins 3 amorces constituent 2 couples d'amorces distincts ;

(ii) les deux couples d'amorces comportent une amorce sens commune s'hybridant spécifiquement en amont et/ou dans un gène V donné et comportent chacun une amorce antisens s'hybridant spécifiquement en aval et/ou dans un gène J donné ;

(iii) les deux amorces antisens s'hybrident spécifiquement en aval et/ou dans deux gènes $J_y$ et $J_z$ appartenant à deux groupes distincts de gènes J du locus TRB ; et

(iv) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

Dans ce qui précède, la "distance entre la région d'hybridation de l'amorce antisens spécifique du gène $J_y$ et le début dudit gène $J_y$" désigne la distance entre l'extrémité 3' de ladite région d'hybridation (correspondant donc, dans l'amplicon, à l'extrémité 5' de l'amorce antisens spécifique du gène $J_y$) et la première base codante du gène $J_y$ (située immédiatement après la séquence de recombinaison *RSS*).

Dans une mise en oeuvre préférée de cet aspect de l'invention, $J_y=J_{1.6}$ et $J_z=J_{2.7}$. Si V= $V_x$, on verra donc, dans l'ordre croissant de taille, les bandes caractéristiques des réarrangements $V_xJ_{2.7}$, $V_xJ_{2.6}$, $V_xJ_{2.5}$, $V_xJ_{2.4}$, $V_xJ_{2.3}$, $V_xJ_{2.2}$, $V_xJ_{2.1}$, $V_xJ_{1.6}$, $V_xJ_{1.5}$, $V_xJ_{1.4}$, $V_xJ_{1.3}$, $V_xJ_{1.2}$ et $V_xJ_{1.1}$. Le cas échéant, si la polymérase utilisée est particulièrement performante, on observera également une bande de haut poids moléculaire correspondant aux réarrangements $V_xJ_{1.n}$ amplifiés avec l'amorce s'hybridant en aval du gène $J_{2.7}$. Alternativement, la méthode peut être mise en oeuvre avec $J_y=J_{2.7}$ et $J_z=J_{1.6}$ ce qui conduira à observer, dans l'ordre croissant, les bandes caractéristiques des réarrangements $V_xJ_{1.6}$ $V_xJ_{1.5}$, $V_xJ_{1.4}$, $V_xJ_{1.3}$, $V_xJ_{1.2}$, $V_xJ_{1.1}$, $V_xJ_{2.7}$, $V_xJ_{2.6}$, $V_xJ_{2.5}$, $V_xJ_{2.4}$, $V_xJ_{2.3}$, $V_xJ_{2.2}$ et $V_xJ_{2.1}$.

Selon une mise en oeuvre préférée de la méthode décrite ci-dessus pour analyser certains réarrangements du locus TRB, au moins une réaction de PCR multi-n-plexe avec n≥2 est effectuée en utilisant une combinaison d'au moins 3 amorces comprenant les amorces hTRBJ1.6 et hTRJB2.7 définies de la façon suivante :

- hTRBJ1.6 (CTTGGTGCATGGCTATGTAATCCTG, SEQ ID No : 1) est un oligonucléotide antisens de 25 nucléotides s'hybridant entre les nucléotides 2341 et 2365 du gène J1.6 du locus TCRB ; et
- hTRBJ2.7 (CTCGCCCTCTGCTCAGCTTTCC, SEQ ID No : 2) est un oligonucléotide antisens de 22 nucléotides s'hybridant entre les nucléotides 214 et 235 du gène J2.7 du locus TCRB.

Une méthode pour déterminer la position des différentes amorces décrites dans le présent texte par rapport à différents gènes des loci TCR ou IgH est expliquée dans l'exemple 1 ci-après. Les séquences génomiques divulguées dans la base de données "*Ensembl Genome Browser*" peuvent être utilisées pour identifier des amorces utilisables dans le cadre de la présente invention.

Les inventeurs ont identifié, dans le locus TRB, 24 familles de gènes V fonctionnels. Ils ont également montré qu'en effectuant au moins 23 PCR multi-2-plexes, il est possible d'analyser au moins 75% des réarrangements V(D)J du locus TRB, impliquant plus de 85% des familles V. L'invention concerne donc plus particulièrement une méthode permettant l'analyse d'au moins 75%, de préférence au moins 80% des réarrangements V(D)J du locus TRB en effectuant 23 ou 24 PCR multi-n-plexes avec n≥2 à l'aide de combinaisons d'au moins 3 amorces, chaque combinaison d'amorces comprenant les amorces hTRBJ1.6, hTRBJ2.7 définies ci-dessus et au moins une amorce hTRBV choisie parmi les amorces définies dans le tableau ci-dessous :

Tableau 2

| Nom du gène | Nom de l'amorce | Taille (nt) | Distance entre l'extrémité 5' de l'amorce et la fin du gène V (pb) | Séquence | SEQ ID N° |
|---|---|---|---|---|---|
| TRBV2 | hTRBV2up2 | 26 | 255 | CACACAGATGGGAC AGGAAGTGATCT | 3 |
| TRBV4 | hTRBV4up_ex | 23 | 100 | GCTTCTCACCTGAAT GCCCCAAC | 4 |
| TRBV5.1, 3, 4, 5, 6, 8 | hTRBV5up_ex1/2 | 25 | 256 | CTGATCAAAACGAG AGGACAGCAAG | 5 |
| TRBV5.7 | hTRBV5up_ex2/2 | 25 | 256 | CTGATCAAAACGAG AGGACAGCACG | 6 |
| TRBV6.4 | hTRBV6up_ex2/2 | 23 | 279 | GATCACCCAGGCAC CAACATCTC | 7 |
| TRBV7.2 | hTRBV7up_ex2/3 | 25 | 301 | CAGATCACACAGGA GCTGGAGTCTC | 8 |
| TRBV7.9 | hTRBV7up_ex3/3 | 27 | 303 | CACAGATCACGCAG ATACTGGAGTCTC | 9 |
| TRBV9 | hTRBV9up_ex | 23 | 92 | CGCACAACAGTTCCC TGACTTGC | 10 |
| TRBV11 | HTRBV11up_ex | 27 | 120 | TTCACAGTTGCCTAA GGATCGATTTTC | 11 |
| TRBV12.1 | hTRBV12.1up1 | 27 | 196 | TTCTCTGGTACAGAC AGACCTTTGTGC | 12 |
| TRBV12.2 | hTRBV12.2up1 | 27 | 196 | TTTTCTGGTACAGAG ATACCTTCGTGC | 13 |
| TRBV13 | hTRBV13up1 | 25 | 356 | GTTGCTGAAGTGTCA AACTCTCCCG | 14 |
| TRBV14 | hTRBV14up_ex | 24 | 271 | TCCCCAGCCACAGC GTAATAGAGA | 15 |
| TRBV15 | hTRBV15up_ex | 24 | 163 | CCCCAAAGCTGCTGT TCCACTACT | 16 |
| TRBV16 | hTRBV16up1 | 22 | 295 | CTCCTGGTGAAGAA GTCGCCCA | 17 |
| TRBV18 | hTRBV18up1 | 22 | 46 | TAGTGCGAGGAGAT TCGGCAGC | 18 |
| TRBV19 | hTRBV19 up 2 | 24 | 217 | CTGGGAGCAAGTGA GTCCTGGGT | 19 |
| TRBV20 | hTRBV20-1up_ex | 24 | 91 | TCATCAACCATGCAA GCCTGACCT | 20 |
| TRBV24 | hTRBV24up_ex | 24 | 96 | AGTGTCTCTCGACAG GCACAGGCT | 21 |

(suite)

| Nom du gène | Nom de l'amorce | Taille (nt) | Distance entre l'extrémité 5' de l'amorce et la fin du gène V (pb) | Séquence | SEQ ID N° |
|---|---|---|---|---|---|
| TRBV25 | hTRBV25up_int | 23 | 273 | CCTCTTTGTTGGGTT TGTGCCTG | 22 |
| TRBV27 | hTRBV27up2 | 22 | 312 | GTCCCCTTCCTTTAC AGGCCCC | 23 |
| TRBV29 | hTRBV29up_G | 21 | 91 | CCATCAGCCGCCCA AACCTAA | 24 |
| TRBV30 | hTRBV30up1 | 26 | 148 | TGCTCTTCTACTCCG TTGGTATTGGC | 25 |

Cette mise en oeuvre de l'invention permet une analyse d'au moins 80% des réarrangements V(D)J du locus TRB, c'est-à-dire, pour plus de 85% des familles de gènes V fonctionnels, la détermination de la fréquence d'utilisation des gènes de chaque famille V fonctionnelle avec chaque famille J fonctionnelle de ce locus (sans information sur la nature du gène D utilisé dans ces réarrangements, ni sur l'utilisation de chaque membre d'une famille V donnée, ni sur la diversité jonctionnelle des réarrangements, *etc.*). Le couplage avec une mesure en temps réel de l'amplificationpar PCR multi-n-plexe permet en outre d'estimer la diversité moléculaire.

Selon une autre mise en oeuvre particulière de la méthode de l'invention, cette méthode permet la détection *in vitro* de réarrangements incomplets D-J dans un locus génétique choisi parmi les loci TRB, et IGH. La détection des réarrangements incomplets est importante, car même s'ils sont non fonctionnels, ils constituent dans certains cas la seule signature d'une population lymphoproliférative.

Cette méthode peut en particulier être adaptée pour analyser des réarrangements incomplets DJ du locus TRB humain, en effectuant au moins une réaction de PCR multi-n-plexe avec n≥2 avec une combinaison d'au moins 3 amorces constituant au moins 2 couples d'amorces présentant les caractéristiques suivantes :

(i) les deux couples d'amorces comportent une amorce sens commune s'hybridant spécifiquement en amont et/ou dans un gène D donné et comportent chacun une amorce antisens s'hybridant spécifiquement en aval et/ou dans un gène J donné ;
(ii) les deux amorces antisens s'hybrident spécifiquement en aval et/ou dans deux gènes $J_y$ et $J_z$ appartenant à deux groupes distincts de gènes J du locus TRB ; et
(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

En particulier, les amorces hTRBJ1.6 et hTRJB2.7 définies plus haut peuvent être combinées à une amorce sens s'hybridant spécifiquement en amont et/ou dans un gène D donné pour effectuer une réaction de PCR multi-n-plexe avec n≥2.

Du fait de la configuration du locus TRB, il est possible d'analyser tous les réarrangements incomplets DJ de ce locus avec seulement 2 PCR multi-2-plexes.

Ainsi, il est possible d'analyser tous les réarrangements incomplets du locus TRB humain en effectuant (i) une PCR multi-2-plexe à l'aide d'un triplet d'amorces constitué des amorces hTRBJ1.6, hTRJB2.7 et d'une amorce hTRDB1, et (ii) une PCR multiplexe simple à l'aide du couple d'amorces constitué des amorces hTRJB2.7 et d'une amorce hTRDB2, en choisissant par exemple les amorces hTRDB1 et hTRDB2 parmi les amorces définies dans le tableau ci-dessous :

Tableau 3

| **nom du gène** | **nom oligonucléotide** | **taille (nt)** | **Distance entre l'extrémité 5' de l'amorce et la fin du gène D (pb)** | **Séquence** | **SEQ ID No :** |
|---|---|---|---|---|---|
| TRBD1 | hTRBD1up1 | 25 | 325 | TTCTCTATAAGGACATGCCCCAACG | 26 |

(suite)

| nom du gène | nom oligonucléotide | taille (nt) | Distance entre l'extrémité 5' de l'amorce et la fin du gène D (pb) | Séquence | SEQ ID No : |
|---|---|---|---|---|---|
| TRBD1 | hTRBD1up2 | 23 | 289 | TTGGAGAGGGGTGGGTACTGGAG | 27 |
| TRBD2 | hTRBD2up1 | 26 | 322 | CTCCCACCCACTTCACTATAAATGCC | 28 |
| TRBD2 | hTRBD2up2 | 21 | 290 | GAGCAGGTGGGCACAGTGAGC | 29 |

Lorsque l'analyse des réarrangements incomplets du locus TRB est couplée à l'analyse d'autres réarrangements, les PCR multiplexes décrites ci-dessus, en particulier la PCR multiplexe simple, peuvent être combinées à d'autres réactions d'amplification, effectuées dans un même tube (augmentant ainsi le degré de multiplexage et diminuant le nombre de réactions nécessaires pour analyser un nombre de réarrangements donné).

Selon une mise en oeuvre préférée de l'invention, la méthode combine l'analyse des réarrangements V(D)J du locus TRB et celle des réarrangements incomplets de ce locus, par la mise en oeuvre des variantes appropriées décrites plus haut. Les amorces décrites plus haut pour cette analyse sont adaptées à l'analyse des réarrangements de ce locus chez l'humain, mais cette méthode peut être transposée, sans aucune difficulté, chez l'animal, par exemple chez la souris. Des amorces utilisables chez la souris sont décrites, à titre d'exemple, dans la partie expérimentale qui suit.

Selon un autre aspect de l'invention, la méthode permet l'analyse des réarrangements de 95% des gènes J du locus TRA humain avec un gène V donné du même locus, en effectuant, à l'étape A), entre 3 et 6 PCR multi-n-plexes avec n≥2, avec des combinaisons d'amorces constituées chacune d'une amorce s'hybridant en amont et/ou dans ledit gène V et d'un ou deux couple(s) d'amorces antisens choisi(s) parmi les couples (hTRAJ56, hTRAJ41), (hTRAJ37, hTRAJ33), (hTRAJ48, hTRAJ29), (hTRAJ24, hTRAJ18), (hTRAJ53, hTRAJ11) et (hTRAJ7, hTRAJ3), lesdites amorces étant définies dans le tableau ci-dessous :

Tableau 4

| nom du gène | nom oligonucléotide | taille (nt) | distance avec le debut du gène J en pb | Séquence | SEQ ID No : |
|---|---|---|---|---|---|
| hTRAJ56 | hTRAJ56do | 24 | 883 | TCCCCCAAGTATTGCATTTGGATT | 30 |
| hTRAJ41 | hTRAJ41do | 25 | 443 | AACTCAACAGGGTCCTTGCCACTTA | 31 |
| hTRAJ37 | hTRAJ37do | 28 | 351 | CCACCCACATTTGATGTTTTTATTTCTT | 32 |
| hTRAJ33 | hTRAJ33do | 24 | 98 | TAGTGTCTCCTCTCCCGTGCAGTC | 33 |
| hTRAJ48 | hTRAJ48do | 28 | 43 | GTTCCAGTCCCAAAGGTTAATTTCTCAT | 34 |
| TRAJ29 | hTRAJ29do | 24 | 300 | AGAACAAGCTGGAGGCAACTAGGC | 35 |
| hTRAJ24 | hTRAJ24do | 28 | 227 | AACACCAGTCTGATCTCTCATTTTTGCT | 36 |
| hTRAJ18 | hTRAJ18do | 29 | 147 | CAAGACTAAAGGAGTTAATTCATCTCCCC | 37 |
| hTRAJ53 | hTRAJ53do | 24 | 200 | AATCCCTCTGATGGGCACCATATC | 38 |
| hTRAJ11 | TRAJ11do | 20 | 88 | ACATGGGTGGGATGGGGTCA | 39 |
| hTRAJ7 | hTRAJ7do | 20 | 478 | TGGGAGTAAAGGGCTGGGGC | 40 |
| hTRAJ3 | hTRAJ3do | 25 | 329 | AACCTCAATTCCAGGCAGCAGTATC | 41 |

Cette analyse peut en particulier être réalisée à partir de 6 PCR multi-2-plexes. Alternativement, elle peut être réalisée à partir de 3 PCR multi-4-plexes effectuées avec des combinaisons d'amorces comportant chacune une amorce sens spécifique d'un gène V et un quadruplet d'amorces choisi parmi les quadruplets suivants : (hTRAJ56, hTRAJ41, hTRAJ37, hTRAJ33), (hTRAJ48, hTRAJ29, hTRAJ24, hTRAJ18) et (hTRAJ53, hTRAJ11, hTRAJ7, hTRAJ3). Bien entendu, les situations intermédiaires (4 PCR multi-2-plexes et 1 PCR multi-4-plexe ; 2 PCR multi-2-plexes et 2 PCR multi-4-plexes) sont également envisagées.

Afin d'avoir une information plus globale sur les réarrangements du locus TRA, il est proposé d'effectuer la méthode décrite ci-dessus avec au moins trois, mais de préférence 4, 5, 6 ou davantage d'amorces s'hybridant en amont et/ou dans des gènes V distincts, situés chacun dans une région distincte du locus. Il est important que les gènes TRAV visés par ces amorces soient bien répartis dans le locus, afin que la diversité combinatoire observée soit effectivement représentative de l'ensemble des réarrangements de ce locus. Des amorces utilisables pour cela sont définies dans le tableau ci-dessous :

Tableau 5

| nom du gène | nom de l'oligonucléotide | taille (pb) | distance avec la fin du gène V (pb) | Séquence | SEQ ID No : |
|---|---|---|---|---|---|
| TRAV1 | hTRAV1up_ex | 26 | 104 | GGTCGTTTTTCTTCATTCCTT AGTCG | 42 |
| TRAV3 | hTRAV3 | 22 | 377 | TCCCCTTCCCATTTTCCACTC G | 43 |
| TRAV4 | hTRAV4up_ex_testAn1 | 23 | 96 | CCCTGTTTATCCCTGCCGAC AGA | 44 |
| TRAV10 | hTRAV10 up n3 | 24 | 85 | CTGGATGCAGACACAAAGC AAAGC | 45 |
| TRAV12.2,3 | hTRAV12.2,3up1 | 27 | 114 | AATGAAGATGGAAGGTTTA CAGCACAG | 46 |
| TRAV12.1 | hTRAV12.1up1 | 28 | 112 | ACAAAGAAGATGGAAGGTT TACAGCACA | 47 |
| TRAV14 | hTRAV14 up n2 | 22 | 69 | CGCCAACCTTGTCATCTCCG CT | 48 |
| TRAV16 | hTRAV16 up n5 | 27 | 118 | CTAGAGAGAGCATCAAAGG CTTCACTG | 49 |
| TRAV17 | hTRAV17 up n2 | 22 | 40 | CGGGCAGCAGACACTGCTTC TT | 50 |
| TRAV19 | hTRAV19 up | 24 | 144 | TCGTCGGAACTCTTTTGATG AGCA | 51 |
| TRAV21 | hTRBV21 up | 24 | 91 | TGCCTCGCTGGATAAATCAT CAGG | 52 |
| TRAV22 | hTRAV22 up | 21 | 42 | CCCAGACCACAGACTCAGG CG | 53 |
| TRAV23 | hTRAV23 up n2 | 28 | 130 | CGTCCAGATGTGAGTGAAA AGAAAGAAG | 54 |
| TRBV25 | hTRAV25 up n3 | 27 | 154 | TGGACATCCCGTTTTTTTGA TACAGTT | 55 |
| TRBV27 | hTRAV27 up | 27 | 138 | TGGTGACAGTAGTTACGGGT GGAGAAG | 56 |
| TRBV29 | hTRAV29 up | 24 | 267 | AGCAAAATTCACCATCCCTG AGCG | 57 |
| TRAV30 | hTRAV30 up n2 | 24 | 139 | TGAAGGGTGGAGAACAGAA GGGTC | 58 |

(suite)

| nom du gène | nom de l'oligonucléotide | taille (pb) | distance avec la fin du gène V (pb) | Séquence | SEQ ID No : |
|---|---|---|---|---|---|
| TRAV35 | hTRAV35_intup | 27 | 377 | GGCTGGGAAGTTTGGTGATA TAGTGTC | 59 |
| TRAV36 | hTRAV36_int up n2 | 27 | 304 | ACATTTTTCTACACAGGGGT GAGCAGT | 60 |
| TRAV41 | hTRAV41_int up | 28 | 368 | GCCCTCCTGAAAATGTGTAA AGAAATGT | 61 |

La mise en oeuvre de la méthode en effectuant, pour chacune des 20 amorces TRAV décrites dans ce tableau, 6 PCR multi-2-plexes (ou 3 PCR multi-4-plexes *etc.*) avec les combinaisons décrites plus haut pour observer les réarrangements de 95% des gènes TRAJ avec un gène V, permet d'observer entre 50 et 75% de la totalité des réarrangements VJ du locus TRA.

Bien entendu, l'homme du métier peut transposer cette méthode à l'analyse du locus TRA d'un animal, par exemple au locus TRA murin.

Selon un autre aspect, l'invention concerne une méthode permettant l'analyse des réarrangements de tous les gènes J du locus TRG humain avec au moins 2 gènes $V_x$ et $V_y$ donnés du même locus, en effectuant, à l'étape A), au moins une PCR multi-2-plexe avec un triplet d'amorces constitué de 2 amorces sens s'hybridant en amont et/ou dans lesdits gènes $V_x$ et $V_y$ et de l'amorce antisens hTRGJdo2 (ACATATGAGCCCTTTATGGAAGTCCG, SEQ ID No : 62) de 26 nucléotides s'hybridant dans le gène J2 du locus TRG humain.

A titre d'exemples d'amorces s'hybridant en amont et/ou dans un gène V du locus TRG humain utilisables pour la mise en oeuvre de cet aspect de l'invention, on peut citer les amorces définies dans le tableau ci-dessous :

Tableau 6

| nom du gène | nom oligonucléotide | taille (nt) | distance avec la fin du gène V en pb | Séquence | SEQ ID No : |
|---|---|---|---|---|---|
| TRGV1.2 | hTRGV1.2up1 | 30 | 99 | TATTATACTTACGCAAGCACAAGGAACAAC | 63 |
| TRGV1.4 | hTRGV1.4up1 | 28 | 158 | TGTACTATGACTCCTACACCTCCAGCGT | 64 |
| TRGV1.5 | hTRGV1.5up1 | 23 | 287 | AAGGGGGAACGAAGTCAGTCACG | 65 |
| TRGV1.8 | hTRGV1.8up1 | 26 | 129 | GTGTTGGAATCAGGAATCAGTCGAGA | 66 |

Cette analyse peut notamment être effectuée en ne faisant que des PCR multiplexes simples, ou des PCR multi-n-plexes avec n≥2 avec, dans une même réaction, un couple d'amorces spécifiques du locus TRG et un couple d'amorces spécifiques d'un autre locus.

La diversité des réarrangements intervenus dans le locus TRD peut également être étudiée par une méthode selon l'invention, en effectuant, à l'étape A), une PCR multi-2-plexe avec un triplet d'amorces constitué d'une amorce s'hybridant en amont et/ou dans un gène V de ce locus et des amorces antisens hTRDJ1do5 et hTRDJ3do2, définies comme suit :

- hTRDJ1do5 (TGCCTCCTTAGATGGAGGATGCC, SEQ ID No : 67) est un oligonucléotide antisens de 23 nucléotides s'hybridant entre les nucléotides 90 et 112 du gène J1 du locus TRD ; et
- hTRDJ3do2 (GCAAGGAGGCACGCATACTAGTTAGC, SEQ ID No : 68) est un oligonucléotide antisens de 26 nucléotides s'hybridant entre les nucléotides 448 et 473 du gène J3 du locus TRD.

Avec cette combinaison d'amorces, il est possible d'analyser, à partir d'une seule PCR multi-2-plexe, les réarrangements de tous les gènes J du locus TRD humain avec un gène V donné. L'analyse complète de tous les réarrangements VJ du locus TRD peut donc être obtenue en faisant une PCR multi-n-plexe avec n≥2 par famille TRDV.

A titre d'exemples d'amorces spécifiques de gènes TRDV utilisables avec les amorces antisens hTRDJ1do5 et hTRDJ3do2 pour mettre en oeuvre cet aspect de l'invention, on peut citer les amorces définies dans le tableau ci-dessous :

Tableau 7

| Nom du gène | Nom de l'amorce | Taille (nt) | Distance entre l'extrémité 5' de l'amorce et la fin du gène V (pb) | Séquence | SEQ ID No. |
|---|---|---|---|---|---|
| **TRAV12.1** | hTRAV12.1up1 | 28 | 112 | ACAAAGAAGATGGAAGG TTTACAGCACA | 69 |
| **TRAV14 (TRDV4)** | hTRAV14 up n2 | 22 | 69 | CGCCAACCTTGTCATCTC CGCT | 70 |
| **TRAV16** | hTRA V16 up n5 | 27 | 118 | CTAGAGAGAGCATCAAA GGCTTCACTG | 71 |
| **TRAV17** | hTRAV 17 up n2 | 22 | 40 | CGGGCAGCAGACACTGC TTCTT | 72 |
| **TRAV21** | hTRAV21 up | 24 | 91 | TGCCTCGCTGGATAAATC ATCAGG | 73 |
| **TRAV22** | hTRAV22 up 2 | 21 | 232 | CAGGAGGGAGCCAATTC CACG | 74 |
| **TRAV23 (TRDV6)** | hTRAV23 up n2 | 28 | 130 | CGTCCAGATGTGAGTGAA AAGAAAGAAG | 75 |
| **TRAV25** | hTRAV25 up n3 | 27 | 154 | TGGACATCCCGTTTTTTT GATACAGTT | 76 |
| **TRAV29 (TRDV5)** | hTRAV29 up | 24 | 267 | AGCAAAATTCACCATCCC TGAGCG | 77 |
| **TRAV30** | hTRAV30 up n2 | 24 | 139 | TGAAGGGTGGAGAACAG AAGGGTC | 78 |
| **TRAV35** | hTRAV35_int up | 27 | 377 | GGCTGGGAAGTTTGGTGA TATAGTGTC | 79 |
| **TRAV36 (TRDV7)** | hTRAV36up1 | 26 | 280 | AGTGAAGACAAGGTGGT ACAAAGCCC | 80 |
| **TRAV39** | hTRAV39up1 | 26 | 352 | GGGAGGAACAGGATTAT TGGGGTAAC | 81 |
| **TRAV41** | hTRAV41_int up | 28 | 368 | GCCCTCCTGAAAATGTGT AAAGAAATGT | 82 |
| **TRDV1** | hTRDV1up1 | 25 | 259 | CAGTATCCATGCCAGTGA GGAAAGC | 83 |
| **TRDV3** | hTRDV3up1 | 24 | 287 | GACAAAGTAACCCAGAG TTCCCCG | 84 |

Selon une autre variante, l'invention permet également l'analyse des réarrangements de tous les gènes J du locus IgH humain avec au moins 2 gènes $V_x$ et $V_y$ donnés du même locus, en effectuant au moins une PCR multi-n-plexe avec n≥2 (et en particulier avec n=2), avec une combinaison d'amorces comprenant 2 amorces sens s'hybridant en amont et/ou dans lesdits gènes $V_x$ et $V_y$ et une amorce antisens s'hybridant en aval et/ou dans le gène IgHJ6, par exemple l'amorce hIgHJ6do2 (GATCTTGCAGTCCTACAGACACCGC, SEQ ID No : 85) s'hybridant entre la 368ième et la 39ième base à compter du début du gène IgHJ6.

A titre d'amorces s'hybridant en amont et/ou dans un gène V du locus IgH humain utilisables selon cet aspect de l'invention, on peut citer les amorces définies dans le tableau ci-dessous :

Tableau 8

| nom du gène | nom oligonucléotide | taille (nt) | distance avec la fin du gène V en pb | Séquence | SEQ ID No : |
|---|---|---|---|---|---|
| IGHV1.2, 8, 18, 46,69 | hIGHV1Aup1 | 25 | 172 | GACAAGGGCTTGAGTGGATGGG | 86 |
| IGHV2 | hIGHV2up1 | 22 | 44 | CATGGACCCTGTGGACACAGCC | 87 |
| IGHV3.7,13,15, 20, 21, 23, 48, 53, 64, 66, 72, 73, 74 | hIGHV3Aup1 | 24 | 315 de V3.7 | TGTTTGCAGGTGTCCAGTGTGAGG | 88 |
| IGHV4 | hIGHV4up1 | 25 | 69 | GAACCAGTTCTCCCTGAAGCTGAGC | 89 |
| IGHV5 | hIGHV5up1 | 21 | 55 | TGCAGTGGAGCAGCCTGAAGG | 90 |
| IGHV6 | hIGHV6up1 | 23 | 371 | AGCAGCATTCACAGACTGAGGGG | 91 |

Selon une mise en oeuvre particulière de cette méthode, les amorces spécifiques des gènes $V_x$ et $V_y$ du locus IgH sont choisies de telle sorte que la somme de la distance entre l'extrémité 5' de la zone d'hybridation de l'amorce spécifique de $V_x$ et la fin dudit gène $V_x$ et de la distance entre l'extrémité 5' de la séquence codante du gène IgHJ1 et l'extrémité 3' de la zone d'hybridation de l'amorce antisens hIgHJ soit supérieure à la somme de la distance entre l'extrémité 5' de la zone d'hybridation de l'amorce spécifique de $V_y$ et la fin dudit gène $V_y$ et de la distance entre l'extrémité 5' de la séquence codante du gène IgHJ6 et l'extrémité 3' de la zone d'hybridation de l'amorce antisens hIgHJ6. Ceci permet que l'amplicon correspondant à $V_x$J6 soit plus grand que celui correspondant à $V_y$J1, pour avoir une résolution des amplicons dans l'ordre $V_y$J6, $V_y$J5,..., $V_y$J1, $V_x$J6, ..., $V_x$J1.

Toutefois, étant donné la taille du cluster de gènes IgHJ, les amorces spécifiques des gènes $V_x$ et $V_y$ du locus IgH seront de préférence choisies de façon à obtenir une disposition "enchâssée" des amplicons, c'est-à-dire telle qu'au moins un amplicon obtenu avec un premier couple d'amorces soit encadré par 2 amplicons obtenus avec un deuxième couple d'amorces.

La méthode de l'invention permet également l'analyse des réarrangements incomplets du locus IgH humain, en effectuant au moins une réaction de PCR multi-n-plexe avec n≥2 avec une combinaison d'au moins 3 amorces constituant au moins 2 couples d'amorces comportant une amorce antisens commune s'hybridant spécifiquement en aval et/ou dans un gène J donné telle que, par exemple, l'amorce hIgHJ6do2 décrite plus haut, chaque couple d'amorces comportant en outre une amorce sens s'hybridant spécifiquement en amont et/ou dans un gène D donné.

A titre d'exemples d'amorces s'hybridant en amont et/ou dans un gène D du locus IgH humain utilisables selon cet aspect de l'invention, on peu citer les amorces définies dans le tableau ci-dessous :

Tableau 9

| nom du gène | nom oligonucléotide | taille (pb) | Distance entre l'extrémité 5' de l'amorce et la fin du gène D (pb) | Séquence | SEQ ID No : |
|---|---|---|---|---|---|
| hIGHD1 | hIGHD1up1 | 23 | 44 | GATTCTGAACAGCCCCGAGTCAC | 92 |
| hIGHD2 | hIGHD2up1 | 22 | 67 | GGACAGGAGGATTTTGTGGGGG | 93 |
| hIGHD3 | hIGHD3up1 | 20 | 102 | AGGTCAGCCCTGGACATCCC | 94 |
| hIGHD4 | hIGHD4up1 | 19 | 132 | ATCCCCAGGACGCAGCACC | 95 |
| hIGHD5 | hIGHD5up2 | 20 | 85 | AGCTCCTCCTGACAGCCCCG | 96 |
| hIGHD6 | hIGHD6up1 | 21 | 160 | ACACCAGACAGAGGGGCAGGC | 97 |

(suite)

| nom du gène | nom oligonucléotide | taille (pb) | Distance entre l'extrémité 5' de l'amorce et la fin du gène D (pb) | Séquence | SEQ ID No : |
|---|---|---|---|---|---|
| hIGHD7 | hIGHD7up2 | 20 | 90 | AGACCGCAGCCACATCAGCC | 98 |

Cette analyse peut notamment être effectuée en ne faisant que des PCR multiplexes simples, ou des PCR multi-n-plexes avec n≥2 avec, dans une même réaction, un couple d'amorces spécifiques du locus IgH et un couple d'amorces spécifiques d'un autre locus.

Selon une mise en oeuvre préférée de l'invention, la méthode combine l'analyse des réarrangements V(D)J du locus IgH et celle des réarrangements incomplets de ce locus, par la mise en oeuvre des variantes appropriées décrites ci-dessus. Les amorces décrites plus haut pour cette analyse sont adaptées à l'analyse des réarrangements de ce locus chez l'humain, mais cette méthode peut être transposée, sans aucune difficulté, chez l'animal, par exemple chez la souris.

Selon une autre mise en oeuvre de l'invention, la méthode est adaptée pour analyser la diversité combinatoire des réarrangements V(D)J d'au moins deux locus génétiques choisis parmi les loci TRA, TRB, TRG, TRD, IGH, IGK et IGL, en combinant les variantes appropriées décrites ci-dessus. L'analyse des différents loci peut se faire de façon simultanée ou séquentielle, en faisant le cas échéant des PCR multi-n-plexes avec, dans la même réaction, au moins un couple spécifique d'un locus et un autre couple spécifique d'un autre locus.

En particulier, l'analyse combinée de la diversité combinatoire des réarrangements V(D)J du locus TRB et de la diversité combinatoire des réarrangements VJ du locus TRG ou du locus TRD permet d'avoir une vision représentative du répertoire de lymphocytes T. En ajoutant l'analyse de la diversité combinatoire des réarrangements V(D)J du locus IgH, on obtient une information sur l'ensemble des lymphocytes (B et T).

Un aspect important de l'invention, illustré dans les exemples ci-après, est la possibilité d'identifier par leur nom les différents réarrangements observés. Selon une mise en oeuvre préférée, l'étape C) de la méthode comporte une étape de traitement des données obtenues par la séparation des amplicons suivant leur taille, ledit traitement étant effectué par un ordinateur et permettant d'attribuer à chaque amplicon observé le nom du réarrangement V(D)J correspondant. De manière encore préférée, le traitement des données intègre également l'intensité du signal de chacun des amplicons observés, pour quantifier la fréquence relative du réarrangement V(D)J correspondant.

Ceci permet de décrire une signature d'une diversité immunitaire en classant les réarrangements VDJ par ordre d'intensité ou par ordre de contribution au sein du répertoire immunitaire observé. Ce classement des réarrangements correspond à une signature du répertoire immunitaire à un instant "t" dans un échantillon.

En particulier, la méthode de l'invention peut être telle que l'étape B) comprend l'acquisition des données concernant la taille des amplicons et, pour chacun, l'intensité du signal, et l'étape C) comprend les étapes suivantes :

(i) identification de chaque amplicon, en déterminant à quel réarrangement V(D)J il correspond, en fonction de sa taille ;
(ii) à partir de l'intensité du signal de chaque amplicon, détermination de la proportion de l'ADN génomique de départ présentant le réarrangement V(D)J correspondant ;
(iii) présentation des résultats sous forme d'un graphique tridimensionnel présentant les gènes ou les familles de gènes $V_x$ suivant un axe, les gènes $J_y$ suivant un autre axe, et la fréquence des réarrangements $V_xJ_y$ suivant le troisième axe.

Si on mesure en outre la diversité moléculaire, par exemple en effectuant une mesure en temps réel des amplifications par PCR multi-n-plexe, la méthode permet de mesurer une diversité immunitaire globale par la prise en compte de la mesure de la diversité combinatoire et de la diversité moléculaire.

La présente invention porte également sur une méthode pour déterminer *in vitro* le niveau d'immunodéficience d'un individu, comportant les étapes suivantes :

A) à partir d'un échantillon biologique dudit individu, faire une numération lymphocytaire ;
B) à partir du même échantillon ou d'un autre échantillon provenant du même individu au même moment, déterminer le niveau de diversité combinatoire du répertoire des lymphocytes dudit individu, par la mise en oeuvre d'une méthode telle que décrite plus haut ;
C) combiner les données obtenues aux étapes A) et B).

Cette méthode peut comporter une étape additionnelle d'interprétation de la combinaison obtenue à l'étape C), au regard d'un graphique attribuant un niveau de risque au moins aux zones (i) à (iv) ci-après :

(i) numération faible (<1000 Ly/μL) et diversité combinatoire V-J faible (<40%) : risque infectieux élevé, associé à un risque élevé de mortalité ;
(ii) numération faible (<1000 Ly/μL) mais diversité combinatoire V-J normale (>65%) : risque infectieux faible ;
(iii) numération normale (1000-3200 Ly/μL) et diversité combinatoire V-J faible (<40%) : risque infectieux moyen ;
(iv) numération normale (1000-3200 Ly/μL) et diversité combinatoire V-J normale (>65%) : le répertoire immunitaire est sain.
(v) numération au dessus de la normale (> 3200Ly/μL), et diversité combinatoire V-J faible (<40%) : risque lymphoproliphératif élevé
(vi) numération au dessus de la normale (> 3200Ly/μL), et diversité combinatoire V-J normale (>65%) : risque lymphoprolifératif moyen.

Cette détermination du niveau d'immunodéficience d'un individu (basée non seulement sur la numération mais également sur la mesure de la diversité lymphocytaire) est cruciale pour mettre en oeuvre une médecine personnalisée, car un patient présentant une diversité immunitaire inférieure à 40% est considéré comme présentant un déficit de diversité qui implique un risque accru de mortalité par infection. Cette méthode permet donc de déterminer, pour un patient, son risque de mortalité par infection.

De façon avantageuse, le clinicien pourra faire un suivi immunologique de son patient, permettant notamment d'identifier si le traitement administré a des conséquences trop sévères sur la diversité immunitaire du patient, induisant un risque de mortalité par infection. Dans ce cas, le clinicien pourra adapter le traitement (changement de molécule, de dose, de fréquence, adjonction de traitements supplémentaires d'antibiotiques, d'immunostimulation par interleukine IL7, IL2 ou autres...) pour réduire ce risque.

L'invention porte donc également sur l'utilisation de la méthode ci-dessus, pour aider un clinicien dans ses choix thérapeutiques, par le choix d'un traitement adapté au niveau de risque d'infectiosité et de mortalité d'un patient. Ainsi, un patient situé dans la zone (iv) définie plus haut peut *a priori* supporter un traitement immunosuppresseur (par exemple, chimiothérapie + anticorps monoclonaux puissants), tandis qu'à l'inverse, un patient situé dans la zone (i) a un « bouclier immunitaire » très fragile (donc un grand risque de mortalité par infection), et doit donc être traité par des médicaments moins immunosuppresseurs. Les patients des zones (ii) et (iii) sont dans une situation intermédiaire.

Dans une mise en oeuvre préférée d'une méthode pour déterminer *in vitro* le niveau d'immunodéficience d'un individu telle que décrite ci-dessus, l'étape B) comprend la détermination du niveau de diversité combinatoire du répertoire des lymphocytes T et des lymphocytes B dudit individu. Dans ce cas de figure, on peut avantageusement examiner les données obtenues à l'aide d'un graphique tridimensionnel présentant le niveau de diversité des immunoglobulines sur un axe, le niveau de diversité des TCR sur un autre axe, et la numération lymphocytaire sur un troisième axe.

Est également décrite ici une méthode de suivi de l'évolution de la diversité du répertoire des lymphocytes T et/ou B d'un individu, comportant les étapes suivantes :

A) Mesure de la diversité du répertoire des lymphocytes dudit individu, par la mise en oeuvre d'une méthode telle que décrite ci-dessus, à partir de deux échantillons dudit individu prélevés à deux dates différentes ;
B) comparaison des deux échantillons en évaluant :

(i) le nombre S de réarrangements observés dans les deux échantillons ;
(ii) le nombre A de réarrangements observés dans l'échantillon le plus récent mais pas dans le plus ancien ;
(iii) le nombre D de réarrangements observés dans l'échantillon le plus ancien mais pas dans le plus récent ;
(iv) le nombre Z de réarrangements qui ne sont observés dans aucun des échantillons.

Un exemple d'interprétation de ce graphique est présenté à l'exemple 11 ci-après. Cette méthode peut également servir aussi à comparer 2 échantillons d'individus différents, par exemple pour comparer un donneur et un receveur en cas de greffe.

La présente invention porte également sur une trousse, ou "*kit*", pour la mise en oeuvre d'une des méthodes décrites plus haut, comprenant au moins une combinaison d'amorces telles que définies dans ce texte, et des réactifs pour effectuer des PCR.

Parmi les réactifs pour effectuer des PCR, une trousse selon l'invention comprendra de préférence une polymérase présentant les caractéristiques suivantes :

(i) elle est capable d'amplifier des fragments de plusieurs dizaines de kb ;
(ii) sa vitesse d'élongation est d'au moins 1 kb/minute ;

(iii) sa robustesse est telle qu'elle n'introduit pas plus d'une erreur par kb, en moyenne.

De façon avantageuse, une trousse de l'invention comprendra une plaque multipuits dans laquelle chaque puit contient une combinaison différente d'amorces, sous forme lyophilisée ou en phase liquide. De préférence, cette plaque multipuits comprend toutes les combinaisons d'amorces nécessaires à l'amplification d'au moins 50, 60, 70, 80 voire 95% des réarrangements V-J d'au moins un locus choisi parmi les loci TRA, TRB, TRG, TRD et IGH.

Est également décrite ici l'utilisation d'une méthode ou d'une trousse telle que décrites plus haut, pour étudier la mise en place et/ou la qualité du répertoire TCR et/ou IgH d'un animal transgénique humanisé et/ou d'une culture de lymphocytes. Ceci permet notamment de vérifier la qualité d'un répertoire immunitaire suite à une culture cellulaire, par exemple pour vérifier que la culture cellulaire reste appropriée pour tester des molécules ou pour étudier des mécanismes biologiques. Dans le cas de lignées T ou B monoclonales ou oligoclonales, cela permet de vérifier qu'il s'agisse bien du ou des clones préalablement identifiés, et ainsi de détecter toute contamination ou erreur d'étiquetage sur un tube avec de lancer une expérience. Une autre application importante est de contrôler la qualité lors de la production de cultures de Lymphocytes (T régulateurs par exemple...) avant réinjection (à visée thérapeutique).

Est également décrite ici l'utilisation d'une méthode ou d'une trousse telle que décrite plus haut, pour cribler des molécules thérapeutiques *in vitro.* Des exemples d'applications sont décrits ci-après.

Un procédé d'évaluation de l'efficacité d'un protocole vaccinal est aussi décrit, comprenant les étapes de

A) mesure de la quantité et de la diversité des lymphocytes avant et après ledit protocole vaccinal, à l'aide d'une méthode ou d'une trousse telle que décrite plus haut ;

B) comparaison des mesures faites à l'étape A ; et

C) interprétation des résultats, une baisse de la diversité lymphocytaire d'au moins 10%, de préférence au moins 15% après vaccination indiquant que le protocole de vaccination a été efficace.

Selon une mise en oeuvre de ce procédé, on peuten outre mesurer, à l'étape A), la quantité de lymphocytes T régulateurs avant et après vaccination. Dans ce cas, l'étape C d'interprétation tient compte également de l'évolution du nombre de lymphocytes T régulateurs, une diminution d'un facteur $\geq 2$ du nombre de lymphocytes T régulateurs suite à la vaccination indiquant que le protocole a été efficace.

Est également décrit un procédé de comparaison de l'efficacité de deux protocoles vaccinaux, comprenant les étapes de

A) mesure, sur deux groupes faisant l'objet d'une vaccination par deux protocoles différents, de la quantité de lymphocytes T régulateurs et de la diversité immunitaire, avant et après vaccination ;

B) comparaison des résultats groupe à groupe,

dans lequel le protocole le plus efficace est celui qui induit la diminution de lymphocytes T régulateurs et/ou la diminution de la diversité lymphocytaire la plus importante.

Outre les dispositions qui précèdent, l'invention comprend encore d'autres dispositions, qui ressortiront des exemples expérimentaux ci-dessous, et des figures annexées.

## Exemple 1 : Choix de la position des amorces utilisables dans le cadre de l'invention

### *Critères de sélection des oligonucléotides*

Les amorces utilisables pour mettre en oeuvre le procédé de l'invention sont choisies en fonction 1) de leurs propriétés thermodynamiques (déterminées à partir d'algorithmes classiquement utilisés par l'homme du métier pour identifier la capacité des oligonucléotides à se fixer sur leur séquence cible, notamment en fonction du nombre de liaisons hydrogènes) ; 2) de leur compatibilité avec les autres amorces utilisées dans le même tube, tant sur le plan thermodynamique que sur l'incapacité des différentes amorces à s'hybrider entre elles ; et 3) de leur position respective qui permet d'obtenir des tailles d'amplicons pouvant être résolus.

Par "résolu", il doit être compris que les amplicons peuvent être observés de façon univoque, suite à leur séparation par la taille par une méthode d'électrophorèse ou tout autre méthode. Lorsque les tailles de certains amplicons sont trop proches, il n'est pas possible de les discerner distinctement avec des conditions de séparation compatibles avec la séparation permettant de "résoudre" l'ensemble des autres tailles d'amplicons. Ce cas est minoritaire et identifié. Il peut être résolu, par exemple, par l'utilisation d'amorces marquées.

Les oligonucléotides sont définis ici en donnant, outre leur séquence, leur position dans le locus ainsi que leur taille, en nombre de bases.

Pour les gènes V, les oligonucléotides sont orientés dans le sens de transcription, ils sont dénommés "SENS" ;

ils sont complémentaires au brin d'ADN non codant.

Pour les gènes J, les oligonucléotides sont dénommés "ANTISENS" : ils sont complémentaires au brin d'ADN codant et inversés (on dit aussi qu'ils sont dans le sens 3'→5').

### *Amorces spécifique des gènes V*

Pour les oligonucléotides spécifiques des gènes V, la position est donnée en fonction de la fin du gène V, c'est-à-dire la dernière base avant le RSS. Cette position correspond à la distance (incluant l'oligonucléotide) entre la 1ère base de l'oligonucléotide et la dernière base du gène V.

◦ Exemple de position de l'oligonucléotide pour la famille de gène TRBV : si la distance est de *n* bases de la fin du gène V et la taille de l'oligonucléotide est de *t* bases, l'oligonucléotide débute à n bases en amont de la fin du gène (en comptant la dernière base) et se termine à *n-t+1* bases de la fin du gène V.

Les oligonucléotides ont été sélectionnés de manière à s'hybrider au plus grand nombre de membres possible d'une même famille V. Deux cas de figures peuvent être décrits concernant le nombre d'oligonucléotides V nécessaires pour suivre toute une famille V:

◦ *Cas 1 : existence d'une zone d'homologie de 100% entre tous les membres d'une même famille,* dans ce cas il est possible de trouver, en faisant un alignement de séquences, une zone commune à 100% pour tous les membres de la famille V en question et répondant aux critères de sélection des oligonucléotides précisés précédemment. Dans ce cas, 1 seul oligonucléotide V est nécessaire pour suivre tous les membres de la famille.

◦ Cas 2 : zone d'homologie inférieure à 100%. Dans ce cas, la plus grande zone (en nombre de bases) qui répond aux critères de sélection est sélectionnée, et tous les oligonucléotides correspondants à cette position nécessaires pour suivre tous les membres de la famille sont dessinés. Ex : pour une famille de 5 membres présentant une zone d'homologie de 100% pour 3 membres, les 2 autres membres étant différents entre eux dans cette zone. Dans ce cas, un total de 3 oligonucléotides différents correspondant à la même position sont dessinés pour suivre l'ensemble des membres de cette famille. Trois sous cas sont alors possibles :

- Les oligonucléotides V choisis à cette position sont compatibles entre eux thermodynamiquement. Dans ce cas, les n oligonucléotides V (dans l'exemple ci-dessus 3 oligonucléotides) sont regroupés dans un même tube de PCR. Puisque tous ces oligonucléotides, même si ils ont quelques bases différentes, sont dessinés à la même position, les amplicons seront de même taille.
- Les oligonucléotides V ne sont pas suffisamment compatibles thermodynamiquement ou ils ne peuvent être mis dans la même réaction de PCR car cela poserait des problèmes de dimères. Dans ce cas, 2 ou n PCR peuvent être réalisées dans des tubes différents, pour suivre spécifiquement les membres V pour lesquels les amorces sont incompatibles avec les autres.
- Il existe un cas particulier (rare) dans lequel les gènes V d'une même famille n'ont pas la même taille. Ceci est dû au fait que l'intron d'un ou de n membre(s) d'une famille V présente(nt) une taille différente de celui des autres membres de ladite famille V (rmq : il n'y a qu'un intron par gène V). Ce cas de figure n'est pas gênant si l'oligonucléotide V est dessiné en aval de cette "zone de taille différente". Si cela n'est pas possible, la solution mise en oeuvre consiste à séparer les 2 ou n oligonucléotides dans 2 ou n PCR.

### *Amorces spécifiques des gènes J*

La position d'une amorce spécifique d'un gène J est donnée en indiquant sa distance par rapport au début du gène J, c'est-à-dire la 1ère base du segment de gène J (séquence codante), après le RSS. Cette position ce situe en aval du début du J et correspond à la distance (incluant l'oligonucléotide) entre la 1ère base du gène J et la première base de l'oligonucléotide (c'est-à-dire la base à l'extrémité 5' de l'amorce).

◦ Exemple de position de l'oligonucléotide pour la famille de gène TRBJx : la distance est de *n* bases du début du J, la taille de l'oligonucléotide est de *t* bases. Donc la zone d'hybridation de l'amorce (sur le brin codant) se termine à la *n*ième base du gène J ou en aval du début du gène J et débute à *n-t+1* bases du début du J.

### *Récupération des séquences des loci du TCR et IG*

Plusieurs méthodes sont possibles pour obtenir les séquences. Deux possibilités sont décrites ci-après.

**1ère possibilité**

La 1ère possibilité nécessite d'aller sur le site internet Européen « *Ensembl Genome Browser* » http://www.ensembl.org et de rechercher le locus d'intérêt : après avoir choisi l'espèce (humain, souris, *etc.*) il faut cliquer sur le chromosome d'intérêt, exemple chromosome 14 pour le locus TRA. Il faut ensuite indiquer, dans la case prévue à cet effet, le chiffre indiquant le début (*start*) de la région chromosomique (p. *ex.* :21158000) et la fin de la région (*end*) (*p. ex.* :22125000). La base de données *Ensembl* fait ressortir graphiquement la disposition des gènes TCR et IG présents dans le locus et l'homme du métier peut exporter (par un clic gauche sur le contig concerné) la séquence d'ADN au format EMBL ou GenBANK avec toutes les annotations de gènes correspondantes. Il a ainsi à sa disposition la localisation de toutes les séquences des gènes TCR et IG, y compris les séquences en amont et aval de ces derniers.

Les régions chromosomiques pour les différents loci humains concernés par la présente invention sont indiquées ci-après.

*Homo sapiens* TRA/TRD : locus à 14q11.2:
http://www.ensembl.org/Homo_sapiens/contigview?region=14&vc_start=2 1158000&vc_end=22125000
*Homo sapiens* TRB : locus à 7q34:
http://www.ensembl.org/Homo_sapiens/contigview?region=7&vc_start=14 1640000&vc_end=142275000
*Homo sapiens* TRG : locus à 7p14:
http://www.ensembl.org/Homo_sapiens/contigview?region=7&vc_start=38 242000&vc_end=38385000
*Homo sapiens* IGH: locus à 14q32.33:
IGHV :

http://www.ensembl.org/Homo_sapiens/contigview?region=14&vc_start=1 05476000&vc_end=106368585

IGHD et IGHJ :
http://www.ensembl.org/Homo_sapiens/contigview?region=114&vc_start=1 05400000&vc_end=105460000
IGHC:
http://www.ensembl.org/Homo_sapiens/contigview?region=14&vc_start=1 05120000&vc_end=105400000
*Homo sapiens* IGK : locus à 2p11.2:
IGKV (proximal cluster), IGKJ et IGKC :

http://www.ensembl.org/Homo_sapiens/contigview?region=2&vc_start=88 920000&vc_end=89480000

IGKV (duplicated distal cluster) :
http://www.ensembl.org/Homo_sapiens/contigview?region=2&vc_start=89 550000&vc_end=89950000
*Homo sapiens* IGL : locus à 22q 11.2:
http://www.ensembl.org/Homo_sapiens/contigview?region=22&vc_start=2 0700000&vc_end=21650000

**2ème possibilité :**

La 2ème possibilité présentée ci après nécessite de regrouper tous les cosmides contenant les séquences d'un locus TCR ou IG. Pour ce faire, nous avons identifié la liste des numéros d'accession des cosmides pour les chaînes des IG et des TCR, chez l'homme et la souris en se basant sur la bibliographie (Lefrancs, *The Immunoglobulin Facts Book* 2001 et Lefrancs *The T cell receptor Facts Book* 2001), ou encore (Baum et al., 2006; Baum et al., 2004).

Tableau 10

| **Humain :** | |
|---|---|
| **Locus** | **Numéro d'accession EMBL-EBI** |
| TRAD* | AE000658 à AE000662 |
| TRB | L36092 |
| TRG | AF159056, X08084, M12950, M12960, M16016 et M12961 |
| IGH | voir Lefranc., The Immunoglobulin FactsBook ISBN:012441351X |
| | |

(suite)

| SOURIS: | |
|---|---|
| **Locus** | **Numéro d'accession EMBL-EBI** |
| **TRAM*** | AE008683 à AE008686 |
| **TRB** | AE00063 à AE00065 |
| **TRG** | AF037352 and AF021335 |
| **IGH** | voir Lefranc ., The Immunoglobulin FactsBook ISBN:012441351X |
| * Rappel : le locus TRD est situé dans le locus TRA. | |

A partir de ces numéros il est possible de récupérer l'ensemble des informations sur les séquences sources des loci dans le site internet de référence Européen « EMBL-EBI » (http://www.ebi.ac.uk), en faisant une « recherche », dans la rubrique « nucleotide sequence ». Les résultats de la « EMBL-BANK » (*Europe's primary nucleotide sequence resource*) sont ensuite téléchargeables au format EMBL. L'étude des séquences peut être réalisée sur des logiciels tel que *NTI Vector*®. Les gènes étant annotés, leur position est indiquée précisément.

***Correspondante entre les différentes nomenclatures des gènes des loci TCR et IG***

Il est important de noter que la nomenclature des gènes a évolué au cours du temps. Afin de se repérer entre les nomenclatures, l'homme du métier dispose de tableaux de correspondances pour les TCR et IG que l'on peut retrouver dans les 2 livres [1] Lefranc, M.-P. and Lefranc, G., The Immunoglobulin FactsBook, Academic Press, 458 pages (2001) ISBN:012441351X [2] Lefranc, M.-P. and Lefranc, G., The T cell receptor FactsBook, Academic Press, 398 pages (2001) ISBN:0124413528. Ces informations peuvent aussi être retrouvées sur le site IMGT (http://imgt.cines.fr).

**Exemple 2 : Protocole pour l'utilisation *d'ImnumTraCkeR Kit Exemple sur le Locus TRB.***

Suivant la nature de l'échantillon (cellules ; PBMC ; extrait de thymus ;...), les quantités nécessaires sont optimisées. Pour un échantillon cellulaire, la quantité nécessaire pour réaliser l'expérimentation est de $10^6$ cellules.
La succession des différentes étapes du protocole est schématisée sur la figure 19.

***2-A. Extraction de l'ADN***

Un ADN extrait d'une haute pureté est nécessaire pour la détection des réarrangements V-J en utilisant la trousse *ImmunTraCkeR Kit.* L'homme du métier sait quel procédé ou kit sont appropriés pour cela. En particulier, l'homme du métier sait que cette extraction doit être réalisée sans aucun EDTA ou autre produit pouvant inhiber la PCR. Les inventeurs recommandent d'extraire l'ADN en utilisant *High Pure PCR Preparation Template Kit* de Roche®.
La concentration d'ADN recommandée est de 100 ng/μl.

***2-B. Contrôle de la qualité de l'ADN et détermination de la quantité***

Une mesure de l'absorbance de l'échantillon à 260 nm avec un spectrophotomètre (exemple *Amersham GenQuant Pro*) est réalisée. Cette mesure permet de calculer la concentration de l'ADN, le taux d'extraction et le rapport ADN/protéine qui donne une estimation de la qualité de l'ADN.
En outre, l'état de dégradation de l'ADN est contrôlé sur un gel d'agarose et ensuite la concentration de l'ADN est normalisée par comparaison avec un contrôle d'actine.

***2-C. Amplification par PCR***

*L'ImmunTraCkeR Kit* contient les combinaisons d'amorces (déshydratées ou en phase liquide) déjà distribuées dans les tubes. Le mélange réactionnel est préparé et distribué dans lesdits tubes de réaction.

a) Protocole optimisé *Herculase®II Fusion*

Préparation du mélange réactionnel

Tableau 13

| Composant | Quantité par réaction |
|---|---|
| Eau distillée | 12,89 µl |
| Tampon réactionnel Herculase® II 5X | 5µl |
| Mélange de dNTP (10 mM) | 0,62 µl |
| Matrice d'ADN (50ng/µl) | 1 µl |
| ADN polymérase *Herculase® II Fusion* | 0.5 µl |
| | Volume final : 20 µl |

Le mélange réactionnel est alors distribué dans des tubes ou des puits, à raison de 20 µl dans chacun.

La PCR est réalisée en utilisant des conditions cycliques optimisées. Des paramètres de cyclage suggérés pour effectuer des PCR avec l'ADN polymérase *Herculase® II Fusion,* en utilisant un dispositif *Primus 96+ (MWG)* sont indiqués ci-dessous.

Paramètres cycliques de la PCR:

Tableau 14

| Segment | Nombre de cycles | Température | Durée |
|---|---|---|---|
| 1 | 1 | 98°C | 3 minutes |
| 2 | 30 | 98°C | 20 secondes |
| | | 62°C | 20 secondes |
| | | 72°C | 3 minutes 30 secondes |
| 3 | 1 | 72°C | 3 minutes |

Durée de la PCR : env 4,5 heures.

***2-D. Electrophorèse sur gel d'agarose***

Préparer un gel d'agarose à 0,8% (p/v) dans un tampon TBE 1X.

Charger les produits de la PCR mélangés auparavant avec un tampon de charge (0,25% du bromophénole bleu, 0,25% du xylène cyanol FF, 30% du Ficoll 400, dans de l'eau), à raison d'environ 10µl de produits PCR et 2µl du tampon de charge.

Déposer un marqueur de taille d'ADN approprié sur chaque extrémité du gel.

Mettre le gel sous tension dans un tampon TBE 1X, sans recirculation du tampon, pendant 1 heure et 30 minutes à 250V et 120mA.

Colorer le gel avec 40µl de bromure d'éthydium dilué dans 150 ml de tampon TBE 1X pendant 30 minutes.

***2-E. Acquisition et interprétation***

Poser le gel sur un transilluminateur à UV et acquérir l'image, par photographie. Enregistrer la présence ou l'absence des produits spécifiques de la PCR.

## Exemple 3: Analyse des réarrangements de tous les gènes J fonctionnels du locus béta avec 1 gène V donné, en 1 PCR multi-2-plexe résolutive

La figure 2a, montre l'analyse du taux de GC moyen des régions TRBJ et TRAJ humaine. La région J2 contient un taux très élevé de GC avec 60% contre 40-45% en moyenne.
La figure 2b montre la disposition particulière des gènes J du locus TRB (ne 2 clusters distincts).
La figure 2c illustre le principe de choix des amorces, avec 1 amorce antisens proche d'un cluster J et deuxième amorce antisen loin de l'autre cluster J, permettant de positionner l'intégralité du cluster J1 au dessus du cluster J2.
La figure 2d présente un exemple de résultat obtenu par cette méthode.
Cette configuration permet de diviser par 2 la quantité de matériel biologique nécessaire et réduire par la même occasion le prix de revient du test. Le temps pour réaliser le test est également diminué. En outre, ceci permet une lecture simplifiée de la piste du gel (ou d'un autre type de séparation) car les gènes J sont dans l'ordre du locus et sont donc facilement identifiables.

## Exemple 4 : Analyse de plusieurs loci dans une même réaction de PCR multi-n-plexe

La figure 3 illustre la possibilité d'analyser des réarrangements de plusieurs loci dans une même réaction, de manière à diminuer la quantité de matériel biologique d'un facteur 3-4, nécessaire pour suivre l'ensemble du répertoire immunitaire. Dans cet exemple, des réarrangements des répertoires TCRB et TCRD sont observés en 1 seule étape. Ceci permet de suivre tous les lymphocytes T alpha/beta et Tgamma/delta.

## Exemple 5 : PCR multiplexe enchâssée.

La figure 4 illustre le principe de PCR multi-n-plexe "enchâssée", c'est-à-dire dans laquelle les séries d'amplicons obtenues avec les différents couples d'amorces sont telles qu'un amplicon obtenu avec un premier couple d'amorces peut être encadré par 2 amplicons obtenus avec un deuxième couple d'amorces.
La figure 4a présente un schéma du locus TRG.
La figure 4b schématise le principe de la résolution avec une famille hTRGV.
La figure 4c montre le résultat d'une PCR multiplexe sur le locus hTRG, visant les 2 clusters J avec une seule amorce J. l'expérience a été réalisée sur des cellules HEK et CaCO comme contrôles négatifs et sur des PBMC et des cellules de thymus comme contrôles positifs.
A noter que pour le locus TRG, il est possible de suivre 2 clusters TRGJ avec 1 seul oligonucléotide J, du fait de l'homologie de séquence de 100% en aval des gènes J1 et J2.
La figure 4d montre un exemple de PCR multi-2-plexe enchâssée avec deux amorces TRAJ.
La figure 4e montre un exemple de résolution de 95% de la région AJ avec seulement 6 PCR multi-2-plexes. La position des primers est indiquée en dessous du nom du gène J des oligonucléotides en aval du (début) du gène J. Cette position est importante. Elle permet de s'assurer que les bandes attendues auront une taille leur permettant d'être bien résolues.

## Exemple 6: Résultat de la trousse *ImmunTraCkeR* TRBV en PCR multi-2-plexe

Les figures 5 et 6 montrent les différentes bandes obtenues après la migration sur gel d'agarose de tous les produits PCR obtenus par PCR multi-n-plexe. La figure 5 montre une représentation schématique du résultat théorique obtenu avec la trousse *ImmunTraCkeR* TRB humain (la représentation est similaire chez d'autres espèces : le rat, la souris, le singe...). Chaque colonne correspond à 1 famille TRBV, chaque bande correspond à un réarrangement V-J donné. Les gènes TRBV ont été étudiés dans l'ordre suivant : BV2, BV3, BV4, BV5, BV6, BV7, BV9, BV10, BV11, BV12, BV13, BV14, BV15, BV16, BV18, BV19, BV20, BV24, BV25, BV27, BV28, BV29, BV30. La figure 6 montre les résultats expérimentaux correspondants obtenus en duplicat (déposé côte à côte), avec trois types d'échantillons : ADNg extrait de thymus, ADNg extrait de PBMC en condition lymphopénique et enfin un pool d'ADN de 4 lignées T comportant chacune un ou 2 réarrangements TRBV-J.
Le fait de disposer côte à côte l'ensemble des PCR des 24 TRBV permet une détection exhaustive des gènes TRBV sur l'ensemble des segments BJ.
On peut également rajouter (en option) un marqueur de reproductibilité de dépôt et/ou de PCR dans chacune des pistes (RMQ : ces 2 marqueurs de reproductibilité ne sont pas indispensables). Avantage recherché : améliorer la normalisation du signal entre les bandes.
Cette figure illustre le fait que l'invention permet d'évaluer la qualité d'un répertoire immunitaire, en mesurant à la fois la diversité combinatoire d'une image du répertoire immunitaire (calcul de la somme des bandes obtenues) ainsi que l'intensité du signal de toutes les bandes (calcul de la somme de tous les signaux des amplicons de l'image).

En outre, le nom de chaque réarrangement V(D)J présent ou absent est identifié (en fonction de sa position dans l'image. La colonne donne le nom du V ; la taille de l'amplicon donne le nom du J. L'intensité du signal détecté donne la proportion respective de chaque réarrangement V(D)J. Au total cela permet donc en 1 seule étape d'avoir un outil qui mesure à la fois la diversité (utile pour mesurer le niveau d'immunodéficience précis d'un patient) et identifie le nom des réarrangements (donc le marqueur TCR ou IG d'un lymphocyte) impliqués dans une pathologie (leucémie, lymphome, GVHD...) ou ayant réagi à la hausse (multiplication cellulaire) ou à la baisse suite à un traitement.

**Exemple 7 : Etude des réarrangements D-J par PCR clustering**

La figure 7 montre le résultat d'un test de détection des réarrangements incomplets D-Jβ à la fois exhaustif et résolutif (Pistes A : réarrangements D1 avec le cluster J1 et le cluster J2. Pistes B : réarrangements D2 avec le cluster J2). Ce test permet de caractériser un clone en précisant le nom des gènes D et J réarrangés.

En comparaison, Biomed-2 ne propose qu'un signal de type ON / OFF : la présence d'une population monoclonale est effectivement détectée, mais cette population n'est pas caractérisée au niveau combinatoire. Il n'est pas possible de faire la différence entre les gènes V et J réarrangés. En effet, la taille des produits PCR de ce test ne varie que de quelques bases, rendant impossible l'identification de la famille V en question, ni du gène J réarrangé. La méthode de la présente invention permet donc de fournir un niveau d'information supérieur.

Analyse des résultats :

Partie A : On observe bien le nombre de bandes attendues sur témoins positifs (PBMC et Thymus). Les bandes correspondant aux réarrangements du gène D1 avec les gènes J1.1 et J1.2 sont fusionnées car les limites résolutives de la technologie utilisée ne permettent pas la séparation de bandes dont la différence de taille n'excède pas 10% de la taille de la plus grande bande. Un séquençage de cette bande, ou l'utilisation d'amorces marquées, serait nécessaire à la validation de cette observation.

Deux bandes très fortes apparaissent sur le témoin négatif. Ces bandes ne sont pas non spécifiques, mais correspondent à l'amplification de l'ADN germinal : les caractéristiques du locus couplées à la capacité de la technologie à amplifier des fragments de grande taille, font qu'un réarrangement n'est pas nécessaire pour observer un produit dans ce cas précis. Ces deux bandes constituent un très beau témoin interne de la présence et de la qualité de l'ADN testé, ainsi que de l'efficacité de l'enzyme.

Partie B : le procédé permet de caractériser très rapidement le réarrangement intermédiaire des lignées de lymphocytes T (voire même B). Dans l'exemple ci-dessus, le réarrangement incomplet D1-J1.3 pour la lignée cellulaire T appelée JURKAT, le réarrangement incomplet D1-J2.5 pour la lignée T MOLT4, et le réarrangement incomplet D2-J2.3 pour la lignée T HUT-78 ont été caractérisés sans besoin de séquençage supplémentaire.

Il est important de noter que les réarrangements incomplets D-J et D1-D2 sont non fonctionnels, mais représentent dans certains cas le seul marqueur biologique pour identifier une lymphoprolifération (leucémie ou lymphome B ou T).

**Exemple 8: Exemples de représentation des résultats d'une cartographie**

La figure 8 illustre un procédé permettant de regrouper toutes les données d'analyse du répertoire immunitaire sur 1 seule page, incluant notamment, la numération, et/ou la diversité de l'échantillon, et/ou l'intensité du signal, et/ou une comparaison avec une diversité de référence provenant d'un autre patient ou du même patient, une information permettant d'identifier que le patient a une lymphopénie en comparant le % de diversité obtenu avec un répertoire de référence, et/ou une information sur le nombre de clones détectés dans l'image, et/ou un graphique de représentation 2Dimensions, et/ou 3 dimensions, et/ou la liste de tous les réarrangements V-J classés par quantité décroissante en quantité de signal détecté, et/ou % de représentativité dans l'image. Le procédé dans ses étapes d'analyse permet ainsi

1- de compiler l'information collectée concernant l'historique clinique du patient et l'historique biologique de l'échantillon (résultats de numérations, cytométries, conditions de prélèvement...) ;
2- de fouiller les données par des méthodes statistiques et de corréler les résultats d'analyses du répertoire immunitaire (diversité combinatoire, ou autre approche) avec les données cliniques et biologiques. Il est notamment possible de classer tous les réarrangements V-D)J par ordre de fréquence de détection (intensité du signal de l'amplicon). Cet ordre varie d'un individu à l'autre en fonction des traitements et infections rencontrés par son répertoire de lymphocytes. Ceci permet d'avoir la signature du répertoire immunitaire d'une personne à un instant T. Cette signature peut être le marqueur biologique d'une pathologie, telle que les maladies auto-immunes, les allergies, les leucémies, les lymphomes, etc...

*N.B. :* il est important de noter que cette approche peut aussi être compatible avec d'autres approches d'analyses du répertoire immunitaire (toutes approches d'analyse de la diversité jonctionnelle, diversité d'appariement, diversité d'hypermutations somatiques, ...).

**Exemple 9 : Numération Diversité Lymphocytaire (NDL)**

La numération lymphocytaire effectuée lors d'une NFS ou d'un marquage en cytométrie donne le nombre de lymphocytes dans un prélèvement. Ce nombre est utilisé pour vérifier que le patient ne présente pas d'immunodéficience. La fourchette de "normalité" est classiquement comprise entre 1000 et 3200 lymphocytes/μL de sang. En dessous de 1000, le patient est considéré comme légèrement immunodéprimé, au dessous de 450 il s'agit d'une immunosuppression sévère. A l'inverse, au dessus de 3200, le patient est considéré comme potentiellement à risque d'expansion lymphocytaire. Il existes 2 points faibles à cette approche : le premier est la taille de la fourchette elle-même, qui correspond à un facteur 3 entre les valeurs minimale et maximale. Le 2e point encore plus gênant est que cette numération ne préfigure pas de la diversité réelle du répertoire immunitaire du patient (figure 9, graphe 1). La présente invention permet de coupler une numération classique avec une mesure de la diversité combinatoire V-J (figure 9, graphe 2).

Ceci peut notamment servir pour la sélection de patients dans le cadre d'essais cliniques, afin de tester des médicaments sur une population homogène, permettant d'interpréter les résultats. Ceci permet également d'effectuer un pronostic du risque infectieux d'un patient, et donc de pratiquer une médecine personnalisée, en adaptant l'immunosuppressivité d'un traitement au niveau de risque de mortalité par infection d'un patient.

Le graphique de la figure 9 distingue notamment les populations suivantes :

1. Numération faible (<1000 Ly/μL) et diversité combinatoire faible (<40%) : Il s'agit d'un patient immunosupprimé.

Pour action : Ne pas inclure ce patient dans l'étude clinique. Faire suivre ce patient par un hématologue.

2. Numération faible (<1000 Ly/μL) mais diversité combinatoire V-J normale (>65%) : le patient a un faible taux de lymphocytes circulants par rapport aux autres populations de cellules immunitaires, mais la qualité de sa défense immunitaire spécifique n'est pas spécialement remise en cause.

Pour action: s'il s'agit d'une étude concernant les personnes âgées, il serait intéressant d'inclure un bras de patients ayant cette caractéristique.

3. Numération normale (1000-3200Ly/μL) et diversité combinatoire faible (<40%) : il existe des "trous" dans le répertoire immunitaire. D'apparence normale, cette numération lymphocytaire cache un trouble d'immunodéficience pouvant être associée à une ou plusieurs expansions clonales. L'efficacité vaccinale peut être remise en cause.

Pour action: Nous recommandons de ne pas inclure ce patient dans l'étude clinique. Faire suivre ce patient par un onco-hématologue.

4. Numération normale (1000-3200Ly/μL) et diversité normale (>65%) : le répertoire immunitaire est sain.

Pour action: le patient peut être inclus dans l'étude clinique.

5. Numération au dessus de la normale, et diversité faible : zone à fort risque, l'échantillon ne contient qu'un ou quelques clones de lymphocytes.

Pour action: Ne pas inclure ce patient dans l'étude clinique. Faire suivre ce patient par un onco-hématologue.

6. Numération au dessus de la normale, mais diversité normale : lymphocytose généralisée, l'ensemble du système immunitaire spécifique de l'individu est suractivé, mais aucun élément ne laisse entendre une expansion monoclonale pouvant être reliée à une leucémie ou un lymphome.

Pour action: Ne pas inclure ce patient dans l'étude clinique. Faire suivre ce patient par un onco-hématologue pour suivre l'évolution de la lymphocytose.

Cette nouvelle technique de numération, dite « Numération Diversité Lymphocytaire », couplant l'analyse du répertoire immunitaire à la numération (quelle que soit la méthode de comptage) du nombre de lymphocytes du patient, est donc largement plus informative que la simple numération des lymphocytes. Elle permet notamment d'éviter le paradoxe de la numération cellulaire qui par moment donne l'impression qu'un patient a un nombre de lymphocytes considéré comme normal, mais qui en réalité présente des clones T ou B et donc a une diversité immunitaire faible (zone 3). A l'inverse, cela permet de s'assurer que des patients ayant une faible numération, ont tout de même une diversité immunitaire « correcte » (zone 2) leur permettant de se défendre contre les infections et donc de ne pas leur imposer un suivi médical aussi lourd que pour des patients réellement immunodéprimés, c'est-à-dire avec une faible numération et une faible diversité (zone 1). En outre, cela permet de faire la distinction entre au moins 2 catégories de patients ayant une très grande numération de lymphocytes. La première catégorie (zone 5) a une diversité faible qui s'associe à la présence d'un ou plusieurs clones, pouvant être due à une leucémie, un lymphome, une GVHD, une maladie auto-immune, une allergie, une réponse à un vaccin ou tout autre thérapie et immunothérapie. La 2e catégorie (zone 6) correspond à une lymphocytose, c'est-à-dire une expansion de la majorité du répertoire immunitaire.

**Exemple 10: stratégie de diagnostique personnalisé en onco-hématologie**

La figure 10 présente un arbre décisionnel en onco-hématologie par NDL.

Selon le schéma présenté, deux jeux d'amorces sont utilisés séquentiellement (le test avec le 2$^{ème}$ jeu étant optionnel) pour repérer les réarrangements VJ et DJ du TRB. Les amorces du 2$^{ème}$ jeu sont décalées par rapport à celles du 1$^{er}$ jeu, afin d'éviter de laisser échapper un clone à cause d'un polymorphisme apparu au niveau du site d'hybridation de l'amorce correspondante, ou d'une hypermutation somatique, *etc.*

Cette procédure permet de réaliser un diagnostic du niveau de risque de lymphoprolifération et en même temps du niveau de risque d'immunodéficence (à associer à un risque d'infection).

**Exemple 11 : comparaison de la diversité lymphocytaire de deux échantillons**

La figure 11 montre un graphique qui permet de comparer deux répertoires immunitaires afin d'identifier les ressemblances ou différences qui peuvent exister. Voici ci-dessous l'ordre de lecture et la description de chaque composante, ainsi qu'un exemple concret d'interprétation des résultats.

La région S pour « stable » représente le nombre de réarrangements observés dans les deux échantillons. La zone A pour « apparu » représente le nombre de réarrangements observés dans l'échantillon 2 mais pas dans l'échantillon 1. La zone D pour « disparu » représente le nombre de réarrangements qui sont observés dans l'échantillon 1 mais pas dans l'échantillon 2. Enfin, la zone Z pour « Jamais vu » représente le nombre de réarrangements qui ne sont observés ni dans l'échantillon 1 ni dans l'échantillon 2.

Interprétation : le nombre élevé de réarrangements observés en commun (184) dans les deux échantillons indique que les deux répertoires semblent identiques.

Le fait de représenter sur 1 graphique, la somme des apparitions, amplification A, disparition D, la non détection Z et la détection S (pour stable) de tous les réarrangements entre 2 cartographies immunitaire permet en 1 étape et de façon très visuelle de savoir si un patient est en cours de reconstitution (il aura beaucoup de A et un faible D) ou si il s'oriente vers une phase d'immunodéficience (il aura beaucoup de D et un faible A), enfin si le répertoire est stable entre les 2 cartographies (il y aura beaucoup de S).

Par ailleurs, le procédé décrit ici permet, en rapportant le nombre de réarrangements apparus ou disparus au temps écoulé entre les deux prélèvements d'échantillons, d'obtenir une indication sur la vitesse de reconstitution ou de diminution d'un répertoire. Ceci permet notamment de comparer l'effet stimulant ou à l'inverse immunosuppresseur d'un traitement.

**Exemple 12: détection précoce d'une lymphoprolifération (oligo)clonale, par PCR multi-n-plexe quantitative**

La figure 12 schématise un mode particulier de mise en oeuvre des procédés de l'invention, dans lequel les PCR multi-n-plexes sont suivies en temps réel, afin de détecter la présence d'une lymphoprolifération avant toute étape (optionnelle) de séparation des amplicons par électrophorèse.

L'amplification de PCR est effectuée dans un appareil de PCR quantitative en temps réelle, en utilisant par exemple le protocole décrit à l'exemple 2 ci-dessus, avec en plus une mesure de florescence à chaque cycle d'amplification.

Un mélange réactionnel spécifique, compatible à la fois avec la PCR quantitative et avec la PCR multi-n-plexe, a été mis au point pour cela. La polymérase utilisée est une enzyme de PCR longue telle que *HerculaseII* ou *IProof.* Le problème majeur pour la mise au point de ce mélange réactionnel est que le *SYBR green* modifie la migration de l'ADNg de manière non linéaire. Le biais de migration est en partie proportionnel à la quantité de *SYBR green* utilisée, et en partie dépendant de la quantité de l'amplicon.

Dans le cas présent, le niveau de multiplexage important et les variations d'intensité (fréquence) entre les amplicons rendent difficile la mise au point de la PCR en temps réel.

Les inventeurs ont déterminé la quantité de *SYBR green* suffisante pour avoir assez de signal fluorescent pour la PCRq (figures 12a et 12b) : la réaction utilise comme base le même mélange réactionnel que celui présenté plus haut, avec en outre du *SYBR green* en quantité supérieure à 0,4x final dans un volume réactionnel de 25μL, (soit 1 μl à 10x initial) et inférieure à 2x final, avec une préférence pour 1x final. Cette quantité est considérée comme un maximum, puisque si cette quantité est trop importante, le *SYBR green* entraîne un biais dans la migration, sans compter l'inhibition de PCR multiplexe qui a été observée si la quantité est supérieure à 1,5x final.

Dans les cas où les PCR multi-n-plexes sont effectuées avec 1 amorce spécifique d'une famille V et au moins 2 amorces spécifiques des gènes J, cette étape, représentée au graphe A de la figure 12c, permet de détecter la présence d'une famille V majoritaire (le signal détecté correspond à la somme des tous les réarrangements V-J de la famille V étudiée de le tube de Q-PCR en question).

Il y a plusieurs types de résultats attendus :

- REPERTOIRE IMMUNITAIRE DIVERSIFIE : dans un échantillon de PBMC ou de thymus sain, en utilisant 50ng d'ADNg par Q-PCR, toutes les familles V sont généralement détectées à partir de 20 cycles et jusqu'à 27 cycles maximum.

Dans un échantillon contenant aucun lymphocyte, aucun signal n'est détecté entre 20 et 27 cycles.

- REPERTOIRE COMPORTANT UN ou DES CLONE(S) T OU B MAJORITAIRE(S) : Dans un échantillon contenant majoritairement 1 clone V-(D)-J, la courbe correspondant à cette famille de gène V, sort entre 20 et 27 cycles, mais les courbes correspondant aux autres familles sont détectées au delà de 27 cycles. Ceci permet de distinguer une famille V majoritairement représentée dans un échantillon.

De la même manière, si n familles V sont présentes en grande quantité dans l'échantillon, nous détectons n courbes dans la fenêtre 20-27 cycles, et les autres familles V sont détectées plus tardivement >27 cycles, voire pas du tout détectées.

Ceci permet donc d'avoir, en temps réel et en moins de 2 heures, un diagnostic de lympho-prolifération sur 1 famille V au niveau génomique, à partir de la technique de PCR multi-n-plexe.

Le graphe B de la figure 12c illustre l'étape optionnelle d'analyse de la courbe de fusion. Cette étape permet de confirmer la présence d'un amplicon majoritaire dans un tube de PCR en 1 phase de *melting curve :* montée en température de 40°C à 95 °C (température de déshybridation totale de l'ADN). Durant cette phase, la fluorescence dans le tube est mesurée en continu. Si la courbe contient plusieurs pics de tailles similaires, c'est qu'il n'y a pas un amplicon majoritaire ; si à l'inverse 1 pic majoritaire est observé, cela vient appuyer le fait qu'un amplicon est majoritaire dans le tube de PCR en question.

Le graphe C de la figure 12c schématise la mesure de la diversité moléculaire. Cette étape permet une confirmation supplémentaire de la présence d'un amplicon majoritaire dans 1 tube de PCR par la mesure de la diversité moléculaire, produite par la combinaision entre la diversité de jonction (CDR3), la diversité combinatoire (V-J) et la diversité issue des hypermutations somatiques.

En bref, après avoir fait tomber la température très rapidement à 30° ou en-dessous, cette étape consiste à mesurer la vitesse de réhybridation des amplicons à température constante dans 1 tube de PCR, en mesurant la réémission de fluorescence.

- Dans le cas d'une grande diversité « moléculaire », le nombre d'amplicons différents dans 1 tube de PCR est grand et leur réhybridation est lente (de l'ordre de plusieurs couples de secondes, voir minutes) (courbe pleine). Si à l'inverse il n'y a qu'un amplicon majoritaire (avec 1 réarrangement V-J donné, 1 région de CDR3 donnée et des hypermutations somatiques données), la ré-hybridation de cet amplicon est rapide (de l'ordre de la seconde), ce qui produit alors une courbe plus verticale, comme celle représentée en pointillés.
- En résumé, cette étape de réhybridation peut être informative pour mesurer l'ordre de grandeur de la diversité « moléculaire » d'un échantillon, sans avoir besoin de faire migrer les produits de PCR. Plus la diversité moléculaire est grande et moins la somme des coefficients directeurs des courbes de chaque famille V est grande.

Au total, les 3 étapes A/B/C permettent d'utiliser les propriétés de la Q-PCR, pour identifier la présence d'une ou plusieurs familles V sur-représentés, sans faire migrer le produit de PCR multi-n-Plexe.

Néanmoins, il n'est pas possible de savoir quel est le nom du gène J sur-représenté, ni de mesurer la diversité combinatoire. Si le scientifique souhaite avoir ces informations supplémentaires, il doit réaliser une séparation des produits de PCR et analyser l'intensité des bandes correspondant à chacun des réarrangements (graphe D de la figure 12c).

Le graphe E de la figure 12c montre une synthèse graphique de la numération en fonction de la diversité combinatoire et moléculaire : connaissant la numération lymphocytaire de l'échantillon (mesurée indépendamment de cette expérience par comptage classique ou par cytométrie), il est possible de réaliser un graphique en 3 dimensions, avec la diversité combinatoire V-J, (en mesurant la somme des réarrangements V-J détectés), et la diversité moléculaire (en mesurant la somme des coefficients directeurs des pentes des familles de gènes V). Au final ce procédé permet de mieux caractériser un patient, en fonction de la diversité de son répertoire immunitaire.

En conclusion, ce procédé permet de suivre avec un niveau de finesse inégalé, l'évolution de la diversité immunitaire suite à un traitement. Il est notamment possible de diagnostiquer très précocement un niveau d'immunodéficience.

**Exemple 13 : mesure de l'efficacité d'un traitement**

La figure 13 présente deux exemples d'analyse des répertoires IgH et TCR à différents temps, pour suivre l'évolution de ces répertoires chez des patients, en réponse à un traitement.

Dans le cas d'une leucémie lymphoïde chronique B (LLC-B), cette pathologie est diagnostiquée en identifiant un signal fort pour 1 ou seulement quelques clones B. L'approche résolutive développée par l'invention permet d'identifier le nom du gène V et J, sans avoir besoin de séquencer le produit de PCR. Ceci est particulièrement intéressant pour suivre la maladie résiduelle. La figure 13 présente 2 cas de figures : un pronostique positif (patient 1) et l'autre négatif (patient 2). En effet, si le traitement est efficace (patient répondant), le test mesure une augmentation de la diversité combinatoire IgH, ainsi qu'une stabilité, voire augmentation des chaînes du TCRb, TCRg. A l'inverse, pour les patients non répondant (NR), la diversité d'un, voir des 3 répertoires diminue. Le patient est plus immunodéprimé (immunosupprimé) après le traitement.

Dans le cas présent, les graphiques résultent des mesures de diversité combinatoire des chaines IgH, TCRb et TCRg.

Ces résultats montrent comment cette approche permet d'avoir le résultat de plusieurs tests en 1 seule étape à partir d'un seul prélèvement de moins de 1ml de sang : diagnostiquer la LLC, identifier de quelle population B ou T provient la lympho-prolifération, identifier le ou les clones impliqué(s) grâce à leur réarrangement V-J afin d'être capable de le suivre entre différents organes, et le cas échéant trouver l'origine de la pathologie. Par ailleurs, la caractérisation du ou des clone(s) majoritaire(s) rend possible un suivi longitudinal de la pathologie, permettant à terme de mesurer à la fois la présence et l'impact de la maladie résiduelle sur le système immunitaire. En parallèle s'ajoute la mesure fine du niveau de reconstitution du répertoire immunitaire en fonction du traitement du patient : grâce à ce type de test il est possible d'évaluer rationnellement le niveau d'immunodéficience global du patient et de le corréler au niveau de risque infectieux.

Cette méthode présente donc un intérêt à plusieurs titres :

- Faciliter le classement des patients
- Diagnostiquer et caractériser un clone B ou T, par le nom de son réarrangement V-J, notamment en analysant simultanément tous les répertoires lymphocytaires T & B,
- Pronostiquer l'évolution d'une pathologie en fonction du ou des clones V-J impliqués dans la pathologie.
- Suivre la maladie résiduelle et faire une comparaison avec la diversité combinatoire V-J.
- Affiner le suivi de l'évolution des patients ayant une lymphocytose.
- Comparer la pathologie entre différentes sources d'échantillon : sang, rate, ganglion et tâcher de préciser si possible «l'origine de la pathologie» en quantifiant la présence du clone V-J entre ces différentes populations.
- Avancer vers une corrélation du risque infectieux avec le niveau de Numération Diversité Lymphocytaire (NDL) de chaque patient.

**Exemple 14: répartition des patients en fonction de leur diversité TCT/IG et de leur numération**

La figure 14 montre une représentation en 3 dimensions de la NDL (Numération/Diversité Lymphocytaire). Ce graphique regroupe la mesure de diversité des lymphocytes B et lymphocytes T, ainsi que la numération en % ou en valeur absolue du nombre de lymphocytes présents dans le prélèvement.

Le patient Répondant passe d'une zone à risque (avec une forte numération (env. 80%), une faible diversité IgH (8%) et une diversité moyenne voire faible pour le TCR (44%)) à une zone à moindre risque proche des prélèvements de PBMC (contrôle sain), avec une diminution de la numération (20%), une reconstitution de la diversité IgH (75%), TCRb (75%) et TCRg (90%).

A l'inverse, un patient Non Répondant (NR) reste dans la zone à risque et subit une immunosupression globale (diminution de la numération, qui ne résulte pas de l'efficacité du traitement, contrairement à ce que pourrait croire le clinicien s'il regardait seulement ce marqueur), une diminution du répertoire IgH, TCRb et TCRg.

Ceci illustre le paradoxe de la numération, dont l'utilisation seule peut aboutir à des erreurs d'interprétation. En effet, il est important de noter que la numération lymphocytaire entre ces deux prélèvements n'évolue pas dans le même sens que la diversité du répertoire combinatoire. Il est donc particulièrement intéressant de coupler les informations de numération et de diversité du répertoire afin de juger du réel état de santé du patient. En effet dans le cas présent, la numération leucocytaire passe de 78% à 21%, alors que la diversité combinatoire V-J passe de 8 à 75%. Cette approche permet de vérifier en une seule étape et à moindre coût que le patient en question a bénéficié d'un bon niveau de reconstitution du répertoire immunitaire et qu'il a moins de risque de déclarer une maladie infectieuse. Notons que ceci est cohérent avec la diminution de cellules CD19/CD5 indiquant la présence de la LCC qui passe de 84% à 4%.

**Exemple 15 : immunomonitorine dynamique du diagnostic initial au suivi de greffe de cellules souches.**

La figure 15 présente sous forme schématique l'application de la méthode de l'invention au suivi de la reconstitution du répertoire immunitaire chez un patient ayant subi une greffe de cellules souches (moelle).

Etape 1 : Mesure de la diversité immunitaire, diagnostic initial, détection du ou des clones de cellules T ou B (clones plus foncés).
Etape 2 : Evaluation de l'efficacité du traitement.
Etape 3 : Suivi de la maladie résiduelle.
Etape 4 : Evaluation de la préparation du répertoire du patient receveur (conditionnement de la greffe).
Etape 5 : Mesure de la reconstitution de la diversité V-J du patient et diagnostic précoce en cas de GVHD (maladie du greffon contre l'hôte).
Etape 6 : Evaluation de l'efficacité du traitement.
Etape 7 : Suivi de la maladie résiduelle.

L'étude longitudinale d'un patient par la méthode présentée ici permet un traitement personnalisé, adapté à chaque situation.

Le résultat du test présenté ici permet de mesurer le niveau de sévérité de la maladie et de s'assurer au maximum que le traitement est efficace, en évitant de traiter des patients qui seraient non répondants au traitement. Le répertoire immunitaire est ici utilisé comme un biomarqueur général de l'état de santé d'un individu. Il est utilisé à 2 niveaux : 1/ pour évaluer le risque infectieux du patient, 2/ tout en suivant de manière résolutive d'éventuels clones de lymphocytes T ou B pouvant être la signature d'une pathologie. Grâce à une étude longitudinale faite à partir de prises de sang successives chez un patient il est ainsi possible de suivre le niveau du répertoire immunitaire durant tout le traitement afin de s'assurer que ce dernier a bien été efficace et que le patient conserve un répertoire immunitaire diversifié pour se défendre contre les infections bactériennes ou virales. Ce diagnostic dynamique permet au clinicien d'adapter au mieux le traitement de son patient en lui proposant le médicament adéquat, à la bonne dose et au bon moment.

Un autre avantage du principe du test diagnostic préalable est d'éviter de trop traiter un patient « répondant » pour qui le traitement à la dose minimale est efficace.

**Exemple 16 : suivi d'un traitement *ex vivo* contre une GVHD**

Les 4 cartographies de la figure 16 illustrent les étapes 5 et 6 de la figure précédente. Les 4 cartographies représentent la diversité du répertoire immunitaire mesuré à partir de prélèvements d'un même patient à 2 temps avant le traitement (environ 200, 250 jours), et deux temps post-traitement (environ 300 et 600 jours). Les résultats concernant ces 4 prélèvements sont représentés selon l'intensité, la numération et la diversité.

1. La diminution de la diversité du répertoire correspond bien à l'apparition d'un clone. Le traitement *ex vivo* semble avoir inhibé l'expansion clonale (qui représente près de 10% du répertoire combinatoire suivi). L'intensité correspondant à ce réarrangement est systématiquement plus forte que l'intensité moyenne des réarrangements.
2. La proportion de lymphocytes dans les PBMC (numération) atteint son maximum vers J300, puis diminue légèrement vers J600.
3. Le niveau de diversité combinatoire suit une tendance inverse. L'augmentation de la proportion de lymphocytes T ne correspond pas à une augmentation de la diversité. Contrairement à ce qui serait attendu, la diversité diminue entre le point 1 et le point 2.

Le traitement *ex-vivo* semble avoir inversé la tendance. La reconstitution se fait en 2 phases : le répertoire récupère rapidement un niveau de diversité de 35% vers J300, puis la reconstitution est plus lente et atteint environ 40% vers J600.

Ces résultats montrent donc un répertoire à la périphérie initialement restreint en comparaison des contrôles positifs sur un répertoire sain de thymus et sur 4 donneurs sains. L'utilisation de la méthode de l'invention permet de mesurer qu'après J600, le niveau de reconstitution du répertoire TCR est proche de celui des donneurs sains. Cette approche permet donc d'évaluer l'efficacité d'un traitement et de voir son impacte sur la cinétique de reconstitution immunitaire. Enfin, l'utilisation du procédé de la présente invention a permis de vérifier que le profil du répertoire combinatoire des donneurs pouvait être conservé durablement chez le receveur, d'où une attention plus systématique à accorder à l'analyse du répertoire des donneurs pour expliquer l'évolution d'une allogreffe au cours du temps.

La méthode présentée ici est donc un outil particulièrement intéressants pour les cliniciens pratiquant des greffes de moelle, puisqu'elle permet, entre autres, de décrire et suivre des expansions clonales correspondant à une *Graft Versus Host* (GVH) chez le receveur. Elle permet en outre de mieux suivre la reconstitution du répertoire post-greffe de moelle osseuse. Le clinicien sera alors en mesure d'adapter le traitement de manière personnalisée, en mesurant avec plus de finesse le rapport GVH/GVL grâce au suivi de la diversité combinatoire V-J.

**<u>Exemple 17: Développement d'animaux transgéniques humanisés (ayant un répertoire immunitaire humain).</u>**

Les résultats sont présentés sur la figure 17.

Le présent procédé permet d'évaluer avec la trousse *ImmunTraCkeR* TRB humain, la qualité de reconstitution du répertoire immunitaire d'une souris transgénique « humanisée ». Dans cet exemple l'échantillon biologique étudié est issu de la rate d'une « souris immunodéficiente » (ne possédant pas de cellules immunitaires, ayant reçue une injection de cellules CD34+. Ces cellules ont la capacité de se diversifier et de reconstituer un système immunitaire. Le procédé permet de représenter la diversité du répertoire V-J dans un graphique 2 dimension. Chaque histogramme correspond à une famille V et au sein d'un histogramme, les sous-divisions correspondent à un gène J donné. La sous-division la plus basse de chaque histogramme correspond dans cet exemple à J2.7, et la plus haute correspond à J1.1. Il est possible de cribler les souris « humanisées », ayant un répertoire immunitaire pas totalement reconstitué (graphique de droite), des souris ayant un répertoire bien diversifié (graphique du centre), avec une répartition des réarrangements proche de celle observée dans un échantillon de PBMC humain (graphique de gauche).

**<u>Exemple 18 : utilisation de la méthode comme outil de criblage *in vitro* de molécules</u>**

L'étude représentée par la figure 18 montre l'évaluation de l'efficacité d'un épitope (un antigène) dans le cas du développement d'un traitement (vaccin), en mesurant la décroissance de diversité du répertoire immunitaire d'un échantillon contenant des lymphocytes T. Il est montré que plus la diversité diminue, et plus la sélection des lymphocytes spécifiques à l'épitope est grande.

Sur cette figure, 3 cas sont représentés :

A : L'épitope testé conduit à une importante sélection lymphocytaire, comme indiqué par la décroissance de la diversité.
B : L'épitope testé est peu sélectif comme cela peut être observé en comparaison avec le contrôle négatif.
C : contrôle négatif

Ce test de criblage d'épitopes permet d'identifier les épitopes stimulant le répertoire immunitaire. Il est possible de corréler le nombre de pics (nb de clones T) avec l'efficacité de sélection et d'activation du répertoire immunitaire.

La figure 18 est issue d'une étude qualitative et quantitative du répertoire TCR Beta murin à partir de l'ADN génomique. En utilisant les amorces décrites à l'exemple 21 ci-dessous (trousse *ImmunTraCkeR* TRB souris), pour obtenir la mesure de la diversité combinatoire mTRBV-J et de l'intensité et de l'homogénéité du répertoire, il est possible d'évaluer sur ce modèle animal l'efficacité de différents protocoles vaccinaux

Dans un autre cas d'évaluation de l'efficacité d'un épitope, illustré par la figure 18, le modèle pour lequel le répertoire T est étudié correspond à une culture *in vitro* de lymphocytes. Dans ce cas, l'étude de la diversité du répertoire T sur des échantillons d'ADNg, provenant de culture de lymphocytes *in vitro,* permet de mesurer l'expansion clonale de certains gènes TCR alpha, beta, gamma, delta et hIgH suite à une présentation antigénique par des cellules présentatrices.

Bien sûr, à la sélection de l'antigène pour induire une réponse immunitaire sélective, s'ajoute la capacité d'identifier le meilleur mode d'injection (nombre et fréquence d'injection, site d'injection et dose).

La figure 18 illustre une autre utilisation, de la méthode présentée ici. En effet, la sélection d'épitope peut être un événement non souhaité lors du développement de nouvelles approches thérapeutiques. Dans ce cas, il est important d'évaluer l'immunotoxicité (immunotoxicologie) pour éliminer les protéines induisant une activation du SI non attendue. Ceci permet de cribler des molécules, telles que les anticorps monoclonaux ou polyclonaux à visée thérapeutique, pour s'assurer qu'ils n'induisent pas une activation lymphocytaire inappropriée.

Ainsi, la méthode présentée ici est un outil particulièrement intéressant pour la recherche pré-clinique et clinique comme outils d'évaluation de nouvelles approches thérapeutiques et notamment vaccinales. Que ce soit sur des cellules en culture, chez l'animal modèle ou chez l'homme, l'outil ainsi décrit a pour objet de statuer sur la qualité de l'impact attendu sur le système immunitaire, soit par une activation spécifique ou par une absence d'activation.

Par extension, il est possible de faire le contrôle de la qualité du répertoire immunitaire en suivant un ou des clones caractérisé(s) pour des modèles d'études ou pour des productions de cultures de Lymphocytes avant réinjection (à visée thérapeutique) par exemple.

**Exemple 19 : comparaison avec d'autres technologie existantes**

La figure 19 montre la synthèse des résultats décrivant la comparaison de différentes techniques de suivi immunitaire (*immunomonitoring*) des répertoires des cellules immunitaires. Différents échantillons ont été étudiés par chacune des techniques. Les échantillons cellulaires ont été traités de façon contrôlée afin d'éviter tout biais dû à la préparation de l'échantillon.

Deux échantillons nommés « Thymus », correspondant à des cellules issues de thymus, ont été testés. Le Thymus est l'organe où a lieu la maturation et la sélection de certaines cellules immunitaires. Ces échantillons sont choisis car ils sont constitués d'un répertoire diversifié ne représentant pas de clonalité avec une présence des nombreuses cellules immunitaires ayant différents réarrangement (polyclonalité).

Deux autres échantillons ont été choisis pour représenter une population cellulaire beaucoup moins diverse que pour des échantillons de Thymus : l'échantillon « Jurkat » qui correspond à la lignée cellulaire jurkat (monoclonalité), ainsi que l'échantillon « Pool de Lignées T », qui correspond à un mélange dans des proportions connues de 3 lignées cellulaires (oligoclonalité). Enfin, un échantillon nommé « T(-) » correspond à témoin négatif, qui est un mélange cellulaire ne comportant pas de cellule immunitaire.

Ont été étudiées, en plus de la présente méthode avec la trousse « ImmunTraCkeR » TRB humain conforme à l'invention, 2 techniques permettant d'apporter différentes informations sur la qualité du répertoire immunitaire. D'une part, le test cellulaire « *Iotests* » (société Beckmann Coulter) qui est basé sur la reconnaissance d'antigène du répertoire Vbeta par cytométrie en flux, grâce à une trousse d'anticorps spécifiques. Ce test permet d'identifier la présence de 72% des familles et 59% des membres V. D'autre part, un test de biologie moléculaire « Biomed-2 » (consortium européen Biomed-2), qui est basé sur des approche de PCR multiplexées selon un principe différent de la présente invention, qui permet d'identifier si dans un échantillon donné il y a un clone très largement représenté.

L'analyse des résultats observés permet d'identifier que le test « ImmunTraCkeR » permet d'identifier lors de la mesure de la PCR en temps réel si l'échantillon est clonal (« Jurkat », oligoclonal (« Pool lignées T ») ou polyclonal (« Thymus »). L'analyse montre également que l'analyse des amplicons (réarrangements amplifiées selon la méthode « ImmunTraCkeR ») permet d'observer précisément quels clones sont présent en terme de réarrangement Vx-Jy, sans étape de séquençage, que se soit pour l'échantillon (« Jurkat », oligoclonal (« Pool lignées T ») ou polyclonal (« Thymus »). La représentativité de la diversité des combinaisons observées est donnée en pourcentage (par rapport à une diversité théorique où 100 % des réarrangements Vx-Jy seraient observés).

Le test « Biomed-2 » permet bien d'identifier la présence très majoritaire d'un clone dans l'échantillon. Le test « Biomed-2 » ne permet pas de discerner entre la présence d'un ou de quelques clones si le cas se présente, et ce test aura une signature montrant que l'échantillon est clonal même si il y a une oligoclonalité (« Pool lignées T ») : le test est positif (« On ») dans les deux cas.

Le test « Iotests », qui est un test effectué à partir des cellules fraiches, permet de discerner la clonalité de l'oligoclonalité et de la polyclonalité. Cependant, à la différence du test « ImmunTraCkeR », seulement les segments V beta sont étudiés. En outre, l'exhaustivité des anticorps Vbeta n'étant pas atteinte dans la trousse d'anticorps de ce test, le réarrangement V4 n'est pas observé alors qu'il l'est avec le test « ImmunTraCkeR ».

Les résultats de cette étude présentés figure 19 montrent que le test « ImmunTraCkeR » a une capacité technique plus grande que les deux autres test évalués pour l'analyse des répertoires immunitaires et qu'en conséquence ce test permet une étude plus étendue des répertoires immunitaires.

**Exemple 20 : protocole de production de tests hTCRB**

Pour utiliser la méthode qui est décrite dans ce document, et réaliser des analyses avec une des trousse « *ImmunTraCkeR* » dans de bonnes conditions, en plus de suivre les étapes de réalisation du test (présentés figure 20) il est préférable d'avoir produit le test en suivant un protocole de production et selon les bonnes pratiques de production.

Pour réaliser une production contrôlée de kits permettant de réaliser des tests hTRbeta (ou trousse « *immunTraCkeR* » TRbeta humain), il est nécessaire de s'assurer que la personne qui doit effectuer une production du test pour une utilisation à terme dans une étude sur des échantillons, respecte les différentes étapes énoncées ci-après en fonction de l'utilisation souhaitée.

Production 12 Tests hTRBeta

Préparation du « mix oligos »
Préparation du matériel
1 plaque Greiner (support)
3 barrettes de 8 puits + bouchons
microtubes de 0,5mL et 1,5mL
pipettes, embouts
$H_2O$ stérile
EB
Dilution des Vβ :
100μM à 20μM
Calcul : 20 x Vf / 100 = Vi
Donc, dans microtube 0,5mL: Vi μL d'oligo à 100μM + (Vf-Vi) d'EB.
20μM à 3,5μM
Calcul du volume nécessaire pour la production de 12kits + 10% : 12*1.67= 20.04+10%= 22μL
Calcul : 3,5 x 22 / 20 = 3,86μL
Donc, dans microtube 0.5mL: 3,85μL d'oligo à 20μM + 18,15μL d'$H_2O$
Dilution de bc1do2 & 2S7do1 :
100μM à 20μM
Calcul : 20 x Vf / 100 = Vi
Donc, dans microtube 0,5mL: Vi μL d'oligo à 100μM + (Vf-Vi) d'EB.
20μM à 3,5μM
Calcul du volume nécessaire pour la production de 12kits + 10% : 23*12*1,67=461+10%= 507,1μL
Calcul : 3,5 x 507.1 / 20 = 88,74μL
Dans tube Eppendorf de 1,5mL: 88,74μL d'oligo à 20μM + 418,36μL d'$H_2O$
Répartition des oligos Bc1do2 & 2S7do1
Pool Bcldo2 & 2S7do1 dilués à 3,5μM dans un tube à hémolyse stérile
Distribuer 44.1μL de ce pool dans chaque microtube 0,5mL contenant les Vβ dilués
Mélanger par refoulement, vortexer intensément puis centrifuger brièvement.
Répartition des oligos Vβ-Bc1do2-2S7do1
Distribuer 66μL du mix « Vβ-Bc1do2-2S7do1 » dans les barrettes de 8 puits en conservant l'ordre des Vβ suivant :
Barrette n°1 : Vβ2up2, Vβ3up2, Vβ4up_ex, Vβ5pool, Vβ6pool, Vβ7pool, Vβ9up_ex, Vβ10pool
Barrette n°2 : Vβ11up_ex, Vβ12poo1, Vβ13up1, Vβ14up_ex, Vβ15up_ex, Vβ16up1, Vβ18up1, Vβ19up2
Barrette n°3 : Vβ20-1up_ex, Vβ24up_ex, Vβ25up_int, Vβ27up2, Vβ28up_G, Vβ29up_G, Vβ30up1
Préparation des kits
Préparation du matériel
3 plaque Greiner (support)
3*12 barrettes de 8 puits + bouchons
pipettes, embouts
Production de 12 tests

A l'aide de la pipette multicanal, distribuer 5μL de mix des barrettes 1 à 3 (vol/puits=64uL) dans chaque puits des 12 barrettes correspondants.

Pour un lot de n tests, au moins deux tests seront utilisés pour contrôler la qualité et la conformité de la production. Parmi les différents contrôles effectués tout au long de la production pour permettre la traçabilité et la maîtrise de toutes dérives sur un lot, les contrôles fonctionnels qui permettent de vérifier la qualité avant validation du lot sont importants. A ce titre, la figure 21 propose des exemples sur trois échantillons de contrôle d'un test issu d'un lot de production. Ces trois échantillons sont ceux sur lesquels un contrôle systématique est réalisé au cours des productions. Les résultats sont comparés systématiquement aux précédents résultats pour identifier tout écart. Chaque nouveau résultat permet d'affiner la tendance et les limites hautes et basses acceptables pour que le test puisse être utilisé dans une étude du répertoire immunitaire.

Sur la figure 21, de haut en bas, est représenté le résultat attendu pour un répertoire immunitaire de thymus (polyclonalité), un répertoire immunitaire lorsque l'échantillon ne comporte qu'un clone très majoritaire (clonalité) et enfin un répertoire immunitaire lorsque l'échantillon comporte plusieurs clones (oligoclonalité).

Le protocole ci-dessus peut être adapté pour produire des tests TRB souris avec la trousse d'oligonucléotides tels que décrits dans l'exemple 21 ci-après, ainsi que pour toute autre production de test.

**Exemple 21 : Application de la méthode à l'analyse du locus TRB chez la souris**

Les protocoles et méthodes décrits dans le présent texte peuvent être adaptés à l'étude du répertoire TRB murin, en utilisant les amorces décrites dans le tableau ci-dessous.

Tableau 15

| nom du gène | nom oligonucléotide | taille (nt) | Distance avec la fin du gène V en pb | Séquence | SEQ ID No: | Orientation oligo-nucléotide |
|---|---|---|---|---|---|---|
| TRBV1 | mTRBV1up1 | 23 | 153 | GTGGCTGTTCACTCTGC GGAGTC | 99 | SENS |
| TRBV2 | mTRBV2up1 | 28 | 135 | TCAAAAACTTATGGACA ATCAGACTGCC | 100 | SENS |
| TRBV3 | mTRBV3up1 | 26 | 292 | CAGGACCCAAAGTCTTA CAGATCCCA | 101 | SENS |
| TRBV4 | mTRBV4up1 | 25 | 124 | TTGTAAACGAAACAGTT CCAAGGCG | 102 | SENS |
| TRBV5 | mTRBV5up1 | 24 | 227 | TTGGAATGTGAGCAACA TCTGGGA | 103 | SENS |
| TRBV12 | mTRBV12up1 | 25 | 105 | CCCAGCAGATTCTCAGT CCAACAGT | 104 | SENS |
| TRBV13 | mTRBV13up1 | 22 | 287 | TGGAGGCTGCAGTCACC CAAAG | 105 | SENS |
| TRBV14 | mTRBV14up1 | 28 | 130 | GTTATAGATAATTCACA GTTGCCCTCGG | 106 | SENS |
| TRBV15 | mTRBV15up1 | 24 | 362 | TTCCGTGTTCATAACTCC ACAGCG | 107 | SENS |
| TRBV16 | mTRBV16up1 | 23 | 62 | CTGAAGATCCAGAGCAC GCAACC | 108 | SENS |
| TRBV17 | mTRBV17up1 | 25 | 108 | TTTTGAGAAGTTCCAAT CCAGTCGG | 109 | SENS |
| TRBV19 | mTRBV19up1 | 27 | 113 | CGATCTATCTGAAGGCT ATGATGCGTC | 110 | SENS |
| TRBV20 | mTRBV20up1 | 25 | 199 | CTGTAGCTTGGTATCGT CAATCGCC | 111 | SENS |
| TRBV23 | mTRBV23up1 | 27 | 376 | AACACACCCAAATAATT TTCCTTGCTG | 112 | SENS |
| TRBV24 | mTRBV24up1 | 27 | 61 | TGGAAATCCTATCCTCT GAAGAAGACG | 113 | SENS |
| TRBV26 | mTRBV26up1 | 24 | 395 | TCTTTGACCTGGAGATT GCCAACC | 114 | SENS |
| TRBV29 | mTRBV29up1 | 24 | 99 | ATACAGGGTCTCACGGA AGAAGCG | 115 | SENS |
| TRBV30 | mTRBV30up2 | 23 | 155 | ATGGCAACTGCAAATGA AGGCTC | 116 | SENS |
| TRBV31 | mTRBV31up1 | 24 | 73 | ACGACCAATTCATCCTA AGCACGG | 117 | SENS |

(suite)

| nom du gène | nom oligonucléotide | taille (pb) | distance avec le début du gène J en pb | | | Orientation oligonucléotide |
|---|---|---|---|---|---|---|
| TRBJ1.7 | mTRBJ1.7dol | 26 | 2315 | GCATGGCTATTTGAAAC AGTGGCTCT | 118 | ANTI-SENS |
| TRBJ2.7 | mTRBJ2.7dol | 22 | 241 | CCTTGTCCTGGCTTGCG AGAGA | 119 | ANTI-SENS |

**Exemple 22 : Exemples de résultats obtenus par l'analyse en PCR quantitative du répertoire immunitaire combinatoire sur ADN génomique**

L'utilisation de PCR quantitative selon l'invention permet de classer rapidement un patient dans une des trois catégories suivantes : sain (pour ce qui concerne l'état de son système immunitaire), lymphoprolifération ou lymphopénie. La PCR en temps réel permet d'avoir des résultats en 2-3 heures au lieu de 5 heures pour une analyse par PCR « non en temps réel » nécessitant la migration des produits d'amplification. A terme, cette augmentation de la vitesse de rendu permettra de faire une surveillance sanitaire sur les patients.

Les trois cas de figure sont illustrés à la figure 22. La figure 22a présente un schéma des 3 cas de figures séparés ; la figure 22b présente un schéma de synthèse des 3 cas de figures. *N.B.* : Hormis pour le gène contrôle (dans cet exemple, le gène Actine), chaque courbe correspond à l'amplification PCR de la somme des réarrangements d'un gène V donné avec l'ensemble des gènes J suivis.

Le premier cas de figure (*sample A*) est celui d'un sujet « sain », où toutes les amplifications sont détectées de manière regroupée, dans une plage de *Ct* inférieur à 5 cycles. Et ceci entre 2 et 6 cycles après la détection du gène de ménage (*house keeping*).

Le deuxième schéma de la figure 22a est caractéristique d'une « lymphoprolifération » (*sample B*), où au moins une amplification d'un gène V est détectée plus tôt que le groupe des autres courbes (ce qui correspond à une monoclonalité si une courbe « sort » avant les autres, et à une oligoclonalité si c'est le cas de quelques courbes). En fonction du niveau de lymphoprolifération, cette (ou ces quelques) courbe(s) sera (seront) plus ou moins proche(s) de la courbe du gène Actine (d'autant plus proche(s) que le degré de lymphoprolifération est important, la détection pourra même être observée avant le gène « housekeeping ») ; à l'inverse, la détection des autres réarrangements sera plus tardive lorsque le degré de lymphoprolifération sera plus important (les courbes sont donc décalées vers la droite). Rmq : si la lymphoprolifération est monoclonale, l'amplification du réarrangement correspondant peut être détectée en même temps que l'actine (dans certains cas, notamment si les amorces correspondantes ont une efficacité d'amplification supérieure à celle du couple d'amorces pour le gène de l'actine, cette détection pourrait être avant l'actine). Si la lymphoprolifération est monoclonale, les autres réarrangements seront quasi-indétectés, ou seront détectés après un nombre de cycle important (plus de 5 cycles après la détection du réarrangement majoritaire).

En cas de détection précoce d'un réarrangement non présent parmi les 10 réarrangements les plus représentés d'habitude, il y a suspicion de clonalité, et il est nécessaire de suivre le patient pour confirmer ou infirmer ce risque.

Les troisième schéma de la figure 22a représente un cas de « lymphopénie » (*sample C*) : malgré une détection du gène contrôle (exemple actine) à un nombre de cycle similaire aux sujets sains, démontrant une quantité d'ADNg comparable et une absence d'inhibition de la PCR, la détection des réarrangements est très tardive. Ceci est le signe d'une présence très faible de réarrangements, ce qui montre qu'il y a très peu de lymphocytes dans l'échantillon.

**Exemple 23 : Interprétation des résultats obtenus par QPCR**

***Identification directe, par PCR quantitative, d'une pénurie de diversité des Lymphocytes associée à une lymphopénie***

Les inventeurs ont défini deux nouveaux indices pour interpréter les résultats obtenus par les méthodes selon l'invention, notamment pour identifier une pénurie de diversité des lymphocytes, notamment associée à une lymphopénie.

Le premier indice, nommé ici « ratio de divpénie » (ou « *divpenia* ratio » en anglais), se calcule de la façon suivante :

« ratio de divpénie » = [cycle de sortie du contrôle qualité actine] / [moyenne (ou médiane) des cycles de sortie des réarrangements V de l'échantillon]

Bien entendu, cet indice, qui a pour but d'identifier facilement une pénurie de diversité, peut être adapté par l'homme de l'art à tout autre gène de contrôle que l'actine. Dans le cas présent, un ratio inférieur à 0,78 et plus particulièrement inférieur à 0,74 (mesuré en QPCR) est indicateur d'une diversité combinatoire immunitaire faible (situation qualifiée ici de « divpénie »).

Cet indice, mesuré dans les trois situations présentées à l'exemple 22 ci-dessus, donne les résultats suivants :

- Echantillon A « Normal »

« ratio de divpénie » = 20 cycles (Ct Actine) / Moyenne (ou médiane) réarrangements 25 cycles = 0.80

- Echantillon C « Lymphopénie »

« ratio de divpénie » = 20 cycles /28 cycles = 0.71

Autre moyen d'identifier une lymphopénie en QPCR : dans le cas ou les 2 échantillons ont une détection du gène contrôle au même nombre de cycles, un autre moyen d'identifier une lymphopénie est de soustraire directement la moyenne du nombre de cycles de détection des réarrangements d'un échantillon de sujet « sain » à la moyenne du sujet testé. Dans cet exemple, entre l'échantillon A (sain) et l'échantillon C, on observe une différence des moyennes de Ct de 28 - 25 = 3 cycles d'écart moyen, soit environ $2^3$ = 8 fois moins de signal détecté en moyenne chez le patient lymphopénique.

***Identification d'une lymphoprolifération directement par QPCR.***

Dans un échantillon donné, la lymphoprolifération (d'un lymphocyte) s'associe à l'augmentation de la détection du réarrangement V-J correspondant. En QPCR ceci consiste à détecter le gène V correspondant dès un faible nombre de cycles de QPCR. Plus la lymphoprolifération de ce lymphocyte est importante et plus le lymphocyte représente une proportion importante dans l'échantillon. Il en résulte que la détection du réarrangement correspondant est réalisée à un nombre de cycles proche du gène Actine (voire avant, cf remarque dans l'exemple 22). Inversement, les autres réarrangements de l'échantillon sont moins fréquents et leur détection nécessite un plus grand nombre de cycles de QPCR. Au final ceci abouti à l'augmentation de la différence des Ct correspondants à la détection du 1$^{er}$ et du dernier réarrangements détectés. Si cette différence (delta Ct) est supérieure à n cycles, ceci indique la présence d'une lymphoprolifération, qui est d'autant plus importante que ce delta est grand.

Cette approche permet donc de détecter la présence d'une lymphoprolifération en QPCR et de caractériser le nom de la famille V impliquée. Pour identifier le réarrangement V-J, il suffit par la suite de faire migrer les produits de QPCR et de le caractériser en fonction de la taille attendue.

*N.B. :* connaissant la liste des 10 réarrangements TRB majoritairement détectés dans les PBMC, il est aussi possible dans certains cas d'avoir des suspicions de clonalité « émergente », en détectant des familles de gènes TRBV non présentes dans cette liste.

- Indice ΔCt = [Ctmax] – [Ctmin] pour un échantillon.

En pratique, on considère qu'il n'y a pas de lymphoprolifération si ΔCt < 6 cycles et qu'un indice ΔCt > 6 cycles indique une lymphoproliferation d'un d'un clone, qui représente alors au moins 10% des lymphocytes de l'échantillon.

- Echantillons A et C :

ΔCt = 27-24 = 3 cycles

- Echantillon B :

  ∆Ct = 30-23 = 7 cycles, ce qui indique bien une lymphoprolifération.

*N.B.* : il est préférable d'utiliser un ratio pour mesurer la pénurie de diversité et une différence de Ct pour identifier un clone, ou inversement, afin d'éviter les erreurs et de pouvoir travailler de manière normalisée vis-à-vis de la quantité d'ADN génomique de l'échantillon, qui est mesurée par la détection du gène contrôle (gène de ménage ou autre).

Le tableau ci-dessous décrit la détection par PCR quantitative des Cycles de sorties (Ct) des 10 premiers gènes V détectés, sur 3 échantillons de PBMC différents. Ce tableau donne le Ctmax (dernier produit de PCR détecté), le Ct min (réarrangement détecté en premier), Delta Ct entre CtMax et Ct Min, la médiane des Ct de l'échantillon (hors CQ actine) la moyenne de Ct de l'échantillon (hors Ct Actine), le «ratio de divpénie» entre Ct Actine et la médiane ou la moyenne des Ct.

| **PBMC Per Genes** | **Ct Cycles** | **Indices** | **Valeur** | **Interprétation** |
|---|---|---|---|---|
| **QC Actine** | **22,02** | | | |
| BV4 | 24,18 | | | |
| BV24 | 24,28 | CtMax | 29,32 | |
| BV20 | 24,46 | CtMin | 24,18 | |
| BV27 | 24,56 | **Delta Ct Max-Min** | 5,14 | **Risque très faible de lymphoprolifération** |
| BV6 | 24,7 | | | |
| BV5 | 25,53 | Médiane Ct | 26,13 | |
| BV7 | 25,72 | **ratio de divpénie [Ct actine] /[Mediane Ct]** | **0,842709529** | **Risque faible de divpénie** |
| BV30 | 25,95 | **Moyenne Ct** | 26,29 | |
| BV12 | 26 | **ratio de divpénie [Ct actine] /[Moyenne Ct]** | **0,837580829** | **Risque faible de divpénie** |
| BV15 | 26,07 | | | |
| **PBMC Br** Gènes | **Ct Cycles** | **Indices** | **Valeur** | **Interprétation** |
| **QC Actinie** | **19,99** | | | |
| BV20 | 23,7 | | | |
| BV6 | 24,17 | CtMax | 28,5 | |
| BV5 | 24,26 | CtMin | 23,7 | |
| BV24 | 24,48 | **Delta Ct Max-Min** | **4,8** | **Risque très faible de lymphoprolifération** |
| BV7 | 24,59 | | | |
| BV27 | 24,63 | Médiane Ct | 25,27 | |
| BV2 | 24,65 | **ratio de divpénie [Ct actine] /[Mediane Ct]** | **0,791056589** | **Risque faible de divpénie** |
| BV30 | 24,7 | Moyenne Ct | 25,54 | |
| BV4 | 24,72 | **ratio de divpénie [Ct actine] /[Moyenne Ct]** | **0,782693814** | **Risque faible de divpénie** |
| BV29 | 24,93 | | | |

| **PBMC SH Gènes** | **Ct Cycles** | **Indices** | **Valeur** | **Interprétation** |
|---|---|---|---|---|
| **QC Actine** | 23,45 | | | |
| BV20 | 23,51 | | | |

(suite)

| PBMC SH Gènes | Ct Cycles | Indices | Valeur | Interprétation |
|---|---|---|---|---|
| BV6 | 23,77 | CtMax | 27,56 | |
| BV4 | 23,83 | CtMin | 23,51 | |
| BV5 | 24,54 | **Delta Ct Max-Min** | 4,05 | **Risque très faible de lymphoprolifération** |
| BV30 | 24,72 | | | |
| BV28 | 24,91 | Médiane Ct | 25,79 | |
| BV7 | 24,98 | **ratio de divpénie [Ct actine] /[Mediane Ct]** | **0,909267158** | **Risque faible de divpénie** |
| BV27 | 24,98 | Moyenne Ct BV detection | 25,73 | |
| BV9 | 25,32 | **ratio de divpénie [Ct actine] /[Moyenne Ct]** | **0,911387485** | **Risque faible de divpénie** |
| BV24 | 25,45 | | | |

Le tableau suivant illustre la détection d'une lymphoprolifération et l'apparition d'une pénurie de diversité (« divpenie ») associée : chaque analyse est réalisée à quantité d'ADNg constante (50ng d'ADNg par réaction de PCR), sur des échantillons composés d'une répartition de PBMC dilués respectivement dans 10%, 50%, 80% et 100% de lignées cellulaires T SUP (ayant un réarrangement TRBV9).

| PBMC 90% + SUP 10% | | | | |
|---|---|---|---|---|
| Gène | Ct | Indice | Valeur | Interprétation |
| CQi | 22,96 | | | |
| BV9 | 23,75 | | | |
| BV5 | 26,2 | CtMax | 30,47 | |
| BV20 | 26,2 | CtMin | 23,75 | |
| BV6 | 26,5 | Delta Ct Max-Min | 6,72 | Risque modéré de lymphoprolifération |
| BV27 | 26,67 | | | |
| BV4 | 26,69 | Médiane Ct | 27,85 | |
| BV3 | 26,84 | Divpenia ratio [Ct actine]/[Mediane Ct] | 0,824416517 | Risque faible de divpénie |
| BV19 | 27,07 | Moyenne Ct | 27,66695652 | |
| BV30 | 27,11 | Divpenia ratio [Ct actine]/[Moyenne Ct] | 0,829870824 | Risque faible de divpénie |
| BV24 | 27,53 | | | |
| BV2 | 27,71 | | | |
| BV12 | 27,85 | | | |
| BV10 | 27,98 | | | |
| BV18 | 28,04 | | | |
| BV11 | 28,09 | | | |
| BV29 | 28,11 | | | |
| BV7 | 28,12 | | | |
| BV14 | 28,44 | | | |
| BV25 | 28,6 | | | |
| BV15 | 29 | | | |
| BV28 | 29,38 | | | |
| BV13 | 29,99 | | | |
| BV16 | 30,47 | | | |
| PBMC 50% + SUP 50% (clone of TRBV9) | | | | |
| Gène | Ct | Indice | Valeur | Interprétation |
| CQi | 23,03 | | | |
| BV9 | 22,12 | | | |
| BV5 | 24,54 | CtMax | 30,77 | |

(suite)

| | | | | |
|---|---|---|---|---|
| PBMC 50% + SUP 50% (clone of TRBV9) | | | | |
| Gène | Ct | Indice | Valeur | Interprétation |
| BV20 | 24,55 | CtMin | 22,12 | |
| BV19 | 25,73 | Delta Ct Max-Min | 8,65 | Risque fort de lymphoprolifération |
| BV24 | 25,84 | | | |
| BV7 | 26,05 | Médiane Ct Divpenia ratio [Ct | 27,22 | |
| BV4 | 26,36 | actine]/[Médianne Ct] | 0,846069067 | |
| BV10 | 26,69 | Moyenne Ct | 27,23130435 | |
| BV30 | 26,75 | Divpenia ratio [Ct actine]/[Moyenne Ct] | 0,845717844 | Risque faible de divpénie |
| BV11 | 26,98 | | | |
| BV3 | 27,05 | | | |
| BV27 | 27,22 | | | |
| BV18 | 27,39 | | | |
| BV12 | 27,43 | | | |
| BV25 | 27,66 | | | |
| BV6 | 28,13 | | | |
| BV14 | 28,16 | | | |
| BV29 | 28,56 | | | |
| BV28 | 28,87 | | | |
| BV13 | 29,24 | | | |
| BV2 | 29,72 | | | |
| BV16 | 30,51 | | | |
| BV15 | 30,77 | | | |
| PBMC 20% + SUP 80% (clone of TRBV9) | | | | |
| Gène | Ct | Indice | Valeur | Interprétation |
| CQi | 23,01 | | | |
| BV9 | 21,23 | | | |
| BV5 | 25,25 | CtMax | 31,1 | |
| BV20 | 25,9 | CtMin | 21,23 | |
| BV24 | 26,45 | Delta Ct Max-Min | 9,87 | Risque très fort de lymphoprolifération |
| BV4 | 27,31 | | | |
| BV6 | 27,33 | Médiane Ct Divpenia ratio [Ct | 28,03 | |
| BV3 | 27,43 | actine]/[Médianne Ct] | 0,820906172 | |
| BV27 | 27,45 | Moyenne Ct | 27,89782609 | |
| BV11 | 27,75 | Divpenia ratio [Ct actine]/[Moyenne Ct] | 0,825 | Risque faible de divpénie |
| BV12 | 27,79 | | | |
| BV30 | 27,89 | | | |
| BV29 | 28,03 | | | |
| BV19 | 28,09 | | | |
| BV7 | 28,19 | | | |
| BV18 | 28,38 | | | |
| BV2 | 28,43 | | | |
| BV10 | 28,82 | | | |
| BV13 | 29,16 | | | |
| BV14 | 29,21 | | | |
| BV15 | 29,36 | | | |
| BV25 | 30,27 | | | |
| BV28 | 30,83 | | | |
| BV16 | 31,1 | | | |

(suite)

| PBMC 0% + SUP 100% (clone of TRBV9) | | | | |
|---|---|---|---|---|
| Gène | Ct | Indice | Valeur | Interprétation |
| CQi | 23,62 | | | |
| BV9 | 21,03 | | | |
| BV5 | 25,35 | CtMax | 34,89 | |
| BV24 | 27,06 | CtMin | 21,03 | |
| BV14 | 30,24 | Delta Ct Max-Min | 13,86 | Risque très fort de lymphoprolifération |
| BV18 | 30,53 | | | |
| BV20 | 30,91 | Médiane Ct | 32,38 | |
| BV10 | 31,39 | Divpenia ratio [Ct actine]/[Médianne Ct] | 0,729462631 | |
| BV11 | 31,92 | Moyenne Ct | 31,53913043 | |
| BV4 | 32,1 | Divpenia ratio [Ct actine]/[Moyenne Ct] | 0,749 | Risque fort de divpénie |
| BV12 | 32,14 | | | |
| BV19 | 32,28 | | | |
| BV6 | 32,38 | | | |
| BV27 | 32,46 | | | |
| BV15 | 32,58 | | | |
| BV25 | 32,59 | | | |
| BV2 | 32,79 | | | |
| BV16 | 32,9 | | | |
| BV7 | 33,05 | | | |
| BV30 | 33,6 | | | |
| BV13 | 34,13 | | | |
| BV3 | 34,2 | | | |
| BV28 | 34,88 | | | |
| BV29 | 34,89 | | | |

**Exemple 24 : Exemple de résultats en vaccinologie : association de la mesure du nombre de lymphocytes T régulateur et répertoire immunitaire**

Cet exemple approfondit ce qui a déjà été présenté à l'exemple 18 ci-dessus.

Grâce à la méthode de l'invention, les inventeurs ont pu observer que lorsqu'un individu a une diversité combinatoire initiale > 50%, le fait d'observer une baisse de diversité combinatoire associée à une baisse de détection de cellules T régulatrices ou de cellules CD25++ est un indicateur de l'efficacité de la vaccination.

Ceci est illustré à la figure 23, qui présente un graphique de la mesure du nombre de lymphocytes T régulateurs en fonction de la diversité immunitaire des lymphocytes T. Chaque point correspond à un individu (dans cet exemple ce sont des souris). Le graphique montre 3 groupes d'individus. A/ avant vaccination ou injection de PBS (= témoin négatif), B/ après vaccination par protocole de vaccination n°1, C/ après vaccination par protocole de vaccination n°2.

Comme déjà observé à l'exemple 18, la vaccination de souris ayant une diversité immunitaire normale (>70% de diversité combinatoire) induit une diminution de la diversité. Cette diminution de la diversité est en outre associée à une diminution du nombre de lymphocytes T régulateurs et cellules CD4+25++. Dans le cas de figure présenté, il est possible de distinguer des différences entre les 2 protocoles de vaccination au niveau de leur impact sur le système immunitaire. D'un point de vu fonctionnel, les souris atteintes d'un cancer et ayant reçu le protocole n°2 (groupe C) ont une survie plus grande que les souris ayant reçu le protocole n°1. Ces résultats indiqueraient qu'il est possible de prédire l'efficacité vaccinale en associant la mesure de la diversité immunitaire à la mesure du nombre de lymphocytes T régulateurs. Ceci permet donc d'évaluer et éventuellement de comparer l'efficacité de différents protocoles vaccinaux, pour tester l'effet de tous les facteurs impliqués, tels que, par exemple, la nature de l'adjuvant, le type de vaccin (peptides, recombinant,...), la dose, le mode d'administration et/ou le lieu d'injection, la fréquence d'administration, *etc.*.

L'observation globale des résultats montre que d'une manière générale, le fait de vacciner les souris permet de rendre plus homogène le % des populations et la diversité des souris au sein d'un même groupe. Sur les graphiques, cela correspond à une « surface » plus grande pour les groupes contrôle PBS, que pour les groupes de souris vaccinées. En effet pour les souris témoins non traitées, de grandes différences sont observées, tant au niveau des phénotypes que de la diversité combinatoire. Après vaccination, la répartition du phénotype des populations lymphocytaires étudiées ainsi que la diversité combinatoire du répertoire sont plus homogènes au sein d'un même groupe de souris.

Un protocole vaccinal induisant une diminution d'un facteur 2 du nombre de lymphocytes T régulateurs dans un ganglion, dans la rate ou à la périphérie, associée à une diminution de la diversité immunitaire supérieure à 10% et de préférence supérieure à 15% par rapport au groupe avant vaccination ou à un groupe contrôle sera, *a priori,* un protocole efficace. *N.B.* : d'après les observations des inventeurs, plus la diminution de ces 2 biomarqueurs est importante et plus le protocole de vaccination aura un effet sur le système immunitaire. La mesure de la diversité lymphocytaire, associée le cas échéant à la mesure du nombre de lymphocytes T régulateurs, permet donc de sélectionner un protocole de vaccination plus efficace parmi différents protocoles.

Dans cette même idée, toute stratégie visant à diminuer de manière transitoire la quantité de T régulateurs dans l'organisme permettrait d'augmenter l'efficacité vaccinale et anti-tumorale.

**REFERENCES**

Aude-Garcia, C., Gallagher, M., Marche, P. N., and Jouvin-Marche, E. (2001). Preferential ADV-AJ association during recombination in the mouse T-cell receptor alpha/delta locus. Immunogenetics 52, 224-230.

Baum, P. D., and McCune, J. M. (2006). Direct measurement of T-cell receptor repertoire diversity with AmpliCot. Nat Methods 3, 895-901.

Baum, T. P., Hierle, V., Pasqual, N., Bellahcene, F., Chaume, D., Lefranc, M. P., Jouvin-Marche, E., Marche, P. N., and Demongeot, J. (2006). IMGT/GeneInfo: T cell receptor gamma TRG and delta TRD genes in database give access to all TR potential V(D)J recombinations. BMC Bioinformatics 7, 224.

Baum, T. P., Pasqual, N., Thuderoz, F., Hierle, V., Chaume, D., Lefranc, M. P., Jouvin-Marche, E., Marche, P. N., and Demongeot, J. (2004). IMGT/GeneInfo: enhancing V(D)J recombination database accessibility. Nucleic Acids Res 32, D51-54.

Berek, C., Griffiths, G. M., and Milstein, C. (1985). Molecular events during maturation of the immune response to oxazolone. Nature 316, 412-418.

Bogue, M., Gilfillan, S., Benoist, C., and Mathis, D. (1992). Regulation of N-region diversity in antigen receptors through thymocyte differentiation and thymus ontogeny. Proc Natl Acad Sci U S A 89, 11011-11015.

Bonarius, H. P., Baas, F., Remmerswaal, E. B., van Lier, R. A., ten Berge, I. J., Tak, P. P., and de Vries, N. (2006). Monitoring the T-cell receptor repertoire at single-clone résolution. PLoS ONE 1, e55.

Cabaniols, J. P., Fazilleau, N., Casrouge, A., Kourilsky, P., and Kanellopoulos, J. M. (2001). Most alpha/beta T cell receptor diversity is due to terminal deoxynucleotidyl transferase. J Exp Med 194, 1385-1390.

Chaudhuri, J., Tian, M., Khuong, C., Chua, K., Pinaud, E., and Alt, F. W. (2003). Transcription-targeted DNA deamination by the AID antibody diversification enzyme. Nature 422, 726-730.

Cochet, M., Pannetier, C., Regnault, A., Darche, S., Leclerc, C., and Kourilsky, P. (1992). Molecular detection and in vivo analysis of the specific T cell response to a protein antigen. Eur J Immunol 22, 2639-2647.

Davis, M. M., and Bjorkman, P. J. (1988). T-cell antigen receptor genes and T-cell recognition. Nature 334, 395-402.

Douek, D. C., McFarland, R. D., Keiser, P. H., Gage, E. A., Massey, J. M., Haynes, B. F., Polis, M. A., Haase, A. T., Feinberg, M. B., Sullivan, J. L., et al. (1998). Changes in thymic function with age and during the treatment of HIV infection. Nature 396, 690-695.

Fugmann, S. D., Lee, A. I., Shockett, P. E., Villey, I. J., and Schatz, D. G. (2000). The RAG proteins and V(D)J recombination: complexes, ends, and transposition. Annu Rev Immunol 18, 495-527.

Fuschiotti, P., Pasqual, N., Hierle, V., Borel, E., London, J., Marche, P. N., and Jouvin-Marche, E. (2007). Analysis of the TCR alpha-chain rearrangement profile in human T lymphocytes. Mol Immunol 44, 3380-3388.

Hamblin, T. J., Davis, Z., Gardiner, A., Oscier, D. G., and Stevenson, F. K. (1999). Unmutated Ig V(H) genes are associated with a more aggressive form of chronic lymphocytic leukemia. Blood 94, 1848-1854.

Huang, C., and Kanagawa, O. (2001). Ordered and coordinated rearrangement of the TCR alpha locus: role of secondary rearrangement in thymic selection. J Immunol 166, 2597-2601.

Jouvin-Marche, E., Aude-Garcia, C., Candeias, S., Borel, E., Hachemi-Rachedi, S., Gahery-Segard, H., Cazenave, P. A., and Marche, P. N. (1998). Differential chronology of TCRADV2 gene use by alpha and delta chains of the mouse TCR. Eur J Immunol 28, 818-827.

Kotani, A., Okazaki, I. M., Muramatsu, M., Kinoshita, K., Begum, N. A., Nakajima, T., Saito, H., and Honjo, T. (2005). A target selection of somatic hypermutations is regulated similarly between T and B cells upon activation-induced cytidine deaminase expression. Proc Natl Acad Sci U S A 102, 4506-4511.

Lang, R., Pfeffer, K., Wagner, H., and Heeg, K. (1997). A rapid method for semiquantitative analysis of the human V beta-repertoire using TaqManR PCR. J Immunol Methods 203, 181-192.

Lefrancs, The Immunoglobulin Facts Book 2001.

Lefrancs The T cell receptor Facts Book 2001.
Oprea, M., and Kepler, T. B. (1999). Genetic plasticity of V genes under somatic hypermutation: statistical analyses using a new resampling-based methodology. Genome Res 9, 1294-1304.
Pannetier, C., Even, J., and Kourilsky, P. (1995). T-cell repertoire diversity and clonal expansions in normal and clinical samples. Immunol Today 16, 176-181.
Pasqual, N., Gallagher, M., Aude-Garcia, C., Loiodice, M., Thuderoz, F., Demongeot, J., Ceredig, R., Marche, P. N., and Jouvin-Marche, E. (2002). Quantitative and qualitative changes in V-J alpha rearrangements during mouse thymocytes differentiation: implication for a limited T cell receptor alpha chain repertoire. J Exp Med 196, 1163-1173.
Pham, T., Belzer, M., Church, J. A., Kitchen, C., Wilson, C. M., Douglas, S. D., Geng, Y., Silva, M., Mitchell, R. M., and Krogstad, P. (2003). Assessment of thymic activity in human immunodeficiency virus-negative and -positive adolescents by real-time PCR quantitation of T-cell receptor rearrangement excision circles. Clin Diagn Lab Immunol 10, 323-328.
Rytkonen, M. A., Hurwitz, J. L., Thompson, S. D., and Pelkonen, J. (1996). Restricted onset of T cell receptor alpha gene rearrangement in fetal and neonatal thymocytes. Eur J Immunol 26, 1892-1896.
Van den Beemd, van Dongen et al. (2000), "Flow cytometric detection of clonality in mature T-cell malignancies by use of a Vb antibody kit", ISAC Abstract.
Wang, F., Huang, C. Y., and Kanagawa, O. (1998). Rapid deletion of rearranged T cell antigen receptor (TCR) Valpha-Jalpha segment by secondary rearrangement in the thymus: role of continuous rearrangement of TCR alpha chain gene and positive selection in the T cell repertoire formation. Proc Natl Acad Sci U S A 95,11834-11839.

## Revendications

**1.** Méthode d'analyse *in vitro* de la diversité combinatoire des réarrangements V(D)J d'au moins un locus génétique choisi parmi les loci TRA, TRB, TRG, TRD, IGH, IGK et IGL d'un individu, à partir d'ADN génomique provenant d'un échantillon biologique dudit individu, comprenant les étapes suivantes :

A) amplification de fragments dudit ADN génomique par des réactions d'amplification en chaîne par polymérase (ACP ou *PCR*) multiplexes, dont l'une au moins est une PCR multi-n-plexe avec n≥2, mettant en oeuvre, dans une même réaction, n couples d'amorces différents, permettant chacun l'amplification d'au moins deux fragments d'ADN caractéristiques d'au moins deux réarrangements chromosomiques différents, effectuée avec une combinaison d'au moins 3 amorces, constituant au moins 2 couples d'amorces distincts présentant les caractéristiques suivantes :

(i) trois desdites au moins 3 amorces constituent 2 couples d'amorces distincts ;
(ii) chaque couple d'amorces est constitué d'une amorce s'hybridant spécifiquement en amont et/ou dans un gène V ou D donné et d'une amorce s'hybridant spécifiquement en aval et/ou dans un gène J donné, de façon à permettre l'amplification d'au moins deux fragments caractéristiques de deux réarrangements V(D)J ou D-J distincts;
(iii) les amorces sont thermodynamiquement compatibles ;
(iv) les amorces sont choisies de telle façon que les fragments amplifiés avec le premier couple d'amorces peuvent être distingués des fragments amplifiés avec le second couple d'amorces ;

B) détection des produits d'amplification obtenus à l'étape A ;
C) interprétation des résultats.

**2.** Méthode selon la revendication 1, dans laquelle l'étape B) comporte une étape de mesure en temps réel de l'amplification des fragments d'ADN, et dans laquelle l'étape C) est effectuée de la façon suivante :

(i) si une ou quelques courbes, en nombre inférieur à la moitié des courbes, présentent un décalage par rapport aux autres courbes, tel que les autres courbes présentent un point d'inflexion au moins 2 cycles après le point d'inflexion de la première courbe, de préférence au moins 3 ou 4 cycles après le point d'inflexion de la première courbe, ou ne montrent aucune amplification, le résultat indique la présence d'une lymphoprolifération clonale ou oligoclonale ;
(ii) si, au contraire, toutes les courbes présentent un point d'inflexion au même cycle, ou dans un décalage maximum de 2 ou 3 cycles d'amplification, le résultat permet d'écarter l'hypothèse d'une lymphoprolifération d'un clone résultant d'un des réarrangements correspondant aux fragments amplifiés.

**3.** Méthode selon la revendication 2, comportant en outre, une étape de confirmation d'une lymphoprolifération par la mesure continue de la fluorescence dans chaque tube au cours d'une montée en température entre 40°C et 95°C, l'observation d'un pic majoritaire étant indicative de la présence d'un amplicon majoritaire et donc d'une lymphoprolifération, alors que l'observation de plusieurs pics de tailles similaires indique au contraire une diversité lymphocytaire.

**4.** Méthode selon l'une quelconque des revendications précédentes, dans laquelle la combinaison de 3 amorces constituant 2 couples d'amorces distincts comporte une amorce sens commune s'hybridant spécifiquement en amont et/ou dans un gène V donné, chaque couple d'amorces comportant en outre une amorce antisens s'hybridant spécifiquement en aval et/ou dans un gène J donné.

**5.** Méthode selon l'une quelconque des revendications 1 à 3, dans laquelle la combinaison de 3 amorces constituant 2 couples d'amorces distincts comporte une amorce antisens commune s'hybridant spécifiquement en aval et/ou dans un gène J donné, chaque couple d'amorces comportant en outre une amorce sens s'hybridant spécifiquement en amont et/ou dans un gène V donné.

**6.** Méthode selon l'une quelconque des revendications 1 à 5, dans laquelle la combinaison de 3 amorces constituant 2 couples d'amorces distincts présente les caractéristiques suivantes :

(i) les deux couples d'amorces comportent une amorce sens commune s'hybridant spécifiquement en amont et/ou dans un gène V donné et comportent chacun une amorce antisens s'hybridant spécifiquement en aval et/ou dans un gène J donné ;
(ii) les deux amorces antisens s'hybrident spécifiquement en aval et/ou dans deux gènes $J_y$ et $J_z$ appartenant à deux groupes distincts de gènes J du locus TRB ; et
(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

**7.** Méthode selon la revendication 6, dans laquelle la combinaison de 3 amorces comprend les amorces hTRBJ 1.6 de séquence SEQ ID No: 1, hTRBJ2.7 de séquence SEQ ID No : 2, et une amorce sens s'hybridant spécifiquement en amont et/ou dans un gène V donné.

**8.** Méthode selon la revendication 6 et la revendication 5, permettant l'analyse des réarrangements V(D)J du locus TRB humain en effectuant 24 PCR multi-n-plexes avec n≥2 à l'aide de combinaisons d'au moins 3 amorces, chaque combinaison d'amorces comprenant les amorces hTRBJ1.6, hTRBJ2.7 définies à la revendication 5 et au moins une amorce hTRBV choisie parmi les amorces définies par les séquences SEQ ID Nos : 3-25.

**9.** Méthode selon la revendication 1, pour la détection *in vitro* de réarrangements incomplets D-J de locus génétique TRB, dans laquelle au moins une réaction de PCR multi-n-plexe avec n≥2 est effectuée pour analyser des réarrangements incomplets DJ du locus TRB humain, en utilisant une combinaison d'au moins 3 amorces, dont 3 constituent 2 couples d'amorces distincts présentant les caractéristiques suivantes :

(i) les deux couples d'amorces comportent une amorce sens commune s'hybridant spécifiquement en amont et/ou dans un gène D donné et comportent chacun une amorce antisens s'hybridant spécifiquement en aval et/ou dans un gène J donné ;
(ii) les deux amorces antisens s'hybrident spécifiquement en aval et/ou dans deux gènes $J_y$ et $J_z$ appartenant à deux groupes distincts de gènes J du locus TRB ; et
(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

**10.** Méthode selon la revendication 9, dans laquelle au moins une réaction de PCR multi-n-plexe avec n≥2 est effectuée pour analyser des réarrangements incomplets du locus TRB, en utilisant une combinaison d'au moins 3 amorces comprenant une amorce sens s'hybridant spécifiquement en amont et/ou dans un gène D donné et les amorces hTRBJ1.6 et hTRBJ2.7 définies à la revendication 5.

**11.** Méthode selon la revendication 9 et la revendication 10, permettant l'analyse de tous les réarrangements incomplets du locus TRB humain en effectuant (i) une PCR multi-2-plexe à l'aide d'un triplet d'amorces constitué des amorces hTRBJl.6 de séquence SEQ ID No : 1, hTRBJ2.7 de séquence SEQ ID No : 2 et hTRBD1 choisie parmi les amorces définies par les séquences SEQ ID No : 26 et SEQ ID No : 27, et (ii) une PCR multiplexe simple à l'aide du couple d'amorces constitué des amorces hTRBJ2.7 et hTRBD2 choisie parmi les amorces définies par les séquences SEQ ID No : 28 et SEQ ID No : 29.

**12.** Méthode selon l'une quelconque des revendications 1 à 4, permettant l'analyse des réarrangements de 95% des gènes J du locus TRA humain avec un gène V donné du même locus, dans laquelle, à l'étape A), 6 PCR multi-2-plexes ou 3 PCR multi-4-plexes sont effectuées avec des combinaisons d'amorces constituées chacune d'une amorce s'hybridant en amont et/ou dans ledit gène V et d'un ou deux couple(s) d'amorces antisens choisi(s) parmi les couples :

- SEQ ID No : 30 et SEQ ID No : 31;

- SEQ ID No : 32 et SEQ ID No: 33;
- SEQ ID No : 34 et SEQ ID No : 35;
- SEQ ID No : 36 et SEQ ID No : 37;
- SEQ ID No : 38 et SEQ ID No : 39; et
- SEQ ID No : 40 et SEQ ID No : 41.

**13.** Méthode permettant une analyse de la diversité des réarrangements VJ du locus TRA, dans laquelle la méthode selon la revendication 12 est mise en oeuvre avec au moins une amorce s'hybridant en amont et/ou dans un gène V du locus TRA choisie parmi les amorces définies par les séquences SEQ ID Nos : 42-61.

**14.** Méthode selon l'une quelconque des revendications 1 à 3 et 5, permettant l'analyse des réarrangements de tous les gènes J du locus TRG humain avec au moins 2 gènes $V_x$ et $V_y$ donnés du même locus, dans laquelle, à l'étape A), au moins une PCR multi-2-plexe est effectuée avec un triplet d'amorces constitué de 2 amorces sens s'hybridant en amont et/ou dans lesdits gènes $V_x$ et $V_y$ et de l'amorce antisens hTRGJdo2 de séquence SEQ ID No : 62 s'hybridant dans le gène J2 du locus TRG humain.

**15.** Méthode selon la revendication 14, dans laquelle au moins une amorce s'hybridant en amont et/ou dans un gène V du locus TRG humain est choisie parmi les amorces définies par les séquences SEQ ID Nos : 63-66.

**16.** Méthode selon l'une quelconque des revendications 1 à 4, permettant l'analyse des réarrangements de tous les gènes J du locus TRD humain avec un gène V donné du même locus, dans laquelle, à l'étape A), une PCR multi-2-plexe est effectuée avec un triplet d'amorces constitué d'une amorce s'hybridant en amont et/ou dans ledit gène V et des amorces antisens hTRDJ1do5 de séquence SEQ ID No : 67 et hTRDJ3do2 de séquence SEQ ID No : 68.

**17.** Méthode selon la revendication 16, permettant l'analyse des réarrangements VJ du locus TRD en effectuant 24 PCR multi-n-plexes avec n≥2 à l'aide de combinaisons d'au moins 3 amorces, chaque combinaison d'amorces comprenant les amorces antisens hTRDJ1do5 et hTRDJ3do2 et au moins une amorce choisie parmi les amorces définies par les séquences SEQ ID Nos : 69-84.

**18.** Méthode selon l'une quelconque des revendications 1 à 4 et 5, permettant l'analyse des réarrangements de tous les gènes J du locus IgH humain avec au moins 2 gènes $V_x$ et $V_y$ donnés du même locus, dans laquelle, à l'étape A), au moins une PCR multi-2-plexe est effectuée avec un triplet d'amorces constitué de 2 amorces sens s'hybridant en amont et/ou dans lesdits gènes $V_x$ et $V_y$ et d'une amorce antisens s'hybridant en aval et/ou dans le gène IgHJ6.

**19.** Méthode selon la revendication 18, dans laquelle l'amorce antisens est l'amorce hIgHJ6do2 de séquence SEQ ID No : 85.

**20.** Méthode selon la revendication 18 ou la revendication 19, dans laquelle au moins une amorce s'hybridant en amont et/ou dans un gène V du locus IgH humain est choisie parmi les amorces définies par les séquences SEQ ID Nos : 86-91.

**21.** Méthode selon la revendication 1, pour la détection *in vitro* de réarrangements incomplets D-J de locus génétique IGH, dans laquelle au moins une réaction de PCR multi-n-plexe avec n≥2 est effectuée pour analyser certains réarrangements incomplets du locus IgH humain, en utilisant une combinaison d'au moins 3 amorces dont 3 constituent 2 couples d'amorces distincts comportant une amorce antisens commune s'hybridant spécifiquement en aval et/ou dans un gène J donné et comportant chacun une amorce sens s'hybridant spécifiquement en amont et/ou dans un gène D donné.

**22.** Méthode selon la revendication 21, dans laquelle l'amorce antisens commune est l'amorce hIgHJ6do2 définie à la revendication 19, et/ou au moins une amorce s'hybridant en amont et/ou dans un gène D du locus IgH humain est choisie parmi les amorces définies par les séquences SEQ ID Nos : 92-98.

**23.** Méthode selon l'une quelconque des revendications 4 à 22, dans laquelle l'étape B) de détection des produits d'amplification comprend une étape de séparation desdits produits suivant leur taille et l'étape C) comporte une étape de traitement des données obtenues par la séparation des amplicons suivant leur taille, ledit traitement étant effectué par un ordinateur et permettant d'attribuer à chaque amplicon observé le nom du réarrangement V(D)J correspondant.

**24.** Méthode selon la revendication 23, dans laquelle le traitement des données intègre également l'intensité du signal de chacun des amplicons observés, pour quantifier la fréquence relative du réarrangement V(D)J correspondant.

**25.** Méthode pour déterminer *in vitro* le niveau d'immunodéfcience d'un individu, comportant les étapes suivantes :

A) à partir d'un échantillon biologique dudit individu, faire une numération lymphocytaire ;
B) à partir du même échantillon ou d'un autre échantillon provenant du même individu au même moment, déterminer le niveau de diversité combinatoire du répertoire des lymphocytes dudit individu, par la mise en oeuvre d'une méthode selon l'une quelconque des revendications précédentes ;
C) combiner les données obtenues aux étapes A) et B), et interpréter la combinaison obtenue au regard d'un graphique attribuant un niveau de risque au moins aux 4 zones suivantes :

(i) numération faible (<1000 Ly/μL) et diversité combinatoire V-J faible (<40%) : risque infectieux élevé et risque de mortalité par infection élevé ;
(ii) numération faible (<1000 Ly/μL) mais diversité combinatoire V-J normale (>65%) : risque infectieux faible ;
(iii) numération normale (1000-3200 Ly/μL) et diversité combinatoire V-J faible (<40%) : risque infectieux moyen ;
(iv) numération normale (1000-3200 Ly/μL) et diversité combinatoire V-J normale (>65%) : le répertoire immunitaire est sain.

**26.** Méthode selon la revendication 25, dans laquelle l'étape B) comprend la détermination du niveau de diversité combinatoire du répertoire des lymphocytes T et des lymphocytes B dudit individu, et dans laquelle, à l'étape C), les données sont examinées à l'aide d'un graphique tridimensionnel présentant le niveau de diversité des immunoglobulines sur un axe, le niveau de diversité des TCR sur un autre axe, et la numération lymphocytaire sur un troisième axe.

**27.** Trousse pour la mise en oeuvre d'une méthode selon l'une quelconque des revendications précédentes, comprenant une combinaison de 3 amorces constituant 2 couples d'amorces distincts permettant chacun l'amplification d'au moins deux fragments d'ADN caractéristiques d'au moins deux réarrangements chromosomiques et des réactifs pour effectuer des PCR, **caractérisée en ce qu'**elle comprend au moins une combinaison d'amorces choisie parmi les combinaisons suivantes :

- les amorces hTRBJ1.6 de séquence SEQ ID No : 1, hTRBJ2.7 de séquence SEQ ID No : 2, et au moins une amorce hTRBV choisie parmi les amorces définies par les séquences SEQ ID Nos : 3-25 ;
- les amorces hTRBJ1.6 de séquence SEQ ID No: 1, hTRBJ2.7 de séquence SEQ ID No : 2, une amorce hTRBD1 choisie parmi les amorces définies par les séquences SEQ ID No : 26 et SEQ ID No : 27, et une amorce hTRBD2 choisie parmi les amorces définies par les séquences SEQ ID No : 28 et SEQ ID No : 29 ;
- les amorces définies par les séquences SEQ ID No : 30 à SEQ ID No : 41, et une amorce choisie parmi les amorces définies par les séquences SEQ ID Nos : 42-61 ;
- l'amorce hTRGJdo2 de séquence SEQ ID No : 62 et deux amorces choisies parmi les amorces définies par les séquences SEQ ID Nos : 63-66 ;
- les amorces hTRDJ1do5 de séquence SEQ ID No : 67 et hTRDJ3do2 de séquence SEQ ID No : 68, et une amorce choisie parmi les amorces définies par les séquences SEQ ID Nos : 69-84 ;
- l'amorce hIgHJ6do2 de séquence SEQ ID No : 85 et deux amorces choisies parmi les amorces définies par les séquences SEQ ID Nos : 86-91 ;
- l'amorce hIgHJ6do2 de séquence SEQ ID No : 85 et deux amorces choisies parmi les amorces définies par les séquences SEQ ID Nos : 92-98.

**28.** Trousse selon la revendication 27, comprenant une plaque multipuits dans laquelle chaque puits comprend une combinaison différente d'amorces, et dans laquelle une plaque multipuits comprend toutes les combinaisons d'amorces nécessaires à l'amplification d'au moins 50% des réarrangements V-J d'au moins un locus choisi parmi les loci TRA, TRB, TRG, TRD et IGH.

**Patentansprüche**

**1.** Verfahren zur In-vitro-Analyse der Kombinationsdiversität der V(D)J-Umordnungen wenigstens eines genetischen Locus, ausgewählt aus den Loci TRA, TRB, TRG, TRD, IGH, IGK und IGL, eines Individuums ausgehend von genomischer DNA, die aus einer biologischen Probe des Individuums stammt, umfassend die folgenden Schritte:

A) Amplifikation von Fragmenten der genomischen DNA durch multiplexe Polymerasekettenreaktionen (ACP oder QPCR), von denen wenigstens eine eine multi-n-plexe PCR mit $n \geq 2$ ist, die, in derselben Reaktion, n unterschiedliche Primer-Paare verwendet, die jeweils die Amplifikation von wenigstens zwei DNA-Fragmenten erlauben, welche für wenigstens zwei unterschiedliche chromosomale Umordnungen charakteristisch sind, die mit einer Kombination von wenigstens 3 Primern durchgeführt wird, die aus wenigstens 2 unterschiedlichen Primer-Paaren bestehen, die die folgenden Charakteristika aufweisen:

(i) drei der wenigstens 3 Primer bilden 2 unterschiedliche Primer-Paare;
(ii) jedes Primer-Paar besteht aus einem Primer, der spezifisch stromaufwärts eines und/oder in einem gegebenen Gen(s) V oder D hybridisiert, und aus einem Primer, der spezifisch stromabwärts eines und/oder in einem gegebenen Gen(s) J hybridisiert, und zwar derart, dass sie die Amplifikation von wenigstens zwei Fragmenten, die für zwei verschiedene Umordnungen V(D)J oder D-J charakteristisch sind, erlauben;
(iii) die Primer sind thermodynamisch kompatibel;
(iv) die Primer sind so gewählt, dass die Fragmente, die mit dem ersten Primer-Paar amplifiziert wurden, von den Fragmenten, die mit dem zweiten Primer-Paar amplifiziert wurden, unterschieden werden können;

B) Detektion der in Stufe A) erhaltenen Amplifikationsprodukte;
C) Interpretation der Resultate.

**2.** Verfahren gemäß Anspruch 1, in dem die Stufe B) eine Stufe der Echtzeitmessung der Amplifikation der DNA-Fragmente umfasst und in dem die Stufe C) wie folgt durchgeführt wird:

(i) wenn eine oder einige Kurven, in der Zahl weniger als die Hälfte der Kurven, eine Verschiebung bezüglich der anderen Kurve zeigt/zeigen, beispielsweise die anderen Kurven einen Wendepunkt wenigstens 2 Zyklen nach dem Wendepunkt der ersten Kurve, vorzugsweise wenigstens 3 oder 4 Zyklen nach dem Wendepunkt der ersten Kurve, zeigen oder keine Amplifikation zeigen, zeigt das Resultat das Vorliegen einer klonalen oder oligklonalen Lymphoproliferation an;
(ii) wenn dagegen alle Kurven einen Wendepunkt im selben Zyklus zeigen oder eine Verschiebung von maximal 2 oder 3 Amplifikationszyklen zeigen, erlaubt das Resultat die Hypothese einer Lymphoproliferation eines Klon, resultierend aus einer der Umordnungen, die den amplifizierten Fragmenten entsprechen, zu verwerfen.

**3.** Verfahren gemäß Anspruch 2, das außerdem eine Stufe der Bestätigung einer Lymphoproliferation durch die fortgesetzte Messung der Fluoreszenz in jedem Röhrchen im Verlauf eines Temperaturanstiegs zwischen 40°C und 90°C umfasst, wobei die Beobachtung eines Hauptpeaks ein Hinweis für das Vorliegen eines Hauptamplikons und somit einer Lymphoproliferation ist, während die Beobachtung vieler Peaks ähnlicher Größen dagegen eine lymphozytäre Diversität anzeigt.

**4.** Verfahren gemäß einem der vorangehenden Ansprüche, in dem die Kombination aus 3 Primern, die 2 unterschiedliche Primer-Paare bilden, einen gemeinsamen Antisinn-Primer, der spezifisch stromabwärts eines und/oder in einem gegebenen Gen(s) J hybridisiert, umfasst, wobei jedes Primer-Paar außerdem einen Sinn-Primer umfasst, der spezifisch stromaufwärts eines und/oder in einem gegebenen V-Gen(s) hybridisiert.

**5.** Verfahren gemäß einem der Ansprüche 1 bis 3, in dem die Kombination aus 3 Primern, die 2 unterschiedliche Primer-Paare bilden, einen gemeinsamen Antisinn-Primer umfasst, der spezifisch stromabwärts und/oder in einem gegebenen Gen(s) J hybridisiert, wobei jedes Primer-Paar außerdem einen Sinn-Primer umfasst, der spezifisch stromaufwärts eines gegebenen Gens V und/oder in einem gegebenen Gen V hybridisiert.

**6.** Verfahren gemäß einem der Ansprüche 1 bis 5, wobei die Kombination aus 3 Primern, die 2 unterschiedliche Primer-Paare bilden, die folgenden Charakteristika aufweist:

(i) die zwei Primer-Paare umfassen einen gemeinsamen Sinn-Primer, der spezifisch stromaufwärts eines und/oder in einem gegebenen Gen(s) V hybridisiert und umfassen jeweils einen Antisinn-Primer, der spezifisch stromabwärts eines und/oder mit einem gegebenen Gen(s) J hybridisiert;
(ii) die zwei Antisinn-Primer hybridisieren spezifisch stromabwärts von und/oder in zwei Genen $J_y$ und $J_z$, die zu zwei verschiedenen Gruppen der Gene J des Locus TRP gehören, und

(iii) der Abstand zwischen der Hybridisierungs.region des spezifischen Antisinn-Primers des Gens $J_Y$ und dem Anfang des Gens $J_Y$ ist größer als der Abstand zwischen der Hybridisierungsregion des spezifischen Antisinn-Primers des Gens $J_Z$ und dem Beginn des ersten Gens J der Gruppe der Gene des Gens $J_Z$.

**7.** Verfahren gemäß Anspruch 6, in dem die Kombination aus 3 Primern die Primer hTRBJ1.6 der Sequenz SEQ ID No:1, hTRBJ2.7 der Sequenz SEQ ID No:2 und einen Sinn-Primer, der spezifisch stromaufwärts des und/oder in einem gegebenen Gen(s) V hybridisiert.

**8.** Verfahren gemäß Anspruch 6 und Anspruch 7, das die Analyse der V(D)J-Umordnungen des humanen Locus TRB erlaubt, indem es 24 multi-n-plexe PCR mit n≥2 mit Hilfe von Kombinationen aus wenigstens 3 Primern durchführt, wobei jede Primerkombination die Primer hTRBJ1.6, hTRBJ2.7, definiert in Anspruch 5, und wenigstens einen Primer hTRBV, ausgewählt unter den durch die Sequenzen SEQ ID No:3-25 definierten Primer, umfasst.

**9.** Verfahren gemäß Anspruch 1 für die In-vitro-Detektion von unvollständigen Umordnungen D-J des genetischen Locus TRB, in dem wenigstens eine multi-n-plexe PCR-Reaktion mit n≥2 durchgeführt wird, um unvollständige Umordnungen DJ des humanen Locus TRB zu analysieren, wobei eine Kombination aus wenigstens 3 Primern verwendet wird, von denen 3 2 unterschiedliche Primer-Paare bilden, die die folgenden Charakteristika aufweisen:

(i) die zwei Primer-Paare umfassen einen gemeinsamen Sinn-Primer, der spezifisch stromaufwärts eines gegebenen Gens D und/oder in einem gegebenen Gen D hybridisiert, und umfassen jeweils einen Antisinn-Primer, der spezifisch stromabwärts eines gegebenen Gens J und/oder in einem gegebenen Gen J hybridisiert;
(ii) die zwei Antisinn-Primer hybridisieren spezifisch stromabwärts der und/oder in den zwei Genen $J_Y$ und $J_Z$, die zu zwei unterschiedlichen Gruppen von Genen J des Locus TRB gehören, und
(iii) der Abstand zwischen der Hybridisierungsregion des spezifischen Antisinn-Primers des Gens $J_Y$ und dem Beginn des Gens $J_Y$ ist größer als der Abstand zwischen der Hybridisierungsregion des spezifischen Antisinn-Primers des Gens $J_z$ und dem Beginn des ersten Gens J der Gruppe von Genen des Gens $J_Z$.

**10.** Verfahren gemäß Anspruch 9, wobei wenigstens eine multi-n-plexe PCR-Reaktion mit n≥2 durchgeführt wird, um unvollständige Umordnungen des Locus TRB zu analysieren, indem eine Kombination aus wenigstens 3 Primern verwendet wird, die einen Sinn-Primer, der spezifisch stromaufwärts eines gegebenen Gens D und/oder in einem gegebenen Gen D hybridisiert, und die Primer hTRBJ1.6 und hTRBJ2.7, die in Anspruch 5 definiert sind, umfasst.

**11.** Verfahren gemäß Anspruch 9 und Anspruch 10, das die Analyse aller unvollständigen Umordnungen des humanen Locus TRB, erlaubt, indem (i) eine multi-2-plexe PCR mit Hilfe eines Primer-Tripletts, bestehend aus den Primern hTRBJ1.6 der Sequenz SEQ ID No:1, hTRBJ2.7 der Sequenz SEQ ID No:2 und hTRBD1, ausgewählt aus den durch die Sequenzen SEQ ID No:26 und SEQ ID No:27 definierten Primern, und (ii) eine einfache multiplexe PCR mit Hilfe eines Primer-Paares, bestehend aus dem Primer hTRBJ2.7 und hTRBD2, ausgewählt aus den durch die Sequenzen SEQ ID No:28 und SEQ ID No:29 definierten Primern, durchgeführt wird.

**12.** Verfahren gemäß einem der Ansprüche 1 bis 4, das die Analyse der Umordnungen von 95% der Gene J des humanen Locus TRA mit einem Gen V desselben Locus erlaubt, wobei in Stufe A) 6 multi-2-plexe PCR oder 3 multi-4-plexe PCR mit Primerkombinationen durchgeführt werden, die jeweils aus einem Primer, der stromaufwärts des genannten Gens V und/oder in dem genannten Gen V hybridisiert, und einem oder zwei Antisinn-Primer-Paar(en), ausgewählt aus den Paaren

- SEQ ID No:30 und SEQ ID No:31
- SEQ ID No:32 und SEQ ID No:33
- SEQ ID No:34 und SEQ ID No:35
- SEQ ID No:36 und SEQ ID No:37
- SEQ ID No:38 und SEQ ID No:39 und
- SEQ ID No:40 und SEQ ID No:41,

bestehen.

**13.** Verfahren, das eine Analyse der Kombinationsdiversität der VJ-Umordnungen des Locus TRA erlaubt, wobei das Verfahren gemäß Anspruch 12 mit wenigstens einem Primer, der stromaufwärts eines Gens V und/oder in einem Gen V des Locus TRA hybridisiert, ausgewählt aus den durch die Sequenzen SEQ ID No:42 bis 61 definierten Primern, durchgeführt wird.

**14.** Verfahren gemäß einem der Ansprüche 1 bis 3 und 5, das die Analyse der Umordnungen aller Gene J des humanen Locus TRG mit wenigstens 2 gegebenen Genen $V_X$ und $V_Y$ desselben Locus erlaubt, wobei in Stufe A) wenigstens eine multi-2-plexe PCR mit einem Primer-Triplett, bestehend aus 2 Sinn-Primern, die stromaufwärts der genannten Gene $V_X$ und $V_Y$ und/oder in den genannten Genen $V_X$ und $V_Y$ hybridisieren, und dem Antisinn-Primer hTRGJdo2 der Sequenz SEQ ID No:62, der in dem Gen J2des humanen Locus TRG hybridisiert, durchgeführt wird.

**15.** Verfahren gemäß Anspruch 14, wobei wenigstens ein Primer, der stromaufwärts eines Gens V und/oder in einem Gen V des humanen Locus TRG hybridisiert, aus den durch die Sequenzen SEQ ID No:63 bis 66 definierten Sequenzen ausgewählt wird.

**16.** Verfahren gemäß einem der Ansprüche 1 bis 4, das die Analyse der Umordnungen aller Gene J des humanen Locus TRG mit einem gegebenen Gen V desselben Locus erlaubt, in dem in der Stufe A) eine multi-2-plexe PCR mit einem Primer-Triplett, bestehend aus einem Primer, der stromaufwärts des genannten Gens V und/oder in dem Gen V hybridisiert, und den Antisinn-Primern hTRDJ1do5 der Sequenz SEQ ID No:67 und hTRDJ3do2 der Sequenz SEQ ID No:68, durchgeführt wird.

**17.** Verfahren gemäß Anspruch 16, das die Analyse der VJ-Umordnungen des Locus TRD ermöglicht, in dem 24 multi-n-plexe PCR mit $n \geq 2$ mit Hilfe von Kombinationen aus wenigstens 3 Primern durchgeführt werden, wobei jede Primerkombination die Antisinn-Primer hTRDJ1do5 und hTRDJ3do2 und wenigstens einen Primer, ausgewählt aus den durch die Sequenzen SEQ ID Nos:69-84 definierten Primern, umfasst.

**18.** Verfahren gemäß einem der Ansprüche 1 bis 4 und 5, das die Analyse der Umordnungen aller Gene J des humanen Locus IgH mit wenigstens 2 gegebenen Genen $V_X$ und $V_Y$ desselben Locus ermöglicht, in dem in Stufe A) wenigstens eine multi-2-plexe PCR mit einem Primer-Triplett, bestehend aus 2 Sinn-Primern, die stromaufwärts der genannten Gene $V_X$ und $V_Y$ und/oder in den genannten Genen $V_X$ und $V_Y$ hybridisieren, und einem Antisinn-Primer, der stromabwärts des Gens IgHJ6 und/oder in dem Gen IgHJ6 hybridisiert, durchgeführt wird.

**19.** Verfahren gemäß Anspruch 18, wobei der Antisinn-Primer der Primer hIgEJ6do2 der Sequenz SEQ ID No:85 ist.

**20.** Verfahren gemäß Anspruch 18 oder Anspruch 19, wobei wenigstens ein Primer, der stromaufwärts eines Gens V und/oder in einem Gen V des humanen Locus IgH hybridisiert, unter den durch die Sequenzen SEQ ID Nos:86-91 definierten Primern ausgewählt wird.

**21.** Verfahren gemäß Anspruch 1 für die In-vitro-Detektion von unvollständigen D-J-Umordnungen des genetischen Locus IGH, in dem wenigstens eine multi-n-plexe PCR mit $n \geq 2$ durchgeführt wird, um bestimmte unvollständige Umordnungen des humanen Locus IgH zu analysieren, wobei eine Kombination aus wenigstens 3 Primern verwendet wird, von denen 3 2 unterschiedliche Primer-Paare bilden, die einen gemeinsamen Antisinn-Primer umfassen, der spezifisch stromabwärts eines gegebenen Gens J und/oder in einem gegebenen Gen J hybridisiert, und jeweils einen Sinn-Primer, der spezifisch stromaufwärts eines gegebenen Gens D und/oder in einem gegebenen Gen D hybridisiert, umfassen.

**22.** Verfahren gemäß Anspruch 21, in dem der gemeinsame Antisinn-Primer der Primer hIgHJ6do2, definiert in Anspruch 19, ist, und/oder wenigstens ein Primer, der stromaufwärts eines Gens D und/oder in einem Gen D des humanen Locus IgH hybridisiert, unter den durch die Sequenzen SEQ ID Nos:92-98 definierten Primern ausgewählt wird.

**23.** Verfahren gemäß einem der Ansprüche 4 bis 22, in dem die Stufe B) de Detektion der Amplifikationsprodukte eine Stufe der Trennung der Produkte nach ihrer Größe umfasst und die Stufe C) eine Stufe der Behandlung der durch die Trennung der Amplikons nach ihrer Größe erhaltenen Daten umfasst, wobei die Behandlung mittels Computer durchgeführt wird und erlaubt, jedem beobachteten Amplikon den Namen der entsprechenden Umordnung V(D)J zuzuordnen.

**24.** Verfahren gemäß Anspruch 23, in dem die Behandlung der Daten auch die Aufnahme der Intensität des Signals jeder der beobachteten Amplikons umfasst, um die relative Häufigkeit der entsprechenden V(D)J-Umordnung zu quantifizieren.

**25.** Verfahren zur In-vitro-Bestimmung des Immundefizienz-Levels eines Individuums, umfassend die folgenden Stufen:

A) Durchführen einer Lymphozyten-Zählung ausgehend von einer biologischen Probe des Individuums;

B) Bestimmen des Levels der Kombinationsdiversität des Repertoires der Lymphozyten des Individuums ausgehend von derselben Probe oder einer anderen Probe, die von demselben Individuum zur gleichen Zeit kommt, durch Durchführen eines Verfahrens gemäß einem der vorangehenden Ansprüche;
C) Kombinieren der in den Stufen A) und B) erhaltenen Daten und Interpretieren der erhaltenen Kombination bezüglich einer Grafik, die ein Risikolevel wenigstens den vier folgenden Zonen zuordnet:

(i) schwache Anzahl (<1000 Ly/μl) und schwache V-J-Kombinationsdiversität (<40%): erhöhtes Infektionsrisiko und Mortalitätsrisiko durch erhöhte Infektion;
(ii) schwache Anzahl (<1000 Ly/μl), aber normale V-J-Kombinationsdiversität (>65%): schwaches Infektionsrisiko;
(iii) normale Anzahl (1000-3200 Ly/μl) und schwache V-J-Kombinationsdiversität (<40%): mittleres Infektionsrisiko;
(iv) normale Anzahl (1000-3200 Ly/μl) und normale V-J-Kombinationsdiversität (>65%): das Immunrepertoire ist gesund.

**26.** Verfahren gemäß Anspruch 25, in dem die Stufe B) die Bestimmung des Kombinationsdiversitäts-Levels des Repertoires der T-Lymphozyten und der B-Lymphozyten des Individuums umfasst und wobei in der Stufe C) die Daten mit Hilfe einer dreidimensionalen Grafik untersucht werden, welche den Diversitäts-Level der Immunoglobuline auf einer Achse, den Diversitäts-Level der TCR auf einer anderen Achse und die Lymphozytenzahl auf einer dritten Achse darstellt.

**27.** Kit zur Durchführung eines Verfahrens gemäß einem der vorangehenden Ansprüche, umfassend eine Kombination aus 3 Primern, die 2 unterschiedliche Primer-Paare bilden, die jeweils die Amplifikation von mindestens zwei DNA-Fragmenten, die für wenigstens zwei chromosomale Umordnungen charakteristisch sind, ermöglichen, und Reagenzien zur Durchführung der PCR, **dadurch gekennzeichnet, dass** er wenigstens eine PrimerKombination, ausgewählt aus den folgenden Kombinationen, umfasst:

- die Primer hTRBJ1.6 der Sequenz SEQ ID No:1, hTRBV2.7 der Sequenz SEQ ID No:2 und wenigstens ein Primer hTRBV, ausgewählt aus den durch die Sequenzen SEQ ID No:3-25 definierten Primern;
- die Primer hTRBJ1.6 der Sequenz SEQ ID No:1, hTRBJ2.7 der Sequenz SEQ ID No:2, einen Primer hTRBD1, ausgewählt aus den durch die Sequenzen SEQ ID No:26 und SEQ 10 No:27 definierten Primern, und ein Primer hTRBD2, ausgewählt aus den durch die Sequenzen SEQ ID No. 28 und SEQ ID No:29 definierten Primern;
- die durch die Sequenzen SEQ ID No:30 bis SEQ ID No:41 definierten Primer und ein Primer, ausgewählt aus den durch die Sequenzen SEQ ID No:42-61 definierten Sequenzen;
- der Primer hTRGJdo22 der Sequenz SEQ ID No:62 und zwei Primer, ausgewählt aus den durch die Sequenzen SEQ ID No:63-66 definierten Primern;
- die Primer hTPDJ1do5 der Sequenz SEQ ID No:67 und hTRDJ3do2 der Sequenz SEQ ID No:68 und ein Primer, ausgewählt unter den durch die Sequenzen SEQ ID Nom:69-84 definierten Primern;
- der Primer hIgHJ6do2 der Sequenz SEQ ID No:85 und zwei Primer, ausgewählt aus den durch die Sequenzen SEQ ID Nos:86-91 definierten Primern;
- der Primer hIgHJ6do2 der Sequenz SEQ ID No:85 und zwei Primer, ausgewählt aus den durch die Sequenzen SEQ ID Nos:92-98 definierten Primern.

**28.** Kit gemäß Anspruch 27, umfassend eine Multiwell-Platte, in der jedes Well eine unterschiedliche Primerkombination umfasst und wobei jede Multiwell-Platte alle Primerkombinationen umfasst, die zur Amplifikation von wenigstens 50% der V-J-Umordnungen wenigstens eines Locus, ausgewählt aus den Loci TRA, TRB, TRG, TRD und IGH, notwendig sind.

## Claims

**1.** Method for *in vitro* analysis of the combinatorial diversity of the V(D)J rearrangements of at least one genetic locus chosen from the TRA, TRB, TRG, TRD, IGH, IGK and IGL loci of a person from genomic DNA obtained from a biological sample taken from said person, comprising the following steps:

A) amplifying fragments of said genomic DNA by multiplex polymerase chain reactions (PCR), at least one of which is a multi-n-plex PCR with n≥2, employing, in the same reaction, n different primer pairs, each enabling the amplification of at least two DNA fragments characteristic of at least two different chromosome rearrangements, effected with a combination of at least three primers, constituting at least two distinct primer pairs having the following characteristics:

(i) three of said at least three primers constitute two distinct primer pairs;
(ii) each primer pair consists of a primer specifically hybridising upstream of and/or in a given V or D gene and a primer specifically hybridising downstream of and/or in a given J gene, so as to enable the amplification of at least two fragments characteristic of two distinct V(D)J or D-J rearrangements;
(iii) the primers are thermodynamically compatible;
(iv) the primers are chosen so that the fragments amplified with the first primer pair can be distinguished from the fragments amplified with the second primer pair;

B) detecting the products of amplification obtained in the step A;
C) interpreting the results.

**2.** Method according to claim 1, wherein the step B) includes a step of measuring the amplification of the DNA fragments in real time and wherein the step C) is effected as follows:

(i) if one or a few curves, the number of which is less than half the curves, feature(s) a shift relative to the other curves, such that the other curves have a point of inflection at least two cycles after the point of inflection of the first curve, preferably at least three or four cycles after the point of inflection of the first curve, or do not show any amplification, the result indicates the presence of clonal or oligoclonal lymphoproliferation;
(ii) if, on the other hand, all the curves feature a point of inflection in the same cycle, or within a maximum shift of two or three amplification cycles, the result enables the hypothesis of lymphoproliferation of a clone resulting from one of the rearrangements corresponding to the amplified fragments to be discarded.

**3.** Method according to claim 2, further comprising a step of confirming lymphoproliferation by continuously measuring the fluorescence in each tube during an increase in temperature between 40°C and 95°C, the observation of a predominant peak being indicative of the presence of a majority amplicon and therefore of lymphoproliferation, whereas the observation of a plurality of peaks of similar size on the contrary indicates lymphocyte diversity.

**4.** Method according to any one of the preceding claims, wherein the combination of three primers constituting two distinct primer pairs includes a common sense primer specifically hybridising upstream of and/or in a given V gene, each primer pair further including an antisense primer specifically hybridising downstream of and/or in a given J gene.

**5.** Method according to any one of claims 1 to 3,
wherein the combination of three primers constituting two distinct primer pairs includes a common antisense primer specifically hybridising downstream of and/or in a given J gene, each primer pair further including a sense primer specifically hybridising upstream of and/or in a given V gene.

**6.** Method according to any one of claims 1 to 5, wherein the combination of three primers constituting two distinct primer pairs has the following characteristics:

(i) the two primer pairs include a common sense primer specifically hybridising upstream of and/or in a given V gene and each includes an antisense primer specifically hybridising downstream of and/or in a given J gene;
(ii) the two antisense primers specifically hybridise downstream of and/or in two genes $J_y$ and $J_z$ belonging to two distinct groups of J genes of the TRB locus; and
(iii) the distance between the hybridisation region of the antisense primer specific to the gene $J_y$ and the beginning of said gene $J_y$ is greater than the distance between the hybridisation region of the antisense primer specific to the gene $J_z$ and the beginning of the first J gene of the gene group of said gene $J_z$.

**7.** Method according to claim 6, wherein the combination of three primers comprises the primer hTRBJ1.6 of sequence SEQ ID No. 1, the primer hTRBJ2.7 of sequence SEQ ID No. 2, and a sense primer specifically hybridising upstream of and/or in a given V gene.

**8.** Method according to claim 6 and claim 5 for analysing the V(D)J rearrangements of the human TRB locus by effecting 24 multi-n-plex PCR with n≥2 using combinations of at least three primers, each combination of primers comprising the primers hTRBJ1.6, hTRBJ2.7 defined in claim 5 and at least one primer hTRBV chosen from the primers defined by the sequences SEQ ID Nos. 3-25.

**9.** Method according to claim 1 for *in vitro* detection of incomplete D-J rearrangements of the TRB genetic locus, wherein at least one multi-n-plex PCR with n≥2 is effected to analyse incomplete DJ rearrangements of the human TRB locus using a combination of at least three primers, three of which constitute two distinct primer pairs having the following characteristics:

(i) the two primer pairs include a common sense primer specifically hybridising upstream of and/or in a given D gene and each includes an antisense primer specifically hybridising downstream of and/or in a given J gene;
(ii) the two antisense primers hybridise specifically downstream of and/or in two genes $J_y$ and $J_z$ belonging to two distinct groups of J genes of the TRB locus; and
(iii) the distance between the hybridisation region of the specific antisense primer of the gene $J_y$ and the beginning of said gene $J_y$ is greater than the distance between the hybridisation region of the specific antisense primer of the gene $J_z$ and the beginning of the first J gene of the gene group of said gene $J_z$.

**10.** Method according to claim 9, wherein at least one multi-n-plex PCR with n≥2 is effected to analyse incomplete rearrangements of the TRB locus using a combination of at least three primers comprising a sense primer specifically hybridising upstream of and/or in a given D gene and the primers hTRBJ1.6 and hTRBJ2.7 defined in claim 5.

**11.** Method according to claim 9 and claim 10 for analysing all incomplete rearrangements of the human TRB locus by effecting (i) a multi-2-plex PCR using a triplet of primers consisting of the primer hTRBV1.6 of sequence SEQ TD No. 1, the primer hTRBJ2.7 of sequence SEQ ID No. 2, and the primer hTRBD1 chosen from the primers defined by the sequences SEQ ID No. 26 and SEQ ID No. 27, and (ii) a single multiplex PCR using the primer pair consisting of the primers hTRBJ2.7 and hTRBD2 chosen from the primers defined by the sequences SEQ ID No. 28 and SEQ ID No. 29.

**12.** Method according to any one of claims 1 to 4 for analysing 95% of rearrangements of the J genes of the human TRA locus with a given V gene of the same locus, in which, in the step A), six multi-2-plex PCR or three multi-4-plex PCR are effected with combinations of primers each consisting of a primer hybridising upstream of and/or in said V gene and one or two antisense primer pair(s) chosen from the pairs:

- SEQ ID No. 30 and SEQ ID No. 31;
- SEQ ID No. 32 and SEQ ID No. 33;
- SEQ ID No. 34 and SEQ ID No. 35;
- SEQ ID No. 36 and SEQ ID No. 37;
- SEQ ID No. 38 and SEQ ID No. 39; and
- SEQ ID No. 40 and SEQ ID No. 41.

**13.** Method for analysing the diversity of the VJ rearrangements of the TRA locus, wherein the method according to claim 12 is used with at least one primer hybridising upstream of and/or in a V gene of the TRA locus chosen from the primers defined by the sequences SEQ ID Nos. 42-61.

**14.** Method according to any one of claims 1 to 3 and 5 for analysing rearrangements of all the J genes of the human TRG locus with at least two given genes $V_x$ and $V_y$ of the same locus, wherein, in the step A), at least one multi-2-plex PCR is effected with a triplet of primers consisting of two sense primers hybridising upstream of and/or in said genes $V_x$ and $V_z$ and the antisense primer hTRGJdo2 of sequence SEQ ID No. 62 hybridising in the gene $J_2$ of the human TRG locus.

**15.** Method according to claim 14, wherein at least one primer hybridising upstream of and/or in a V gene of the human TRG locus is chosen from the primers defined by the sequences SEQ ID Nos. 63-66.

**16.** Method according to any one of claims 1 to 4 for analysing the rearrangements of all the J genes of the human TRD locus with a given V gene of the same locus, wherein, in the step A, a multi-2-plex PCR is effected with a triplet of primers consisting of a primer hybridising upstream of and/or in said V gene and antisense primers hTRDJ1do5 of sequence SEX ID No. 67 and hTRDJ3do2 of sequence SEQ ID No. 68.

**17.** Method according to claim 16 for analysing the VJ rearrangements of the TRD locus by effecting 24 multi-n-plex PCR with n≥2 using combinations of at least three primers, each primer combination comprising the antisense primers hTRDJ1do5 and hTRDJ3do2 and at least one primer chosen from the primers defined by the sequences SEQ ID No. 69-84.

**18.** Method according to any one of claims 1 to 4 and 5 for analysing the rearrangements of all the J genes of the human IgH locus with at least two given genes $V_x$ and $V_y$ of the same locus, wherein, in the step A), at least one multi-2-plex PCR is effected with a triplet of primers consisting of two sense primers hybridising upstream of and/or in said genes $V_x$ and $V_z$ and an antisense primer hybridising downstream of and/or in the IgHJ6 gene.

**19.** Method according to claim 18, wherein the antisense primer is the primer hIgHJ6do2 of the sequence SEQ ID No. 85.

**20.** Method according to claim 18 or claim 19, wherein at least one primer hybridising upstream of and/or in a V gene of the human IgH locus is chosen from the primers defined by the sequences SEQ ID Nos. 86-91.

**21.** Method according to claim 1 for *in vitro* detection of incomplete D-J rearrangements of the IgH genetic locus, wherein at least one multi-n-plex PCR with $n \geq 2$ is effected to analyse some incomplete rearrangements of the human IgH locus using a combination of at least three primers, three of which constitute two distinct primer pairs including a common antisense primer specifically hybridising downstream of and/or in a given J gene and each including a sense primer specifically hybridising upstream of and/or in a given D gene.

**22.** Method according to claim 21, wherein the common antisense primer is the primer hIgHJ6do2 defined in claim 19 and/or at least one primer hybridising upstream of and/or in a D gene of the human IgH locus is chosen from the primers defined by the sequences SEQ ID Nos. 92-98.

**23.** Method according to any one of claims 4 to 22, wherein the step B) of detecting products of amplification includes a step of separating said products according to their size and the step C) includes a step of processing the data obtained by separating the amplicons according to their size, said processing being effected by a computer and enabling each amplicon observed to be assigned the name of the corresponding V(D)J rearrangement.

**24.** Method according to claim 23, wherein the processing of the data also integrates the intensity of the signal of each of the observed amplicons to quantify the relative frequency of the corresponding V(D)J rearrangement.

**25.** Method for *in vitro* determination of the level of immunodeficiency of a person, including the following steps:

A) from a biological sample taken from said person, effecting a lymphocyte count;
B) from the same sample or from another sample taken from the same person at the same time, determining the level of combinatorial diversity of the lymphocyte repertoire of said person using a method according to any one of the preceding claims;
C) combining the data obtained in the steps A) and B) and interpreting the combination obtained with reference to a graph assigning a level of risk at least to the following four zones:

(i) low count (<1000 Ly/μL) and low V-J combinatorial diversity (<40%): high infectious risk and high risk of mortality by infection;
(ii) low count (<1000 Ly/μL) but normal V-J combinatorial diversity (>65%): low infectious risk;
(iii) normal count (1000-3200 Ly/μL) and low V-J combinatorial diversity (<40%): moderate infectious risk;
(iv) normal count (1000-3200 Ly/μL) and normal V-J combinatorial diversity (>65%): the immune repertoire is healthy.

**26.** Method according to claim 25, wherein the step B) includes determining the level of combinatorial diversity of the repertoire of T lymphocytes and B lymphocytes of said person, and wherein, in the step C), the data is examined using a three-dimensional graph showing the level of diversity of the immunoglobulins on one axis, the TCR diversity level on another axis and the lymphocyte count on a third axis.

**27.** Kit for implementing the method according to any one of the preceding claims, comprising a combination of three primers constituting two distinct primer pairs each enabling the amplification of at least two DNA fragments characteristic of at least two chromosome rearrangements and reagents for effecting PCR, **characterised in that** it comprises at least one combination of primers chosen from the following combination:

- the primer hTRBJ1.6 of sequence SEQ ID No. 1, the primer hTRBJ2.7 of sequence SEQ ID No. 2, and at least one primer hTRBV chosen from the primers defined by the sequences SEQ ID Nos. 3-25.
- the primer hTRBJ1.6 of SEQ ID No. 1, the primer hTRBV2.7 of SEQ ID No. 2, a primer HTRBD2 chosen from the primers defined by the sequences SEQ ID No. 26 and SEQ ID No. 27, and a primer hTRBD2 chosen from the primers defined by the sequences SEQ ID No. 28 and SEQ ID No. 29;
- the primers defined by the sequences SEQ ID No. 30 to SEQ ID No. 41, and a primer chosen from the primers defined by the sequences SEQ ID Nos. 42-61;
- the primer hTRGJdo2 of sequence SEQ ID No. 62 and two primers chosen from the primers defined by the sequences SEQ ID Nos. 63-66;
- the primers hTRDJ1do5 of sequence SEQ ID No. 67 and hTRDJ3do2 of sequence SEQ ID No. 68, and a primer chosen from the primers defined by the sequences SEQ ID Nos. 69-84;
- the primer hIgHJ6do2 of sequence SEQ ID No. 85 and two primers chosen from the primers defined by the sequences SEQ ID Nos. 86-91;
- the primer hIgHJ6do2 of sequence SEQ ID No. 85 and two primers chosen from the primers defined by the sequences SEQ ID Nos. 92-98.

**28.** Kit according to claim 27, comprising a multiwell plate in which each well contains a different combination of primers and wherein a multiwell plate comprises all the combinations of primers necessary for the amplification of at least 50% of the V-J rearrangements of at least one locus chosen from the TRA, TRB, TRG, TRD and IGH loci.

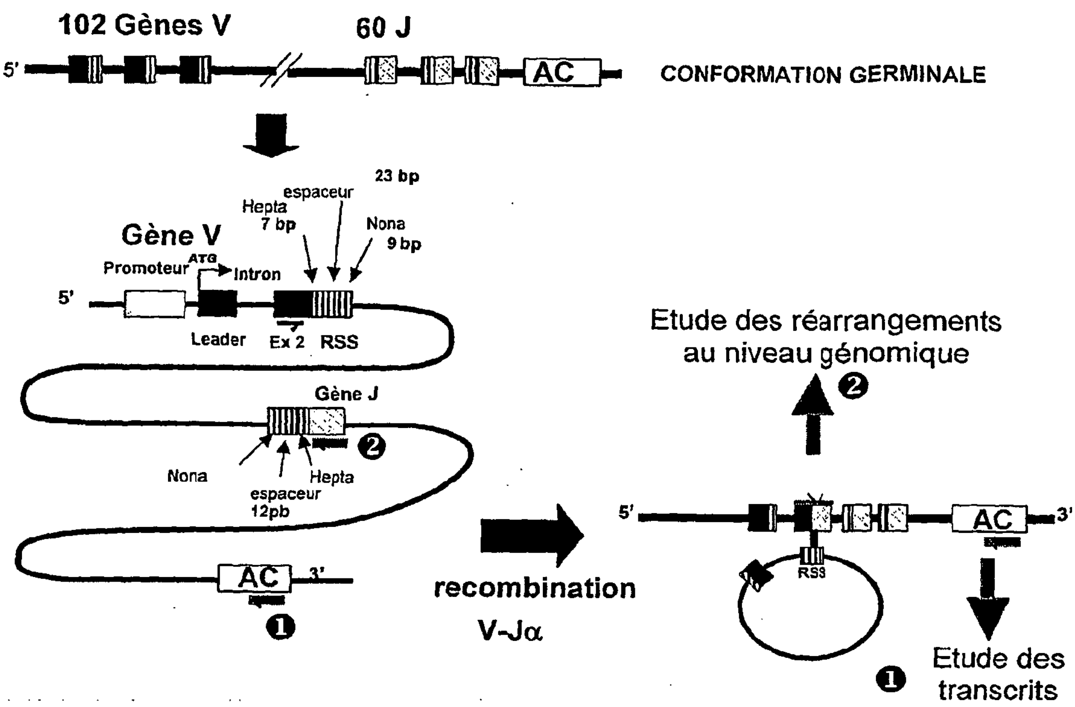


Figure 1a

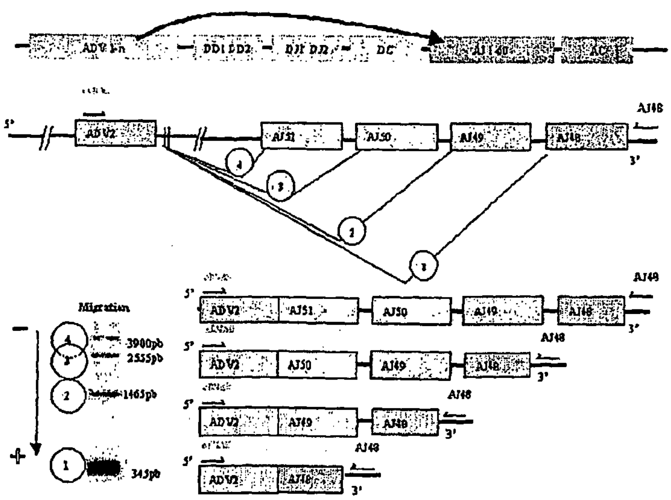

Figure 1b

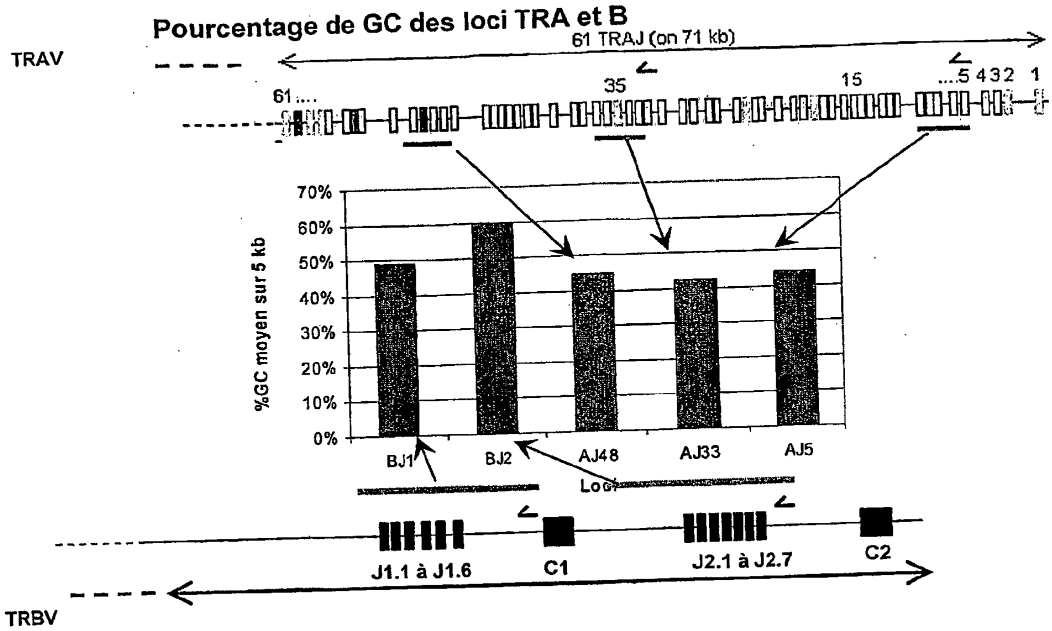


Figure 2a

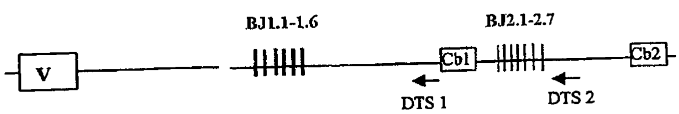


Figure 2b

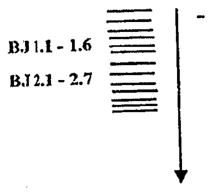


Figure 2c

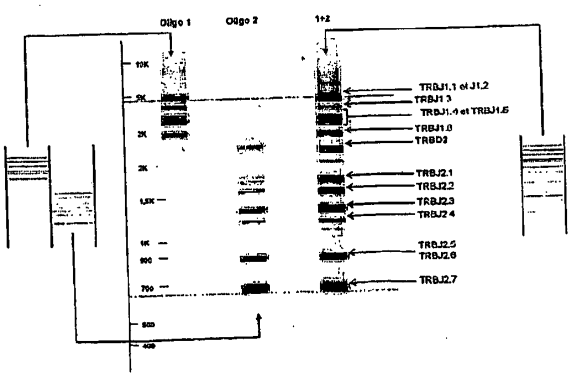

Figure 2d

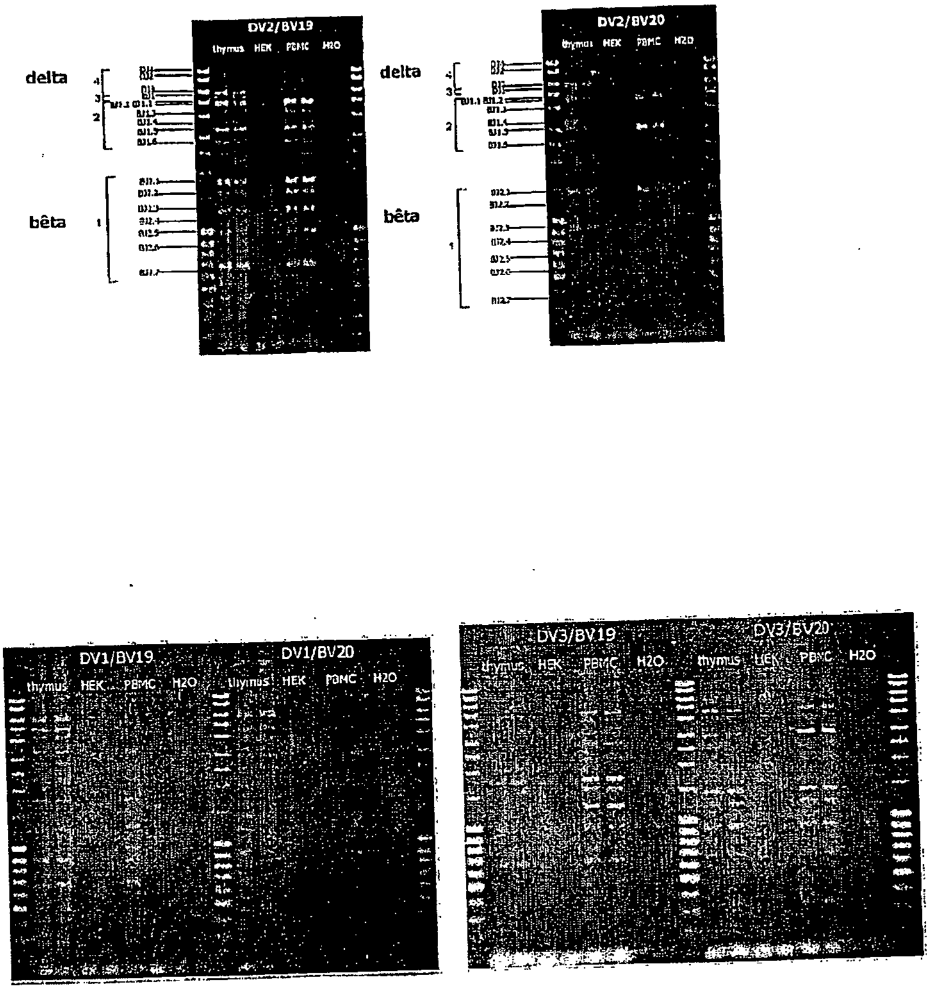
DV2/BV19
thymus HEK PBMC H2O
delta
bêta
DV2/BV20
thymus HEK PBMC H2O
delta
bêta
DV1/BV19
thymus HEK PBMC H2O
DV1/BV20
thymus HEK PBMC H2O
DV3/BV19
thymus HEK PBMC H2O
DV3/BV20
thymus HEK PBMC H2O

Figure 3

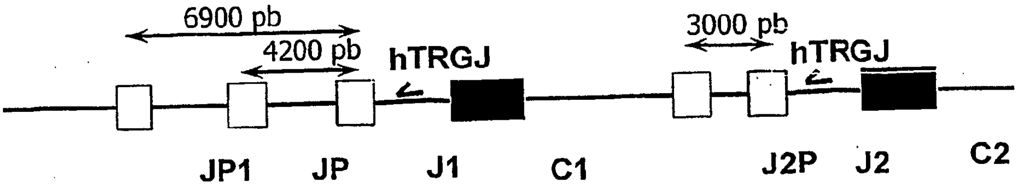


Figure 4a

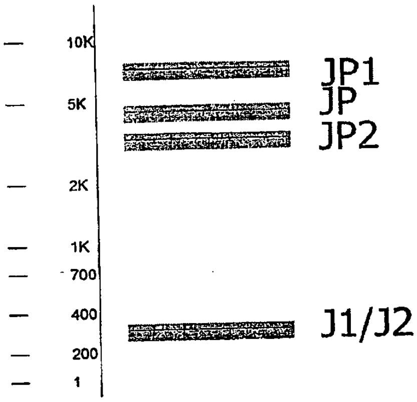


Figure 4b

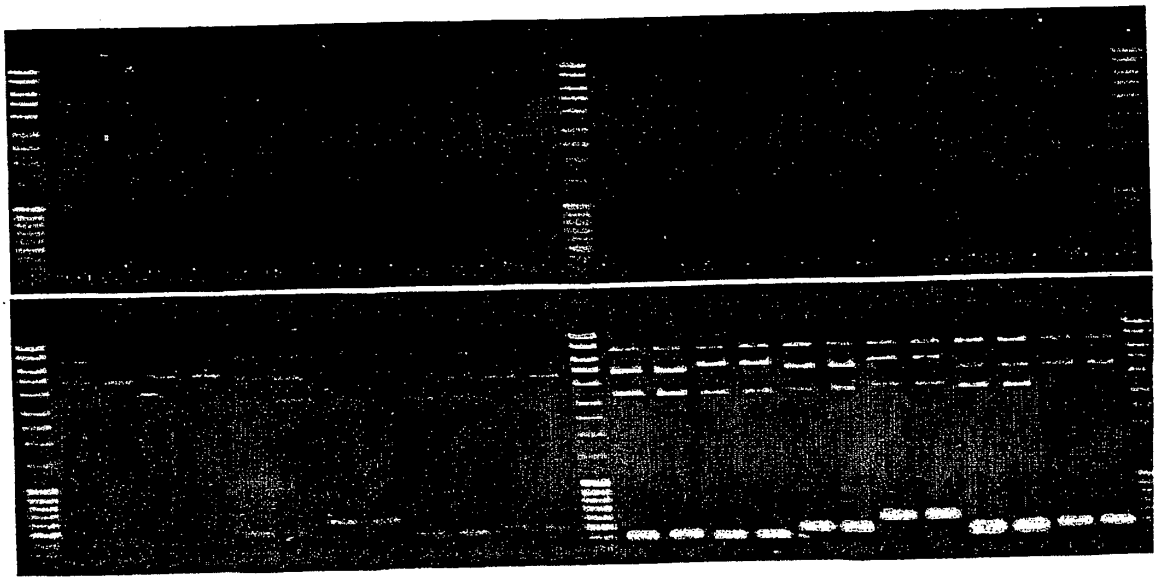

Figure 4c

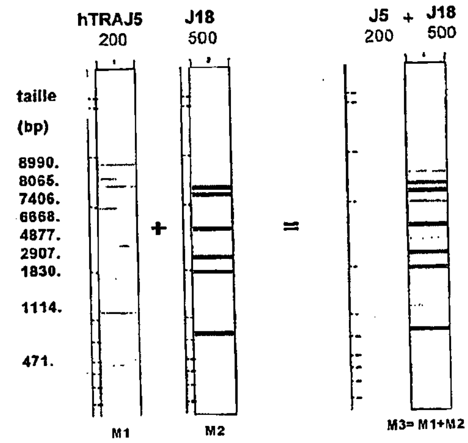


Figure 4d

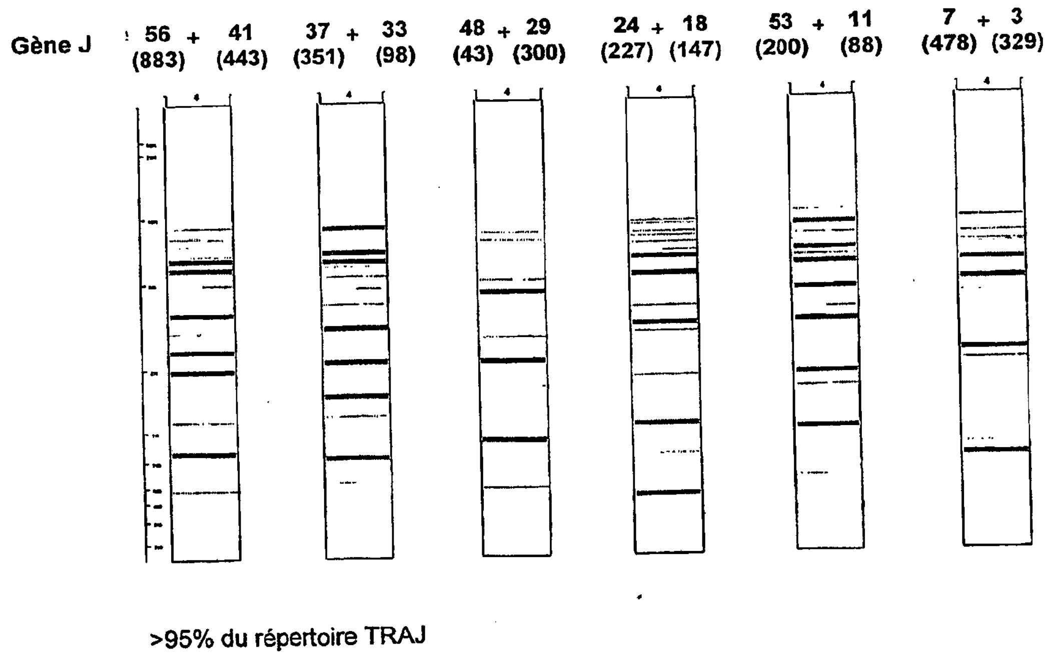


Figure 4e

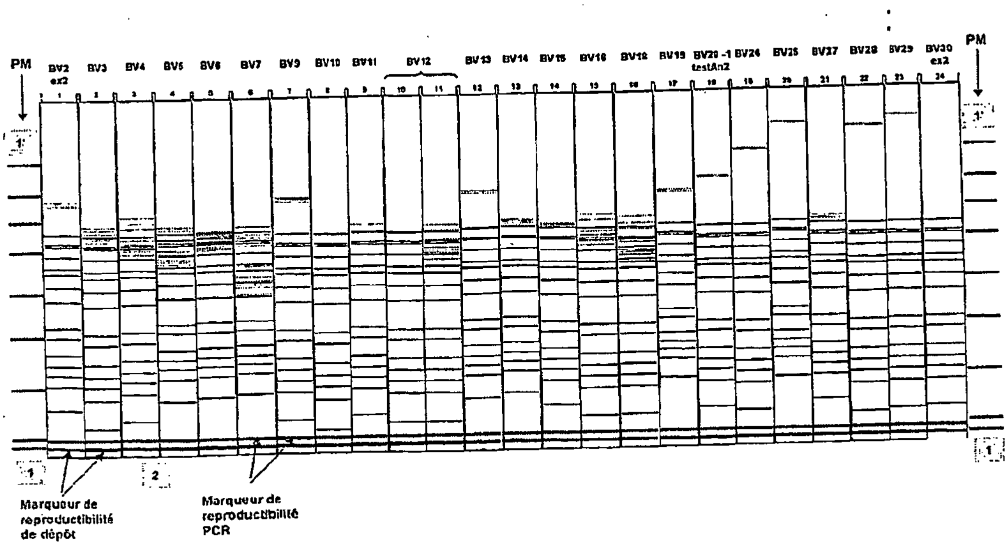
PM
PM
Marqueur de reproductibilité de dépôt
Marqueur de reproductibilité PCR

Figure 5

Thymus (90% répertoire total) (duplicat)

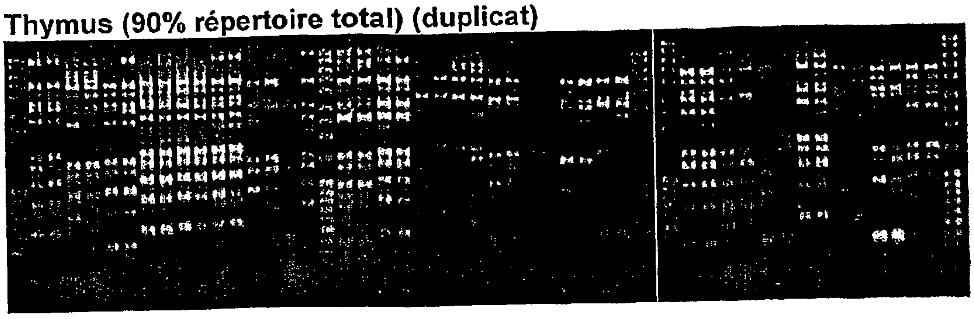

Lymphopenia (duplicat)

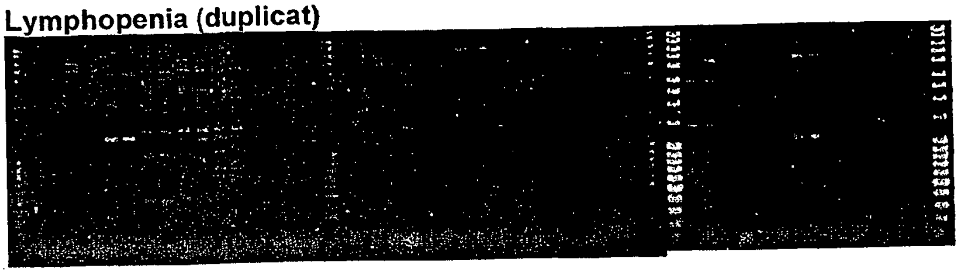

Pool de 4 lignées JURKAT, MOLT4, HUT et SUP : caractérisation des réarrangements TRBV-J (simplicat)

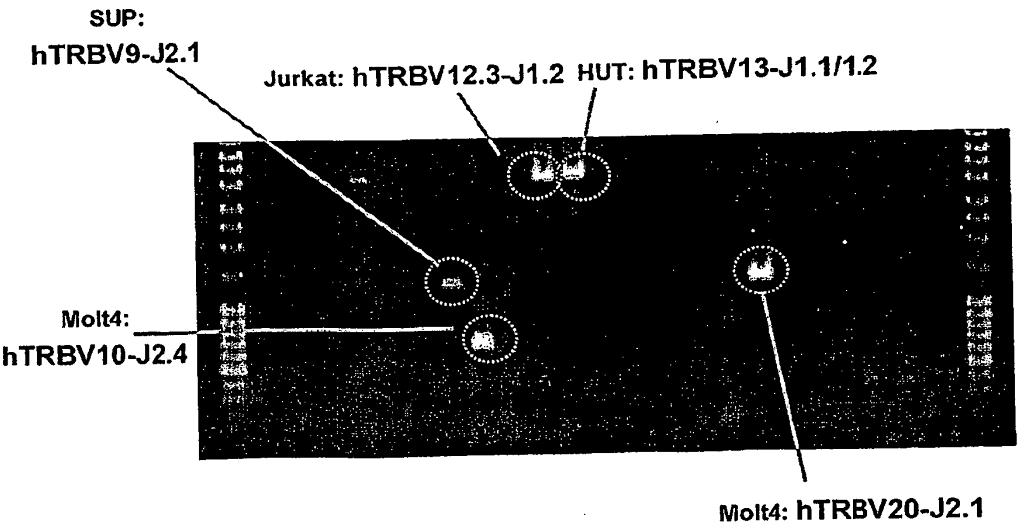


Figure 6

A

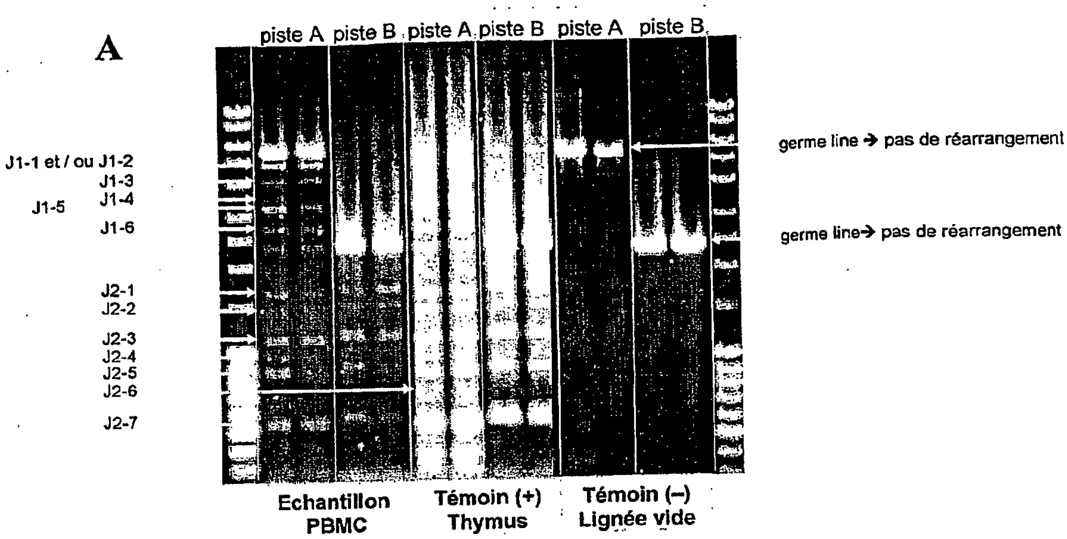
piste A
piste B
piste A
piste B
piste A
piste B
J1-1 et / ou J1-2
J1-3
J1-5
J1-4
J1-6
J2-1
J2-2
J2-3
J2-4
J2-5
J2-6
J2-7
germe line → pas de réarrangement
germe line→ pas de réarrangement
Echantillon PBMC
Témoin (+) Thymus
Témoin (–) Lignée vide

B

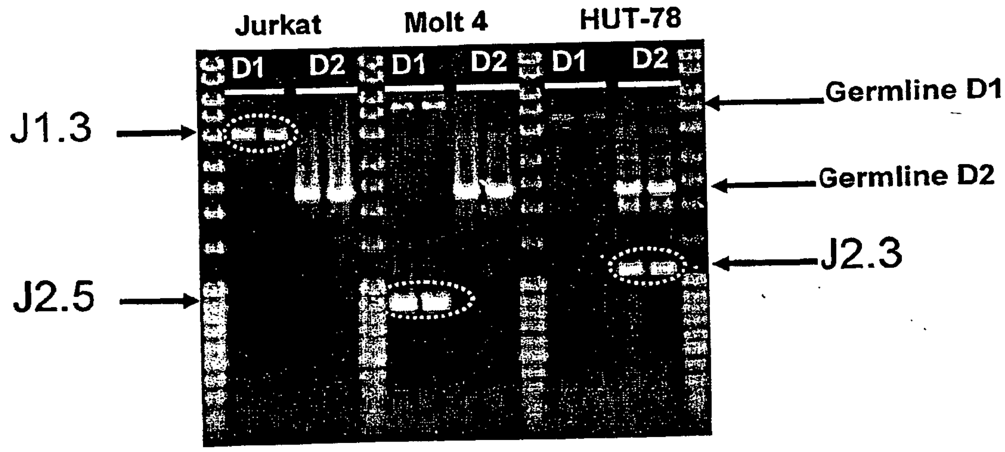
Jurkat
Molt 4
HUT-78
D1
D2
D1
D2
D1
D2
J1.3
J2.5
Germline D1
Germline D2
J2.3

Figure 7

A-

| Intensité | Valeur | % / Ref |
|---|---|---|
| Intensité Totale (U.A) | 15453,46 | 10,90% |
| Intensité hTRBD1,2 | 2494,39 | |
| **Diversité** | | |
| Nbre de réarrangements théoriques | 276 | |
| Nbre de réarrangements observés | 120 | |
| Nbre réarrangements avec hTRBD1,2 | 19 | |
| Diversité / Théo | 43,48% | |
| Diversité / Ref | 47,06% | |
| **Lymphopénie** | Non | Diversité Théorique < 40 % |
| **Prolifération(s) Clonale(s)** | (en rouge dans information réarrangements) | |
| Nombre | 0 | |
| **Délétion(s) potentielle(s)** | (en rouge dans informations gènes) | |
| Nombre | 2 | |
| **Homogénéité** | | |
| CV Inhomogénéité | 1,88 | |
| Int moyenne / Réarr observé | 128,78 | |

B-

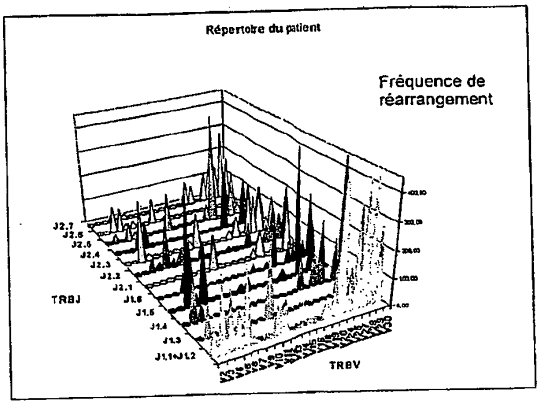


C-

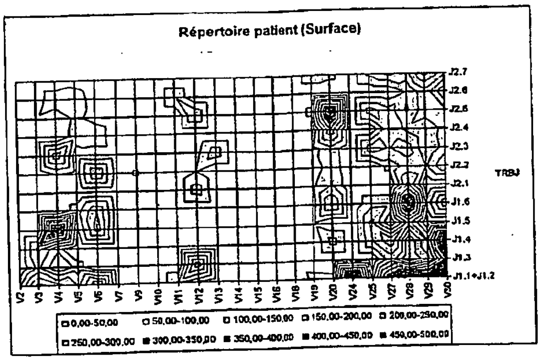


D-

| Ordre | Nom | Valeur | Contrib / Int.totale (en %) |
|---|---|---|---|
| 1 | J1.1+J1.2-V24 | 651,54 | 4,22% |
| 2 | J1.4-V30 | 651,54 | 4,22% |
| 3 | J1.1+J1.2-V29 | 565,31 | 3,66% |
| 4 | J1.1+J1.2-V28 | 464,02 | 3,00% |
| 5 | J1.6-V28 | 439,38 | 2,84% |
| 6 | J2.5-V20 | 427,06 | 2,76% |
| 7 | J1.5-V4 | 424,04 | 2,74% |
| 8 | J1.1+J1.2-V27 | 353,14 | 2,29% |
| 9 | J1.3-V30 | 320,29 | 2,07% |
| 10 | J1.1+J1.2-V6 | 302,88 | 1,96% |
| 11 | J1.1+J1.2-V30 | 288,81 | 1,87% |

Figure 8

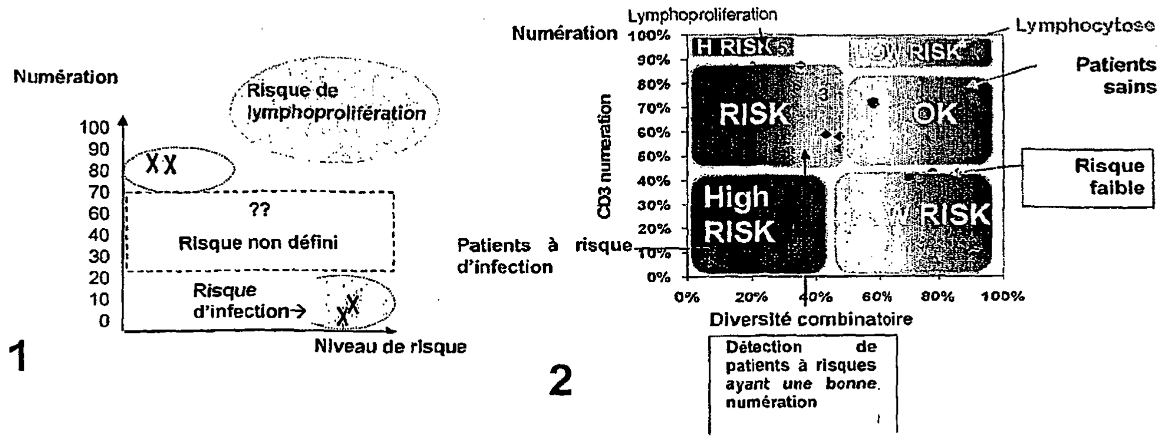


Figure 9

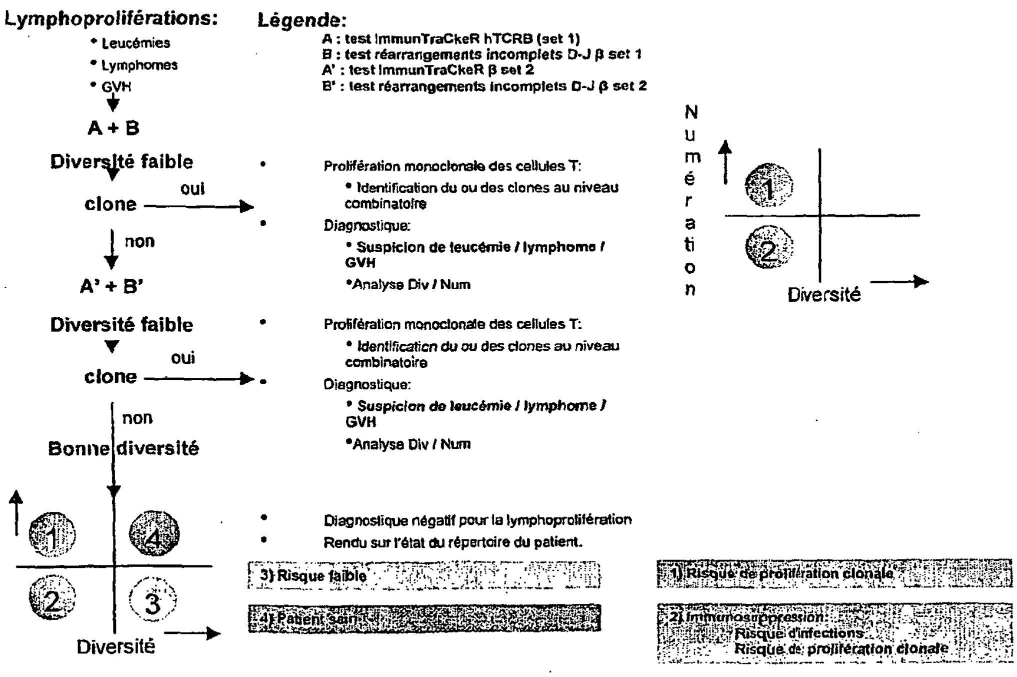


Figure 10

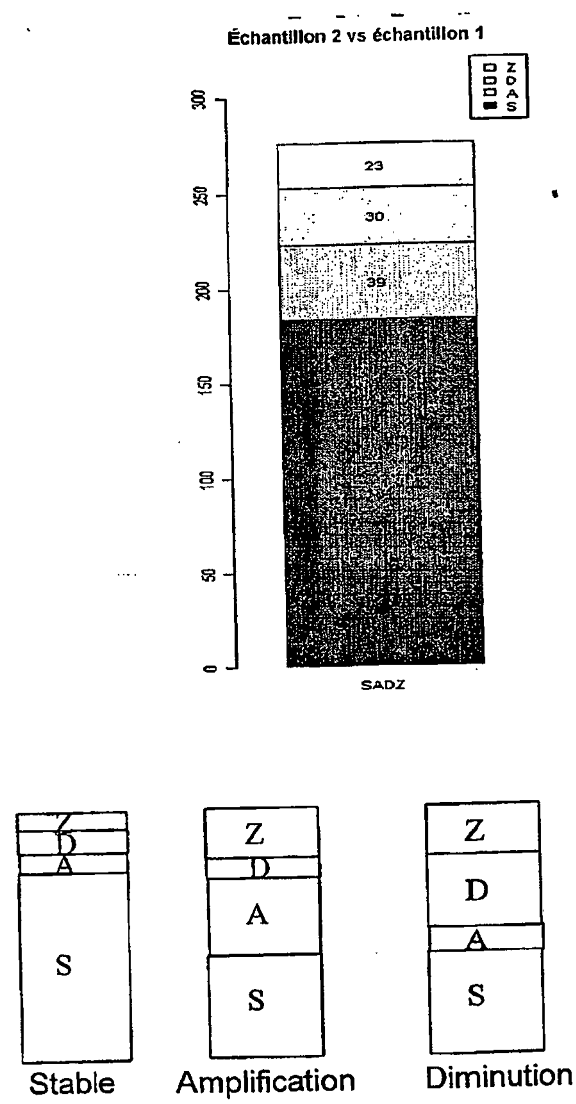
Échantillon 2 vs échantillon 1
Z
D
A
S
300
250
200
150
100
50
0
23
30
39
SADZ
Z
D
A
S
Stable
Z
D
A
S
Amplification
Z
D
A
S
Diminution

**Figure 11**

**ImmunTraCkeR trousse hTRB sur Thymus**

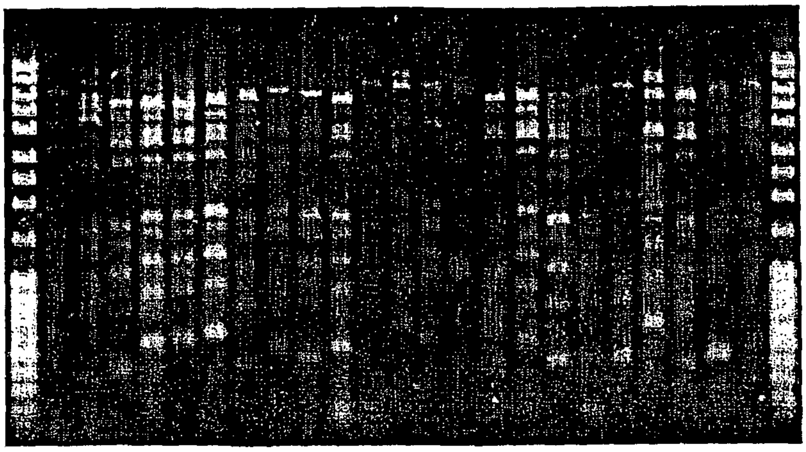

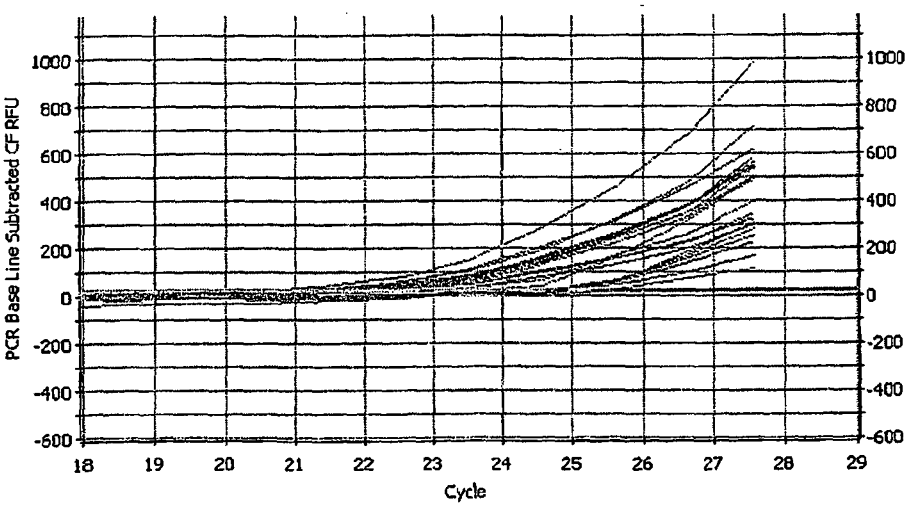


**Figure 12a**

Pool 3 lignées :

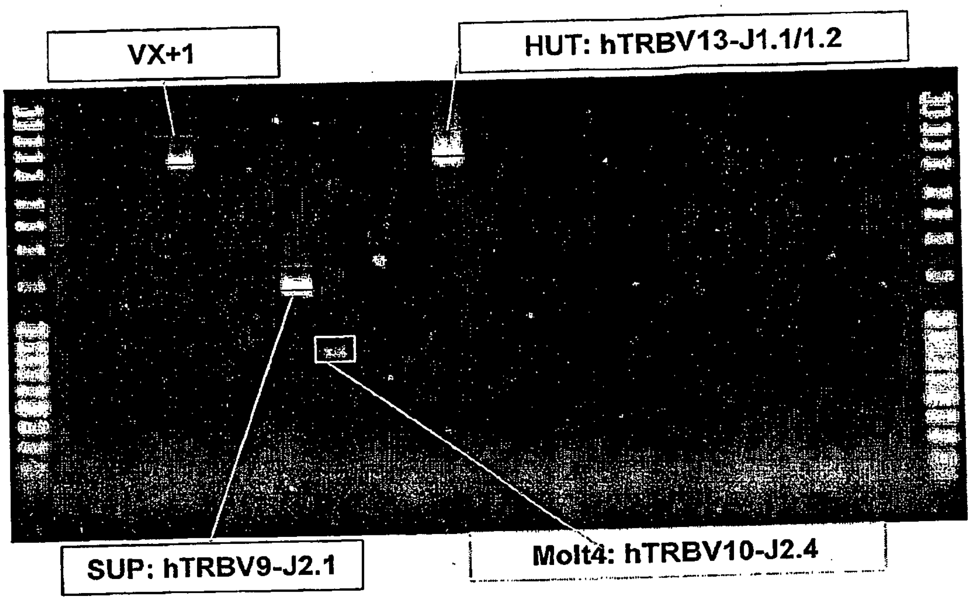


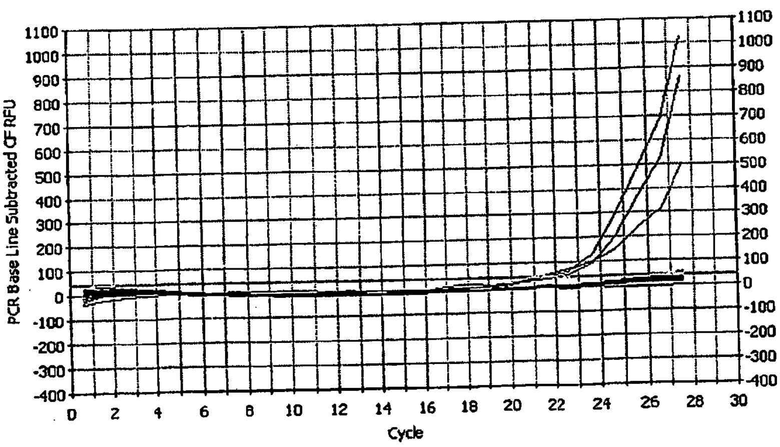


Figure 12b

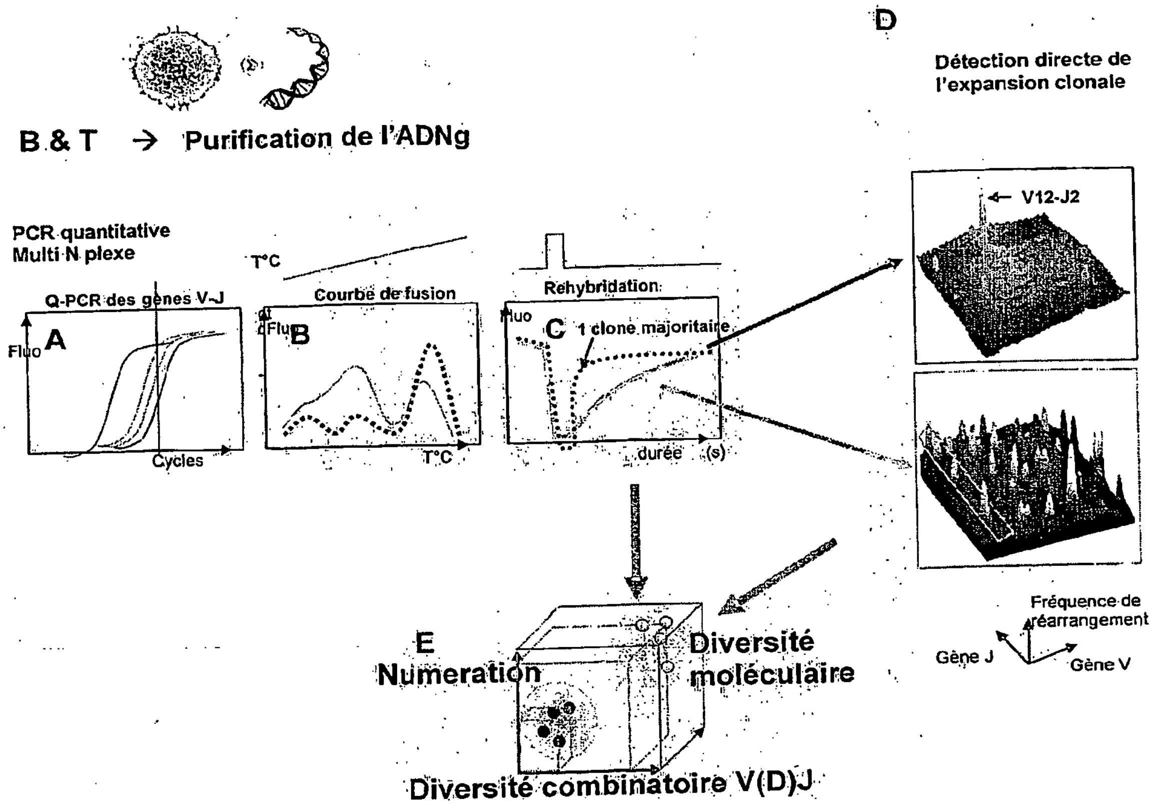
B & T → Purification de l'ADNg
PCR quantitative
Multi N plexe
Q-PCR des gènes V-J
A
Fluo
Cycles
T°C
Courbe de fusion
B
Flu
T°C
Rehybridation
C
Fluo
1 clone majoritaire
durée (s)
D
Détection directe de
l'expansion clonale
V12-J2
Fréquence de
réarrangement
Gène J
Gène V
E
Numeration
Diversité
moléculaire
Diversité combinatoire V(D)J

Figure 12c

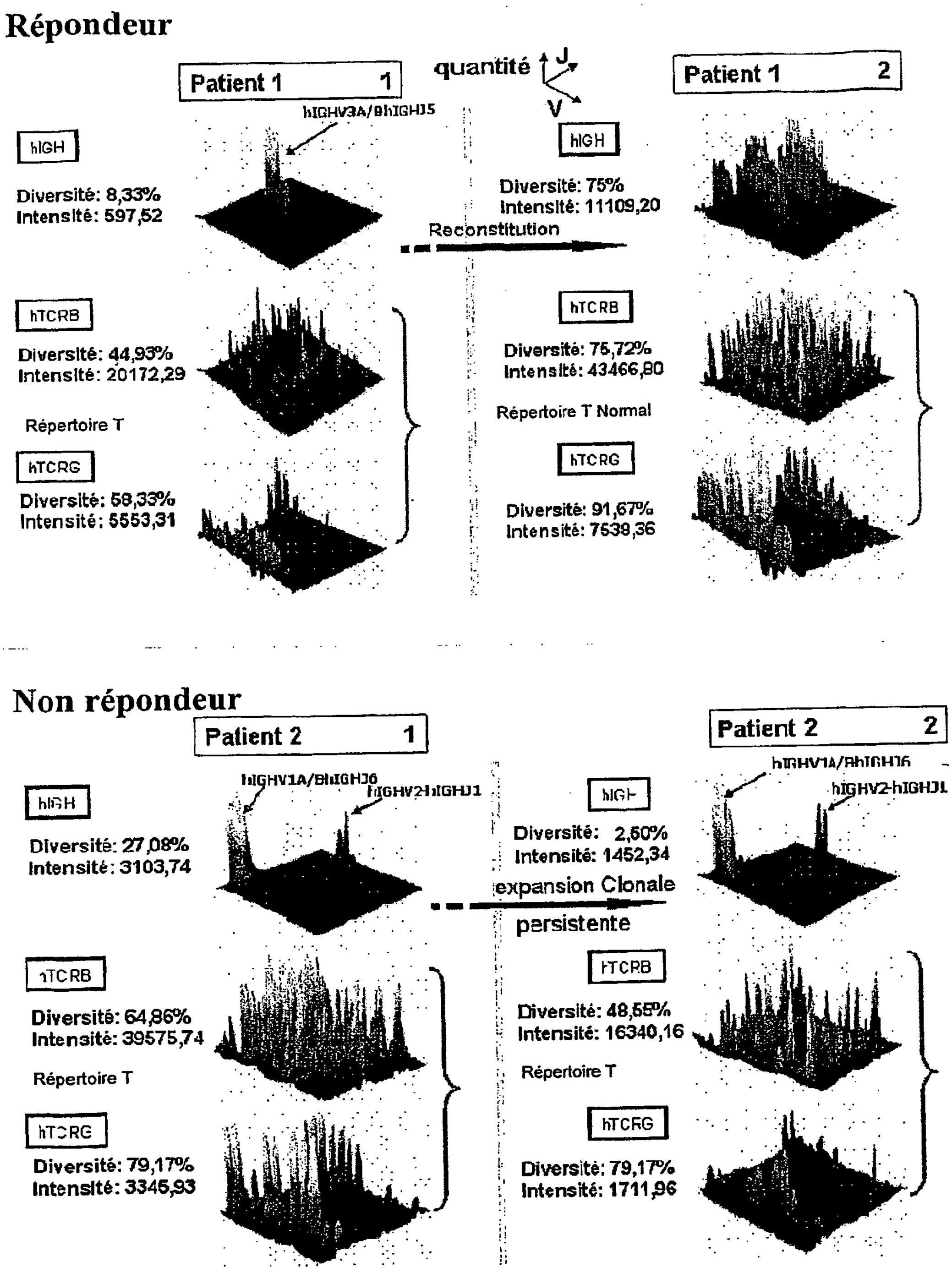
Répondeur
Patient 1 1
quantité J V
Patient 1 2
hIGHV3A/8hIGHJ5
hIGH
Diversité: 8,33%
Intensité: 597,52
hIGH
Diversité: 75%
Intensité: 11109,20
Reconstitution
hTCRB
Diversité: 44,93%
Intensité: 20172,29
Répertoire T
hTCRB
Diversité: 75,72%
Intensité: 43466,80
Répertoire T Normal
hTCRG
Diversité: 58,33%
Intensité: 5553,31
hTCRG
Diversité: 91,67%
Intensité: 7539,36
Non répondeur
Patient 2 1
Patient 2 2
hIGHV1A/8hIGHJ6
hIGHV2-hIGHJ1
hIGH
Diversité: 27,08%
Intensité: 3103,74
hIGH
Diversité: 2,60%
Intensité: 1452,34
expansion Clonale persistente
hTCRB
Diversité: 64,86%
Intensité: 39575,74
Répertoire T
hTCRB
Diversité: 48,65%
Intensité: 16340,16
Répertoire T
hTCRG
Diversité: 79,17%
Intensité: 3345,93
hTCRG
Diversité: 79,17%
Intensité: 1711,96

Figure 13

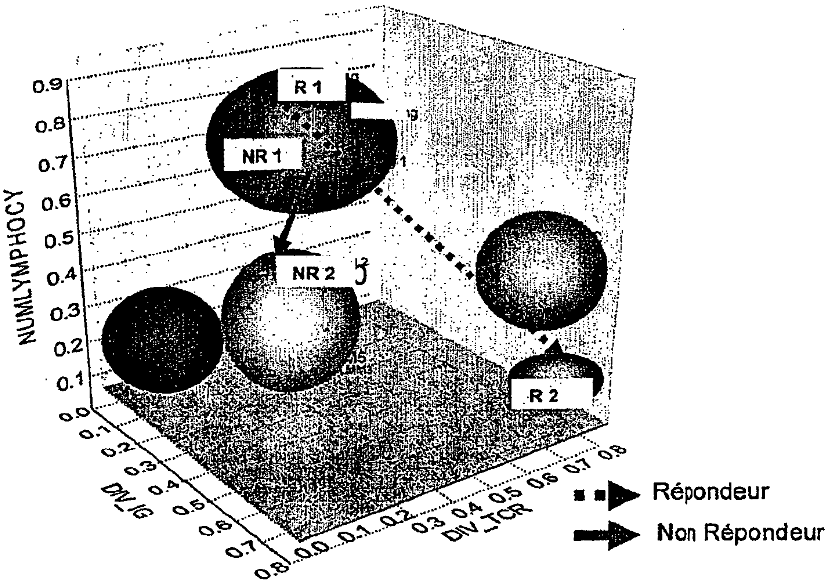


Figure 14

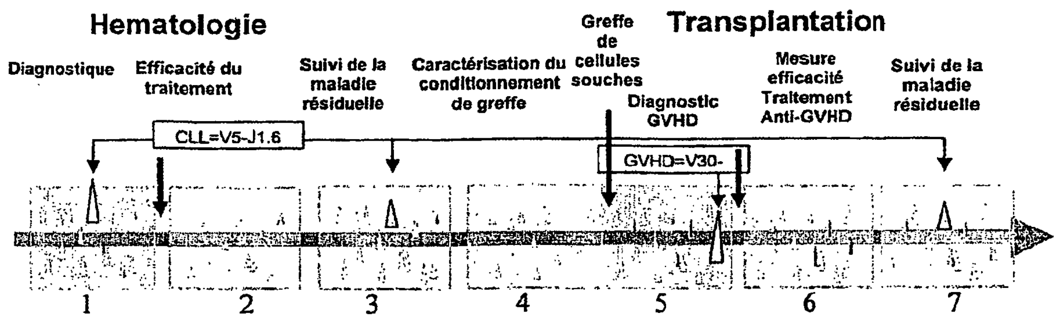


Figure 15

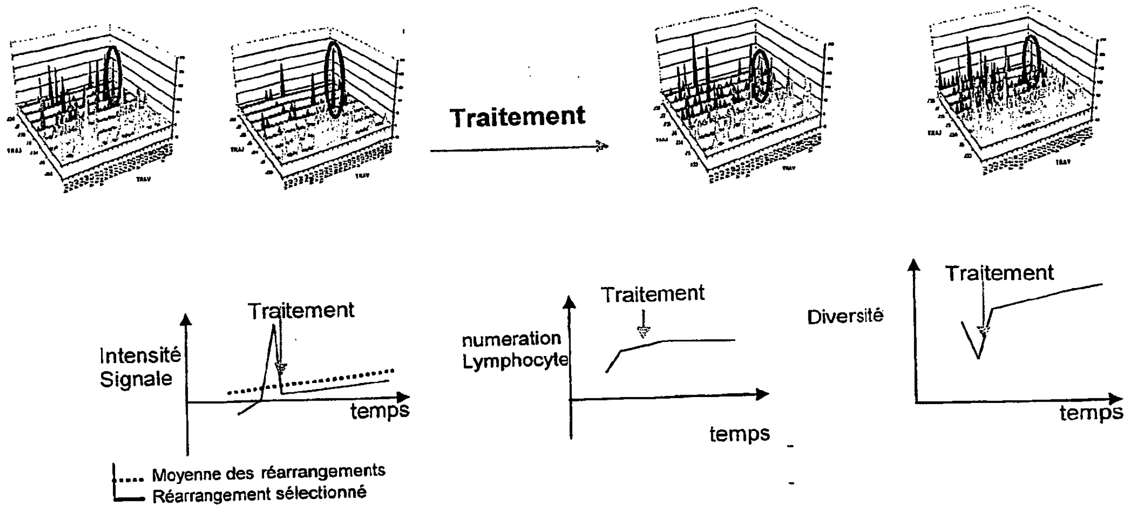


Figure 16

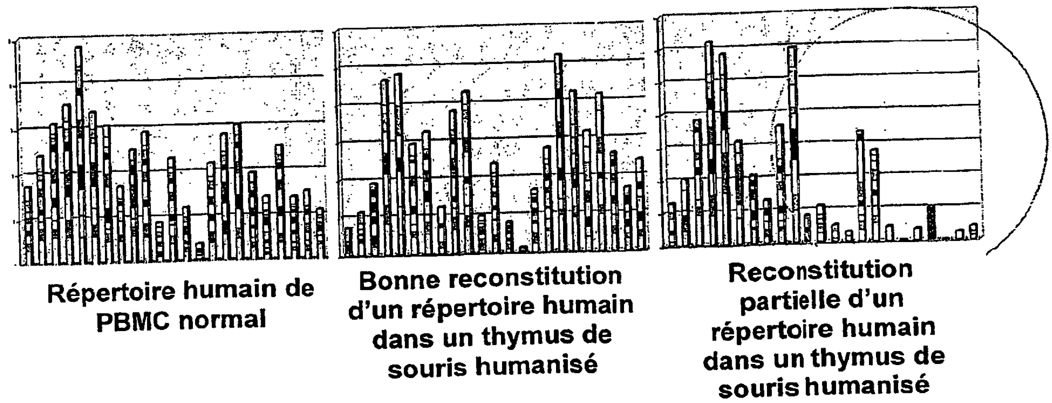


Figure 17

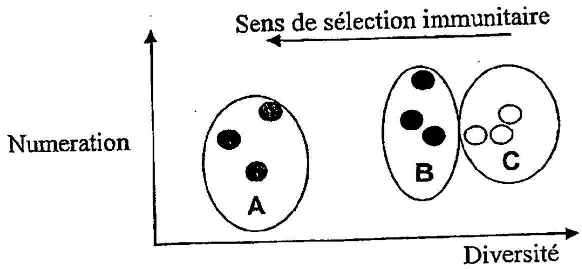


**Figure 18**

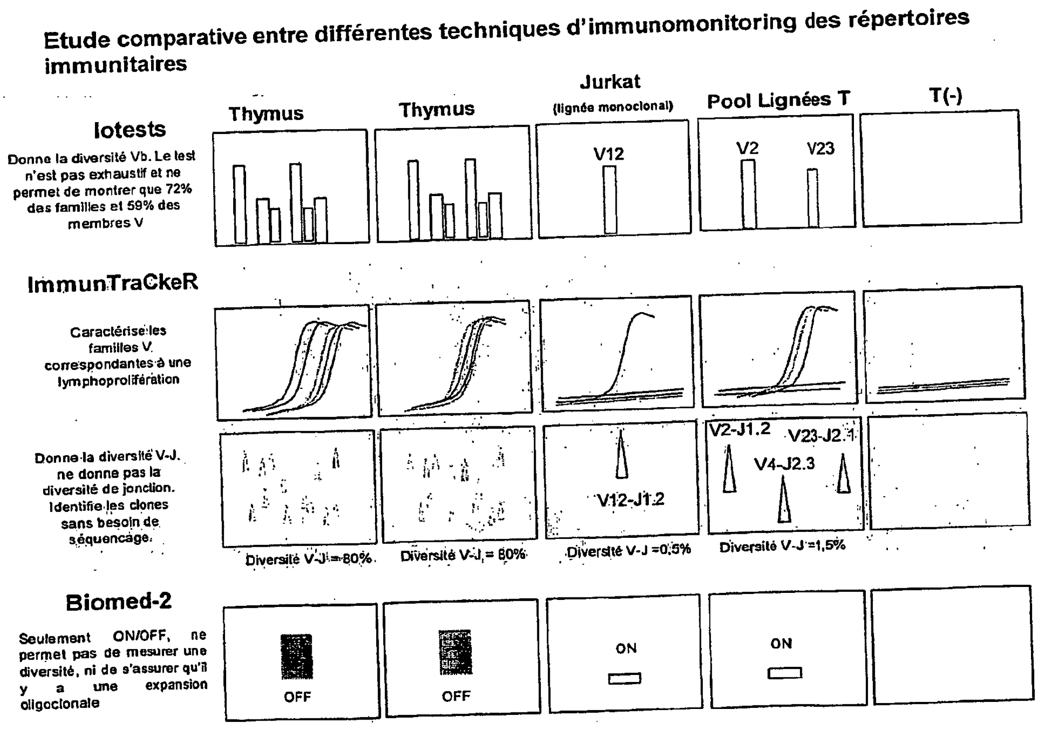


**Figure 19**

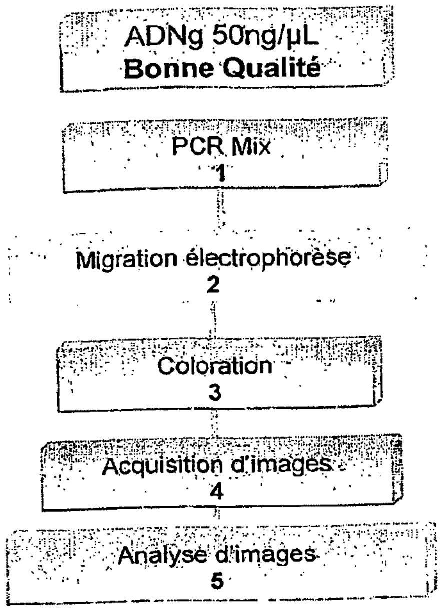
ADNg 50ng/µL
Bonne Qualité
PCR Mix
1
Migration électrophorèse
2
Coloration
3
Acquisition d'images
4
Analyse d'images
5

**Figure 20**

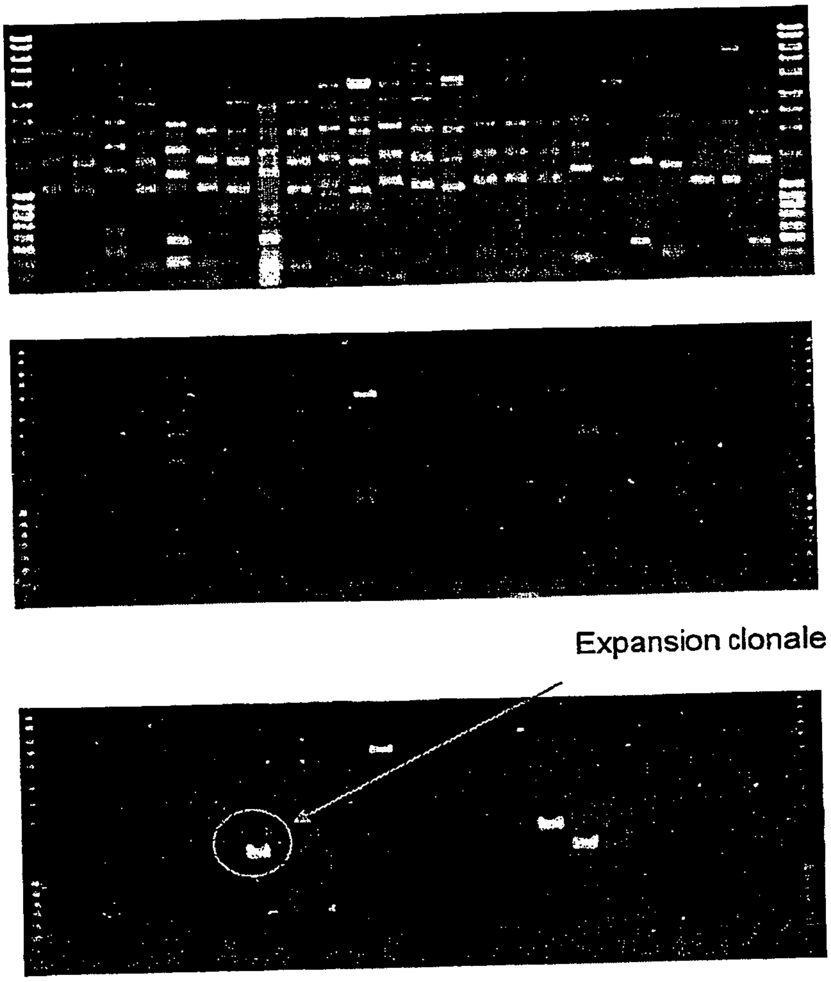


Figure 21

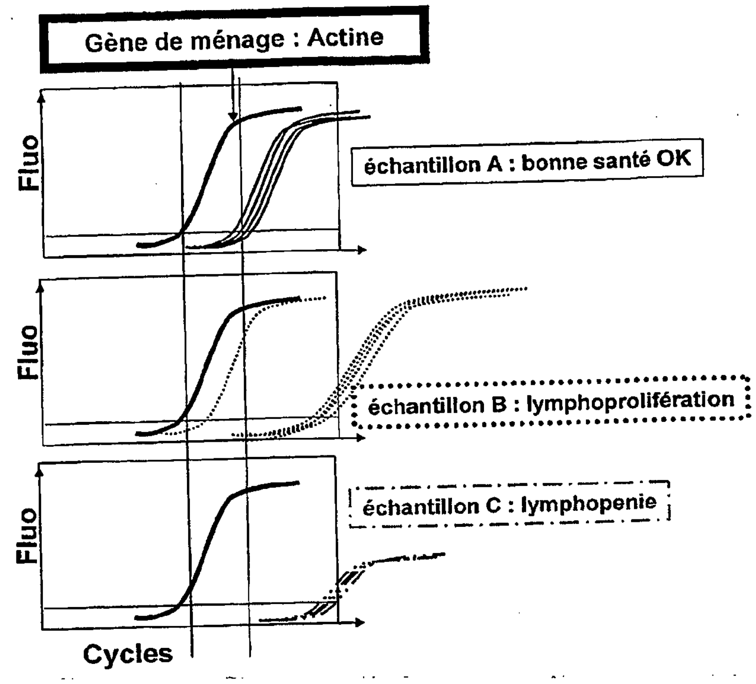


Figure 22a

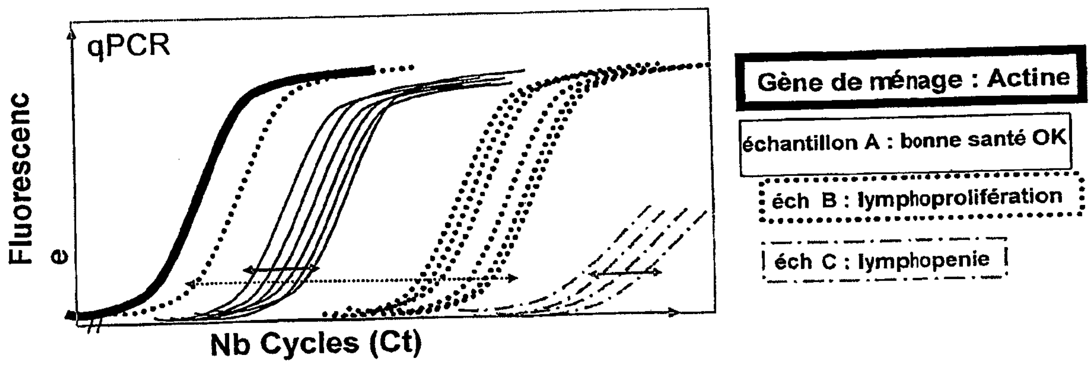


Figure 22b

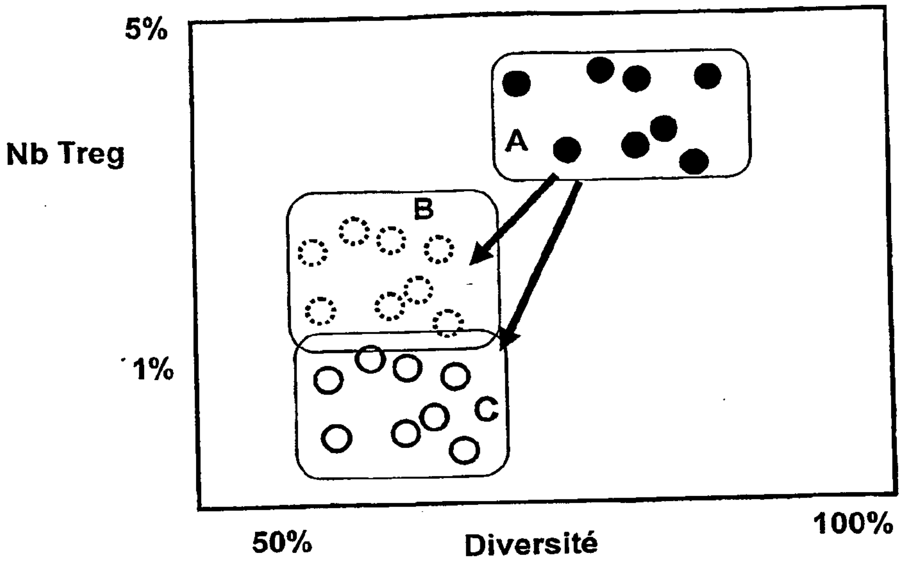


Figure 23

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5296351 A
- US 5418134 A
- WO 2005056828 A


### Littérature non-brevet citée dans la description


- **LEFRANC, M.-P ; LEFRANC, G.** The Immunoglobulin FactsBook. Academic Press, 2001, 458
- **LEFRANC, M.-P. ; LEFRANC, G.** The T cell receptor FactsBook. Academic Press, 2001, 398
- **AUDE-GARCIA, C. ; GALLAGHER, M. ; MARCHE, P. N. ; JOUVIN-MARCHE, E.** Preferential ADV-AJ association during recombination in the mouse T-cell receptor alpha/delta locus. *Immunogenetics,* 2001, vol. 52, 224-230
- **BAUM, P. D. ; MCCUNE, J. M.** Direct measurement of T-cell receptor repertoire diversity with AmpliCot. *Nat Methods,* 2006, vol. 3, 895-901
- **BAUM, T. P. ; HIERLE, V. ; PASQUAL, N. ; BELLAHCENE, F. ; CHAUME, D. ; LEFRANC, M. P. ; JOUVIN-MARCHE, E. ; MARCHE, P. N. ; DEMONGEOT, J.** IMGT/GeneInfo: T cell receptor gamma TRG and delta TRD genes in database give access to all TR potential V(D)J recombinations. *BMC Bioinformatics,* 2006, vol. 7, 224
- **BAUM, T. P. ; PASQUAL, N. ; THUDEROZ, F. ; HIERLE, V. ; CHAUME, D. ; LEFRANC, M. P. ; JOUVIN-MARCHE, E. ; MARCHE, P. N. ; DEMONGEOT, J.** IMGT/GeneInfo: enhancing V(D)J recombination database accessibility. *Nucleic Acids Res,* 2004, vol. 32, D51-54
- **BEREK, C. ; GRIFFITHS, G. M. ; MILSTEIN, C.** Molecular events during maturation of the immune response to oxazolone. *Nature,* 1985, vol. 316, 412-418
- **BOGUE, M. ; GILFILLAN, S. ; BENOIST, C. ; MATHIS, D.** Regulation of N-region diversity in antigen receptors through thymocyte differentiation and thymus ontogeny. *Proc Natl Acad Sci U S A,* 1992, vol. 89, 11011-11015
- **BONARIUS, H. P. ; BAAS, F. ; REMMERSWAAL, E. B. ; VAN LIER, R. A. ; TEN BERGE, I. J. ; TAK, P. P. ; DE VRIES, N.** Monitoring the T-cell receptor repertoire at single-clone résolution. *PLoS ONE,* 2006, vol. 1, e55
- **CABANIOLS, J. P. ; FAZILLEAU, N. ; CASROUGE, A. ; KOURILSKY, P. ; KANELLOPOULOS, J. M.** Most alpha/beta T cell receptor diversity is due to terminal deoxynucleotidyl transferase. *J Exp Med,* 2001, vol. 194, 1385-1390
- **CHAUDHURI, J. ; TIAN, M. ; KHUONG, C. ; CHUA, K. ; PINAUD, E. ; ALT, F. W.** Transcription-targeted DNA deamination by the AID antibody diversification enzyme. *Nature,* 2003, vol. 422, 726-730
- **COCHET, M. ; PANNETIER, C. ; REGNAULT, A. ; DARCHE, S. ; LECLERC, C. ; KOURILSKY, P.** Molecular detection and in vivo analysis of the specific T cell response to a protein antigen. *Eur J Immunol,* 1992, vol. 22, 2639-2647
- **DAVIS, M. M. ; BJORKMAN, P. J.** T-cell antigen receptor genes and T-cell recognition. *Nature,* 1988, vol. 334, 395-402
- **DOUEK, D. C. ; MCFARLAND, R. D. ; KEISER, P. H. ; GAGE, E. A. ; MASSEY, J. M. ; HAYNES, B. F. ; POLIS, M. A. ; HAASE, A. T. ; FEINBERG, M. B. ; SULLIVAN, J. L. et al.** Changes in thymic function with age and during the treatment of HIV infection. *Nature,* 1998, vol. 396, 690-695
- **FUGMANN, S. D. ; LEE, A. I. ; SHOCKETT, P. E. ; VILLEY, I. J. ; SCHATZ, D. G.** The RAG proteins and V(D)J recombination: complexes, ends, and transposition. *Annu Rev Immunol,* 2000, vol. 18, 495-527
- **FUSCHIOTTI, P. ; PASQUAL, N. ; HIERLE, V. ; BOREL, E. ; LONDON, J. ; MARCHE, P. N. ; JOUVIN-MARCHE, E.** Analysis of the TCR alpha-chain rearrangement profile in human T lymphocytes. *Mol Immunol,* 2007, vol. 44, 3380-3388
- **HAMBLIN, T. J. ; DAVIS, Z. ; GARDINER, A. ; OSCIER, D. G. ; STEVENSON, F. K.** Unmutated Ig V(H) genes are associated with a more aggressive form of chronic lymphocytic leukemia. *Blood,* 1999, vol. 94, 1848-1854
- **HUANG, C. ; KANAGAWA, O.** Ordered and coordinated rearrangement of the TCR alpha locus: role of secondary rearrangement in thymic selection. *J Immunol,* 2001, vol. 166, 2597-2601

- **JOUVIN-MARCHE, E. ; AUDE-GARCIA, C. ; CANDEIAS, S. ; BOREL, E. ; HACHEMI-RACHEDI, S. ; GAHERY-SEGARD, H. ; CAZENAVE, P. A. ; MARCHE, P. N.** Differential chronology of TCRADV2 gene use by alpha and delta chains of the mouse TCR. *Eur J Immunol,* 1998, vol. 28, 818-827
- **KOTANI, A. ; OKAZAKI, I. M. ; MURAMATSU, M. ; KINOSHITA, K. ; BEGUM, N. A. ; NAKAJIMA, T. ; SAITO, H. ; HONJO, T.** A target selection of somatic hypermutations is regulated similarly between T and B cells upon activation-induced cytidine deaminase expression. *Proc Natl Acad Sci U S A,* 2005, vol. 102, 4506-4511
- **LANG, R. ; PFEFFER, K. ; WAGNER, H. ; HEEG, K.** A rapid method for semiquantitative analysis of the human V beta-repertoire using TaqManR PCR. *J Immunol Methods,* 1997, vol. 203, 181-192
- **LEFRANCS.** The Immunoglobulin Facts Book. 2001
- **LEFRANCS.** The T cell receptor Facts Book. 2001
- **OPREA, M. ; KEPLER, T. B.** Genetic plasticity of V genes under somatic hypermutation: statistical analyses using a new resampling-based methodology. *Genome Res,* 1999, vol. 9, 1294-1304
- **PANNETIER, C. ; EVEN, J. ; KOURILSKY, P.** T-cell repertoire diversity and clonal expansions in normal and clinical samples. *Immunol Today,* 1995, vol. 16, 176-181
- **PASQUAL, N. ; GALLAGHER, M. ; AUDE-GARCIA, C. ; LOIODICE, M. ; THUDEROZ, F. ; DEMONGEOT, J. ; CEREDIG, R. ; MARCHE, P. N. ; JOUVIN-MARCHE, E.** Quantitative and qualitative changes in V-J alpha rearrangements during mouse thymocytes differentiation: implication for a limited T cell receptor alpha chain repertoire. *J Exp Med,* 2002, vol. 196, 1163-1173
- **PHAM, T. ; BELZER, M. ; CHURCH, J. A. ; KITCHEN, C. ; WILSON, C. M. ; DOUGLAS, S. D. ; GENG, Y. ; SILVA, M. ; MITCHELL, R. M. ; KROGSTAD, P.** Assessment of thymic activity in human immunodeficiency virus-negative and -positive adolescents by real-time PCR quantitation of T-cell receptor rearrangement excision circles. *Clin Diagn Lab Immunol,* 2003, vol. 10, 323-328
- **RYTKONEN, M. A. ; HURWITZ, J. L. ; THOMPSON, S. D. ; PELKONEN, J.** Restricted onset of T cell receptor alpha gene rearrangement in fetal and neonatal thymocytes. *Eur J Immunol,* 1996, vol. 26, 1892-1896
- **VAN DEN BEEMD ; VAN DONGEN et al.** Flow cytometric detection of clonality in mature T-cell malignancies by use of a Vb antibody kit. *ISAC Abstract,* 2000
- **WANG, F. ; HUANG, C. Y. ; KANAGAWA, O.** Rapid deletion of rearranged T cell antigen receptor (TCR) Valpha-Jalpha segment by secondary rearrangement in the thymus: role of continuous rearrangement of TCR alpha chain gene and positive selection in the T cell repertoire formation. *Proc Natl Acad Sci U S A,* 1998, vol. 95, 11834-11839